# EUROPEAN PATENT APPLICATION

(11) **EP 4 227 411 A1**
(43) Date of publication of application: **16.08.2023**
(21) Application number: 21878063.3
(22) Date of filing: 08.10.2021
(51) Int. Cl.: C12N 15/113, C12N 15/63, C12N 9/22, C12N 15/90, C12N 15/10

(54) **ENGINEERED GUIDE RNA FOR INCREASING EFFICIENCY OF CRISPR/CAS12F1 SYSTEM, AND USE OF SAME**

(30) Priority: 08.10.2020 KR 20200129937; 29.12.2020 KR 20200185528; 21.04.2021 KR 20210051552
(71) Applicant: Genkore Inc, Yuseong-gu, Daejeon 34141 (KR)
(72) Inventor: KIM, Yong-Sam, Daejeon 34118 (KR); KIM, Do Yon, Daejeon 34069 (KR)
(74) Representative: Dehns
(86) International application number: PCT/KR2021/013923
(87) International publication number: WO 2022/075813

(57) **Abstract**

In the present disclosure, there is provided an engineered Cas12f1 guide RNA for increasing intracellular gene editing activity of a CRISPR/Cas12f1 system by overcoming limitations of the prior art. The engineered Cas12f1 guide RNA is an engineered form of a naturally occurring guide RNA whose structure is partially modified. In addition, the engineered Cas12f1 guide RNA is characterized in that at least a portion of the scaffold region, which serves to interact with the Cas12f1 protein, is modified. The engineered scaffold region is characterized in that it is different from a scaffold region of a naturally occurring guide RNA.

## Description

### TECHNICAL FIELD

### [Related applications]

This application claims priority to Korean Patent Application No. 10-2020-0129937, filed on October 8, 2020, and Korean Patent Application No. 10-2021-0051552, filed on April 21, 2021, the disclosure of each of which is incorporated herein by reference in its entirety.

### [Technical field]

Disclosed herein is a technology for the field of using a CRISPR/Cas system, particularly a CRISPR/Cas12f1 system, for gene editing.

### BACKGROUND ART

A CRISPR/Cas12f1 system is a CRISPR/Cas system classified as Class 2, Type V. A previous study (Harrington et al., Programmed DNA destruction by miniature CRISPR-Cas14 enzymes, Science 362, 839-842 (2018)) has reported for the first time the CRISPR/Cas14a system which is a CRISPR/Cas system derived from Archaea. A subsequent study (Karvelis et al., Nucleic Acids Research, Vol. 48, No. 9 5017 (2020)) classified the CRISPR/Cas14 system as a CRISPR/Cas12f1 system. The CRISPR/Cas12f1 system belongs to a V-F1 system, which is a subtype of the CRISPR/Cas system classified as Class 2, Type V, and includes the CRISPR/Cas14a system having Cas14a as an effector protein. The CRISPR/Cas12f1 system is characterized in that a size of its effector protein is significantly smaller than a CRISPR/Cas9 system. However, as revealed in a previous study (Harrington et al., Programmed DNA destruction by miniature CRISPR-Cas14 enzymes, Science 362, 839-842 (2018), US 2020/0190494 A1), the CRISPR/Cas12f1 system, particularly the CRISPR/Cas14a system shows cleavage activity on single-stranded DNA and has no or extremely low cleavage activity on double-strand DNA, which limits its application to gene editing technology.

As a result of continuing studies, a recent prior literature (Takeda et al., Structure of the miniature type V-F CRISPR-Cas effector enzyme, Molecular Cell 81, 1-13 (2021)) revealed the fact that a Cas12f1 protein forms a dimer in the CRISPR/Cas12f1 system, and a structure of its guide RNA. It was found by the prior literature that the guide RNA comprises a so-called disordered region which does not directly interact with the Cas12f1 protein. In another prior literature (Xiao et al., Structural basis for the dimerization-dependent CRISPR-Cas12f nuclease, bioRxiv (2020)), the disordered region was removed and double-stranded DNA cleavage efficiency of the system was investigated *in vitro.*

However, 1) the preceding studies did not show intracellular gene editing activity, nor related to methods of increasing intracellular gene editing activity (for example, indel generation efficiency); 2) among the studies, there was an experiment in which the disordered region was removed, but it was reported in this experiment that the cleavage activity was even lowered; and moreover, 3) the experiment was performed *in vitro* and failed to reveal what modifications exhibit intracellular gene editing activity or increase efficiency.

### DETAILED DESCRIPTION OF THE INVENTION

### TECHNICAL PROBLEM

The present disclosure intends to provide an engineered Cas12f1 guide RNA that can be used in a CRISPR/Cas12f1 system to increase gene editing efficiency.

The present disclosure intends to provide an engineered scaffold region that is included in the engineered Cas12f1 guide RNA and can increase gene editing efficiency.

The present disclosure intends to provide an engineered CRISPR/Cas12f1 complex with increased gene editing efficiency.

The present disclosure intends to provide an engineered CRISPR/Cas12f1 system with increased gene editing efficiency.

The present disclosure intends to provide a vector having nucleic acids that encode respective components of the engineered CRISPR/Cas12f1 system.

The present disclosure intends to provide a gene editing method using the engineered CRISPR/Cas12f1 system.

The present disclosure intends to provide a use of the engineered CRISPR/Cas12f1 system.

### SOLUTION TO PROBLEM

In an embodiment, there is provided herein an engineered guide RNA for a CRISPR/Cas12f1 system, comprising:
an engineered scaffold region, and
a spacer;
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
the sequence of the engineered scaffold region is such that the following sequences are sequentially linked to each other in a 5' to 3' direction:
   a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
   a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
   5'-GCUGCUUGCAUCAGCCUAAUGUCGAGAAGUGCUUUCUUCGGAAAGUAAC CCUCGA-3' (SEQ ID NO: 11);
   a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
   5'-AUGCAAC-3'.

In order to solve the problem, there is provided herein an engineered guide RNA for a CRISPR/Cas12f1 system, comprising:
an engineered scaffold region, and
a spacer,
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAAGAAAGUUGCAGAACCCGAAUAGACGAAUGAAGGA AUGCAAC-3' (SEQ ID NO: 315), and
the sequence of the engineered scaffold region is such that the following sequences are sequentially linked to each other in a 5' to 3' direction:
   a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
   a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

   a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), 5'-AACAAAUUCAUUUGAAAGAAUGAAGGA-3' (SEQ ID NO: 117), 5'-AACAAAUUCAUUUUGAAACGAAUGAAGGA-3' (SEQ ID NO: 293), 5'-AACAAAUUCAUUUUUGAAAACGAAUGAAGGA-3' (SEQ ID NO: 294), 5'-AACAAAUUCAUUUUUCGAAAGACGAAUGAAGGA-3' (SEQ ID NO: 295), 5'-AACAAAUUCAUUUUUCCGAAAAGACGAAUGAAGGA-3' (SEQ ID NO: 296), 5'-AACAAAUUCAUUUUUCCUGAAAUAGACGAAUGAAGGA-3' (SEQ ID NO: 297), 5'-AACAAAUUCAUUUUUCCUCGAAAAUAGACGAAUGAAGGA-3' (SEQ ID NO: 298), 5'-AACAAAUUCAUUUUUCCUCUGAAAAAUAGACGAAUGAAGGA-3' (SEQ ID NO: 299), 5'-AACAAAUUCAUUUUUCCUCUCGAAAGAAUAGACGAAUGAAGGA-3' (SEQ ID NO: 300), 5'-AACAAAUUCAUUUUUCCUCUCCGAAACGAAUAGACGAAUGAAGGA-3' (SEQ ID NO: 301), 5'-AACAAAUUCAUUUUUCCUCUCCAGAAACCGAAUAGACGAAUGAAGGA-3' (SEQ ID NO: 302), 5'-AACAAAUUCAUUUUUCCUCUCCAAGAAACCCGAAUAGACGAAUGAAGGA-3' (SEQ ID NO: 303), 5'-AACAAAUUCAUUUUUCCUCUCCAAUGAAAACCCGAAUAGACGAAUGAAGG A-3' (SEQ ID NO: 304), 5'-AACAAAUUCAUUUUUCCUCUCCAAUUGAAAAACCCGAAUAGACGAAUGAA GGA-3' (SEQ ID NO: 305), 5'-AACAAAUUCAUUUUUCCUCUCCAAUUCGAAAGAACCCGAAUAGACGAAUG AAGGA-3' (SEQ ID NO: 306), 5'-AACAAAUUCAUUUUUCCUCUCCAAUUCUGAAAAGAACCCGAAUAGACGAA UGAAGGA-3' (SEQ ID NO: 307), 5'-AACAAAUUCAUUUUUCCUCUCCAAUUCUGGAAACAGAACCCGAAUAGAC GAAUGAAGGA-3' (SEQ ID NO: 308), 5'-AACAAAUUCAUUUUUCCUCUCCAAUUCUGCGAAAGCAGAACCCGAAUAG ACGAAUGAAGGA-3' (SEQ ID NO: 309), 5'-AACAAAUUCAUUUUUCCUCUCCAAUUCUGCAGAAAUGCAGAACCCGAAUA GACGAAUGAAGGA-3' (SEQ ID NO: 310), 5'-AACAAAUUCAUUUUUCCUCUCCAAUUCUGCACGAAAUUGCAGAACCCGA AUAGACGAAUGAAGGA-3' (SEQ ID NO: 311), 5'-AACAAAUUCAUUUUUCCUCUCCAAUUCUGCACAGAAAGUUGCAGAACCC GAAUAGACGAAUGAAGGA-3' (SEQ ID NO: 312), 5'-AACAAAUUCAUUUUUCCUCUCCAAUUCUGCACAGAAAUUGCAGAACCCG AAUAGACGAAUGAAGGA-3' (SEQ ID NO: 313), and 5'-AACAAAUUCAUUUUUCCUCUCCAAUUCUGCACAAGAAAGUUGCAGAACC CGAAUAGACGAAUGAAGGA-3' (SEQ ID NO: 314); and
   5'-AUGCAAC-3'.

In an embodiment, there is provided herein an engineered guide RNA for a CRISPR/Cas12f1 system, comprising:
an engineered scaffold region, and
a spacer,
wherein the engineered scaffold region and the spacer are sequentially linked to each other in a 5' to 3' direction,
the spacer has a length of 10 to 50 nucleotides and has a sequence complementary to a target sequence, and
a sequence of the engineered scaffold region is such that the following sequences are sequentially linked to each other in a 5' to 3' direction:
   a first sequence represented by 5'-A-3';
   a second sequence represented by 5'-CCGCUUCAC-3' (SEQ ID NO: 432);
   a third sequence represented by 5'-UUAG-3';
   a fourth sequence represented by 5'-AGUGAAGGUGG-3' (SEQ ID NO: 433);
   a fifth sequence represented by 5'-GCUGCUUGCAUCAGCCUAAUGUCGAGAAGUGCUUUCUUCGGAAAGUAAC CCUCGA-3' (SEQ ID NO: 11);
   a sixth sequence represented by 5'-AACAAA-3';
   a linker;
   a seventh sequence represented by 5'-GGA-3'; and
   an eighth sequence represented by 5'-AUGCAAC-3'.

In order to solve the problem, there is provided herein an engineered guide RNA for a CRISPR/Cas12f1 system, comprising:
an engineered scaffold region, and
a spacer,
wherein the spacer has a length of 10 to 30 nucleotides and has a sequence complementary to a target sequence,
a sequence of the engineered scaffold region comprises in a 5' to 3' direction:
   an engineered tracrRNA in which the following sequences are linked to each other:
   a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9),
   a sequence selected from the group consisting of 5'-CCGCUUCACUUAGAGUGAAGGUGG-3' (SEQ ID NO: ID NO: 430), 5'-CCGCUUCACCUUAGGAGUGAAGGUG'-3' (SEQ ID NO: 431), 5'-CCGCUUCACCAUUAGUGAGUGAAGGUG'-3' (SEQ ID NO: 38), 5'-CCGCUUCACCAAUUAGUUGAGUGAAGGUG'-3' (SEQ ID NO: 39), 5'-CCGCUUCACCAAAUUAGCUUGAGUGAAGGUG'-3' (SEQ ID NO: 40), 5'-CCGCUUCACCAAAAUUAGACUUGAGUGAAGGUG'-3' (SEQ ID NO: 41), 5'-CCGCUUCACCAAAAGUUAGAACUUGAGUGAAGGUG'-3' (SEQ ID NO: 42), 5'-CCGCUUCACCAAAAGCUUAGGAACUUGAGUGAAGGUG'-3' (SEQ ID NO: 43), 5'-CCGCUUCACCAAAAGCUUUAGAGAACUUGAGUGAAGGUG'-3' (SEQ ID NO: 44), 5'-CCGCUUCACCAAAAGCUGUUAGUUAGAACUUGAGUGAAGGUG'-3' (SEQ ID NO: 45), 5'-CCGCUUCACCAAAAGCUGUUAGUAGAACUUGAGUGAAGGUG'-3' (SEQ ID NO: 46), 5'-CCGCUUCACCAAAAGCUGUUUAGAUUAGAACUUGAGUGAAGGUG'-3' (SEQ ID NO: 47), 5'-CCGCUUCACCAAAAGCUGUCUUAGGAUUAGAACUUGAGUGAAGGUG'-3' (SEQ ID NO: 48), 5'-CCGCUUCACCAAAAGCUGUCCUUAGGGAUUAGAACUUGAGUGAAGGUG'-3' (SEQ ID NO: 49), and 5'-CCGCUUCACCAAAAGCUGUCCCUUAGGGGAUUAGAACUUGAGUGAAGGU G'-3' (SEQ ID NO: 10), 
5'-
GCUGCUUGCAUCAGCCUAAUGUCGAGAAGUGCUUUCUUCGGAAAGUAAC
CCUCGA-3' (SEQ ID NO: 11),
   and
   a sequence selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 66), 5'-AACAAAUUCAU-3' (SEQ ID NO: 67), 5'-AACAAAUUCAUU-3' (SEQ ID NO: 68), and 5'-AACAAAUUCAUUU-3' (SEQ ID NO: 12); and
   an engineered crRNA repeat sequence portion in which the following sequences are linked to each other:
      a sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', and 5'-GAAUGAAGGA-3' (SEQ ID NO: 14), and
      5'-AUGCAAC-3',
      wherein the 3' end of the engineered crRNA repeat sequence is linked to the 5' end of the spacer, and
      wherein one in which a sequence of the engineered tracrRNA is the same as 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUU-3' (SEQ ID NO: 1) and the engineered crRNA repeat sequence is the same as 5'-GAAUGAAGGAAUGCAAC-3' (SEQ ID NO: 3) is excluded.

In an embodiment, there is provided herein an engineered CRISPR/Cas12f1 complex comprising:
a Cas12f1 protein, and
an engineered guide RNA,
wherein the engineered guide RNA comprises:
   an engineered scaffold region, and
   a spacer,
   wherein the engineered scaffold region and the spacer are sequentially linked to each other in a 5' to 3' direction,
   the spacer comprises 10 nucleotides to 50 nucleotides and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
      5'-GCUGCUUGCAUCAGCCUAAUGUCGAGAAGUGCUUUCUUCGGAAAGUAAC CCUCGA-3' (SEQ ID NO: 11);
      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
      5'-AUGCAAC-3'.

In an embodiment, there is provided herein a vector that is capable of expressing respective components of a CRISPR/Cas12f1 system, comprising:
a first sequence comprising a nucleic acid sequence encoding a Cas12f1 protein,
a first promoter sequence operably linked to the first sequence,
a second sequence comprising a nucleic acid sequence encoding the engineered guide RNA, and
a second promoter sequence operably linked to the second sequence,
wherein the engineered guide RNA comprises:
   an engineered scaffold region, and
   a spacer,
   wherein the engineered scaffold region and the spacer are sequentially linked to each other in a 5' to 3' direction,
   the spacer comprises 10 nucleotides to 50 nucleotides and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
      5'-AUGCAAC-3'.

In an embodiment, there is provided herein a method of editing a target nucleic acid in a cell or a method of targeting a target nucleic acid in a cell, comprising:
delivering, into the cell, a Cas12f1 protein or a nucleic acid encoding the same, and an engineered guide RNA or a nucleic acid encoding the same,
which allows a CRISPR/Cas12f1 complex to be formed in the cell, and
wherein the CRISPR/Cas12f1 complex is capable of editing or targeting the nucleic acid containing a target sequence,
the engineered guide RNA comprises:
   an engineered scaffold region, and
   a spacer,
   wherein the engineered scaffold region and the spacer are sequentially linked to each other in a 5' to 3' direction,
   the spacer comprises 10 nucleotides to 50 nucleotides and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
      5'-GCUGCUUGCAUCAGCCUAAUGUCGAGAAGUGCUUUCUUCGGAAAGUAAC CCUCGA-3' (SEQ ID NO: 11);
      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
      5'-AUGCAAC-3'.

In an embodiment, there is provided herein a method of editing a target nucleic acid in a cell or a method of targeting a target nucleic acid in a cell, comprising:
delivering, into the cell, a Cas12f1 protein or a nucleic acid encoding the same, and an engineered guide RNA or a nucleic acid encoding the same,
which allows a CRISPR/Cas12f1 complex to be formed in the cell, and
wherein the CRISPR/Cas12f1 complex is capable of editing or targeting the target nucleic acid, and
the engineered guide RNA comprises:
   an engineered scaffold region, and
   a spacer,
   wherein the engineered scaffold region and the spacer are sequentially linked to each other in a 5' to 3' direction,
   the spacer comprises 10 nucleotides to 50 nucleotides and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAAGAAAGUUGCAGAACCCGAAUAGACGAAUGAAGGA AUGCAAC-3' (SEQ ID NO: 315), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
      5'-GCUGCUUGCAUCAGCCUAAUGUCGAGAAGUGCUUUCUUCGGAAAGUAAC CCUCGA-3' (SEQ ID NO: 11);
      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), 5'-AACAAAUUCAUUUGAAAGAAUGAAGGA-3' (SEQ ID NO: 117), 5'-AACAAAUUCAUUUUGAAACGAAUGAAGGA-3' (SEQ ID NO: 293), 5'-AACAAAUUCAUUUUUGAAAACGAAUGAAGGA-3' (SEQ ID NO: 294), 5'-AACAAAUUCAUUUUUCGAAAGACGAAUGAAGGA-3' (SEQ ID NO: 295), 5'-AACAAAUUCAUUUUUCCGAAAAGACGAAUGAAGGA-3' (SEQ ID NO: 296), 5'-AACAAAUUCAUUUUUCCUGAAAUAGACGAAUGAAGGA-3' (SEQ ID NO: 297), 5'-AACAAAUUCAUUUUUCCUCGAAAAUAGACGAAUGAAGGA-3' (SEQ ID NO: 298), 5'-AACAAAUUCAUUUUUCCUCUGAAAAAUAGACGAAUGAAGGA-3' (SEQ ID NO: 299), 5'-AACAAAUUCAUUUUUCCUCUCGAAAGAAUAGACGAAUGAAGGA-3' (SEQ ID NO: 300), 5'-AACAAAUUCAUUUUUCCUCUCCGAAACGAAUAGACGAAUGAAGGA-3' (SEQ ID NO: 301), 5'-AACAAAUUCAUUUUUCCUCUCCAGAAACCGAAUAGACGAAUGAAGGA-3' (SEQ ID NO: 302), 5'-AACAAAUUCAUUUUUCCUCUCCAAGAAACCCGAAUAGACGAAUGAAGGA-3' (SEQ ID NO: 303), 5'-AACAAAUUCAUUUUUCCUCUCCAAUGAAAACCCGAAUAGACGAAUGAAGG A-3' (SEQ ID NO: 304), 5'-AACAAAUUCAUUUUUCCUCUCCAAUUGAAAAACCCGAAUAGACGAAUGAA GGA-3' (SEQ ID NO: 305), 5'-AACAAAUUCAUUUUUCCUCUCCAAUUCGAAAGAACCCGAAUAGACGAAUG AAGGA-3' (SEQ ID NO: 306), 5'-AACAAAUUCAUUUUUCCUCUCCAAUUCUGAAAAGAACCCGAAUAGACGAA UGAAGGA-3' (SEQ ID NO: 307), 5'-AACAAAUUCAUUUUUCCUCUCCAAUUCUGGAAACAGAACCCGAAUAGAC GAAUGAAGGA-3' (SEQ ID NO: 308), 5'-AACAAAUUCAUUUUUCCUCUCCAAUUCUGCGAAAGCAGAACCCGAAUAG ACGAAUGAAGGA-3' (SEQ ID NO: 309), 5'-AACAAAUUCAUUUUUCCUCUCCAAUUCUGCAGAAAUGCAGAACCCGAAUA GACGAAUGAAGGA-3' (SEQ ID NO: 310), 5'-AACAAAUUCAUUUUUCCUCUCCAAUUCUGCACGAAAUUGCAGAACCCGA AUAGACGAAUGAAGGA-3' (SEQ ID NO: 311), 5'-AACAAAUUCAUUUUUCCUCUCCAAUUCUGCACAGAAAGUUGCAGAACCC GAAUAGACGAAUGAAGGA-3' (SEQ ID NO: 312), 5'-AACAAAUUCAUUUUUCCUCUCCAAUUCUGCACAGAAAUUGCAGAACCCG AAUAGACGAAUGAAGGA-3' (SEQ ID NO: 313), and 5'-AACAAAUUCAUUUUUCCUCUCCAAUUCUGCACAAGAAAGUUGCAGAACC CGAAUAGACGAAUGAAGGA-3' (SEQ ID NO: 314); and
      5'-AUGCAAC-3'.

### ADVANTAGEOUS EFFECTS OF DISCLOSURE

In a case where a CRISPR/Cas12f1 system, which comprises the engineered Cas12f1 guide RNA provided herein having an engineered scaffold region, is used for gene editing, the CRISPR/Cas12f1 system exhibits higher gene editing efficiency than a naturally occurring CRISPR/Cas12f1 system.

### BRIEF DESCRIPTION OF DRAWINGS

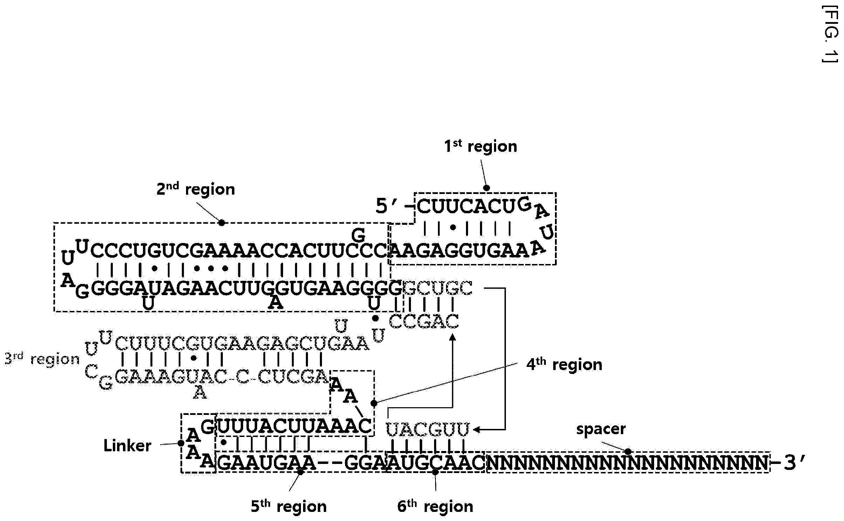

FIG. 1 is a schematic diagram illustrating an engineered Cas12f1 guide RNA disclosed herein.

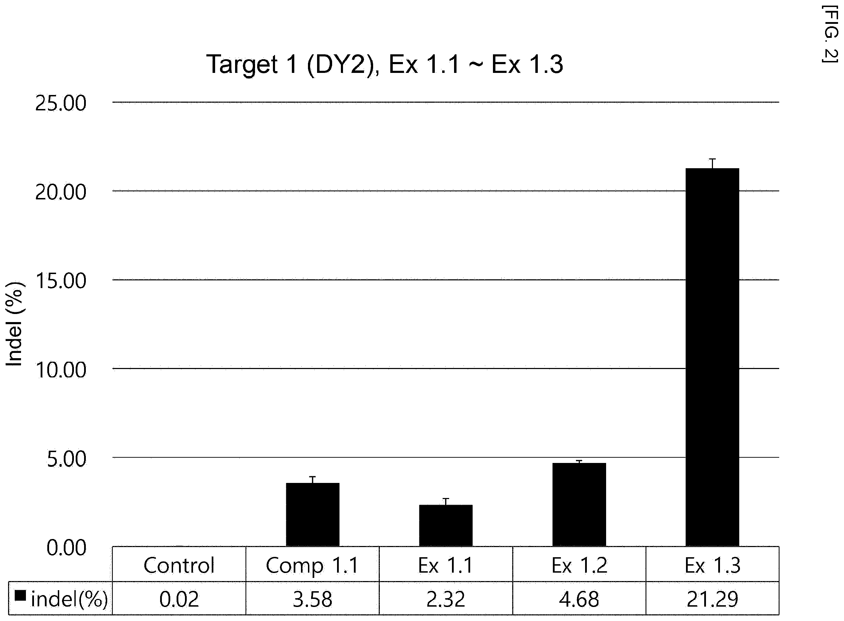

FIG. 2 is a graph showing average indel efficiency for Examples 1.1 to 1.3, which target DY2, among the examples disclosed in Experimental Example 2. Here, Ex is an abbreviation for Example, Comp is an abbreviation for Comparative Example, and Control refers to a negative control that is not treated with CRISPR/Cas12f1.

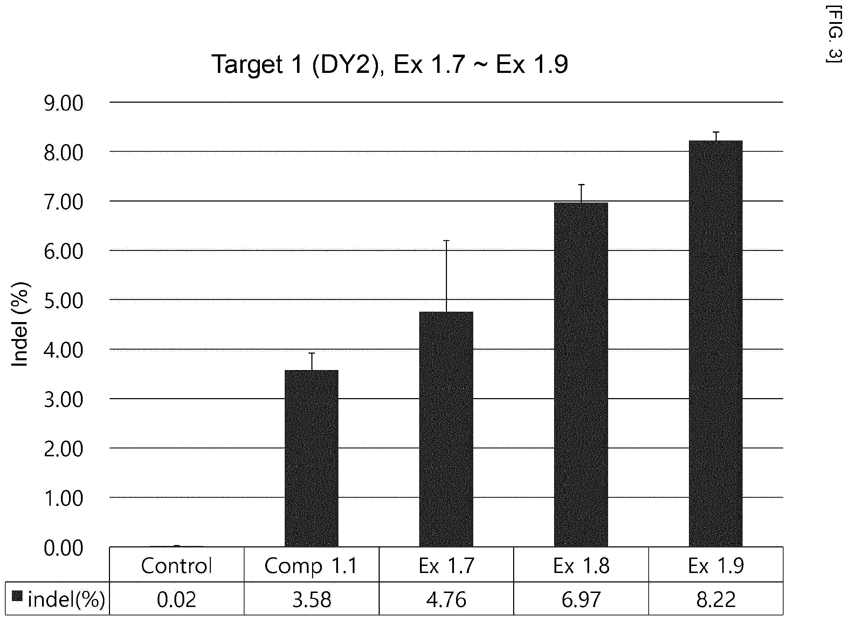

FIG. 3 is a graph showing average indel efficiency for Examples 1.7 to 1.9, which target DY2, among the examples disclosed in Experimental Example 2. Here, Ex is an abbreviation for Example, Comp is an abbreviation for Comparative Example, and Control refers to a negative control that is not treated with CRISPR/Cas12f1.

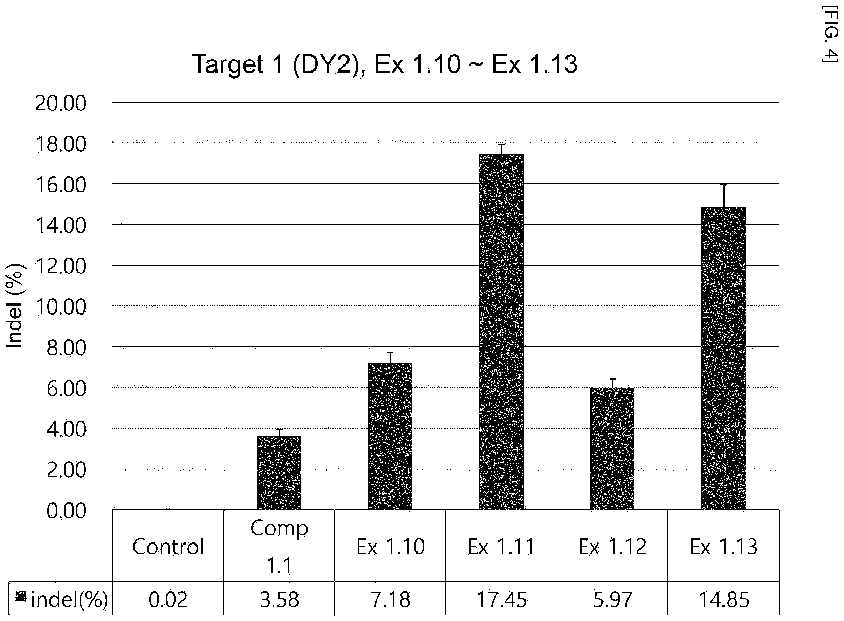

FIG. 4 is a graph showing average indel efficiency for Examples 1.11 to 1.13, which target DY2, among the examples disclosed in Experimental Example 2. Here, Ex is an abbreviation for Example, Comp is an abbreviation for Comparative Example, and Control refers to a negative control that is not treated with CRISPR/Cas12f1.

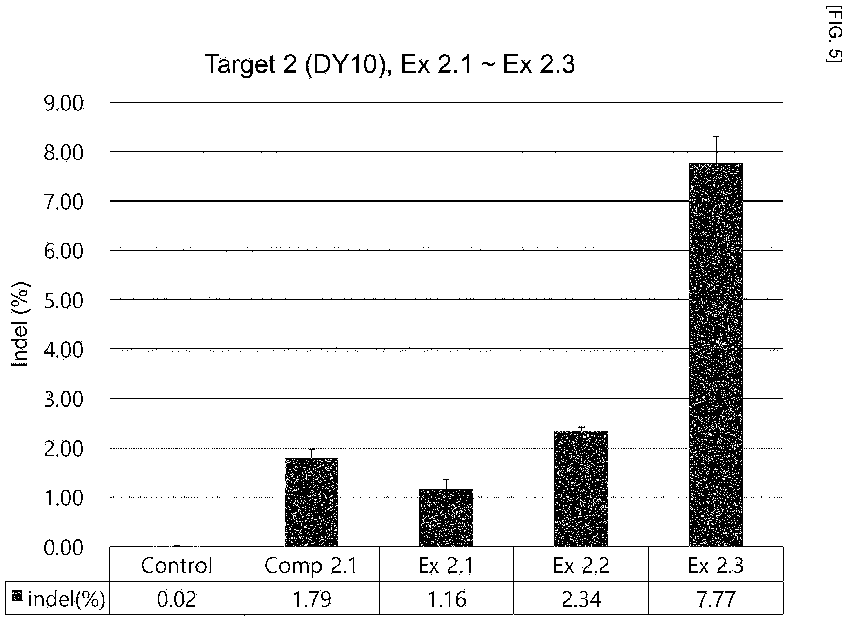

FIG. 5 is a graph showing average indel efficiency for Examples 2.1 to 2.3, which target DY10, among the examples disclosed in Experimental Example 2. Here, Ex is an abbreviation for Example, Comp is an abbreviation for Comparative Example, and Control refers to a negative control that is not treated with CRISPR/Cas12f1.

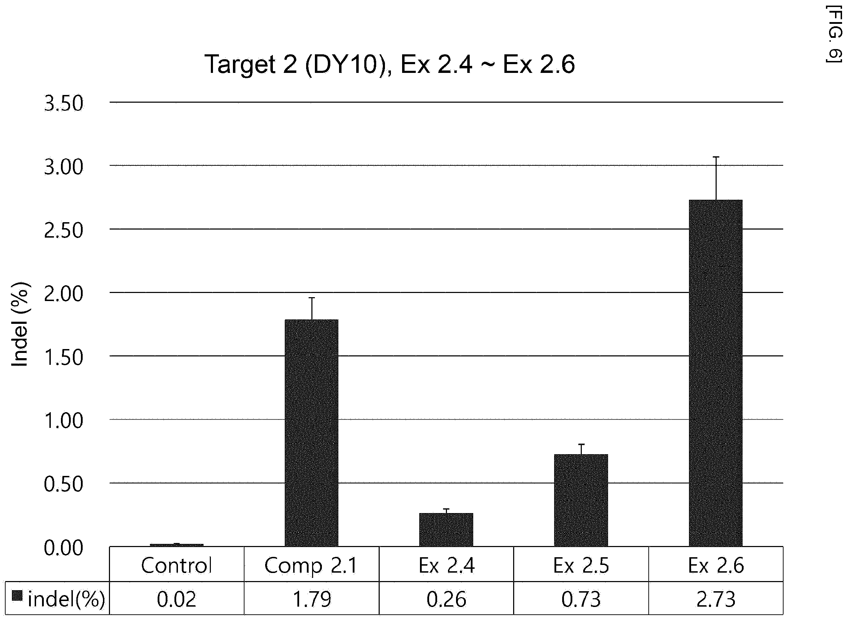

FIG. 6 is a graph showing average indel efficiency for Examples 2.4 to 2.6, target DY10, among the examples disclosed in Experimental Example 2. Here, Ex is an abbreviation for Example, Comp is an abbreviation for Comparative Example, and Control refers to a negative control that is not treated with CRISPR/Cas12f1.

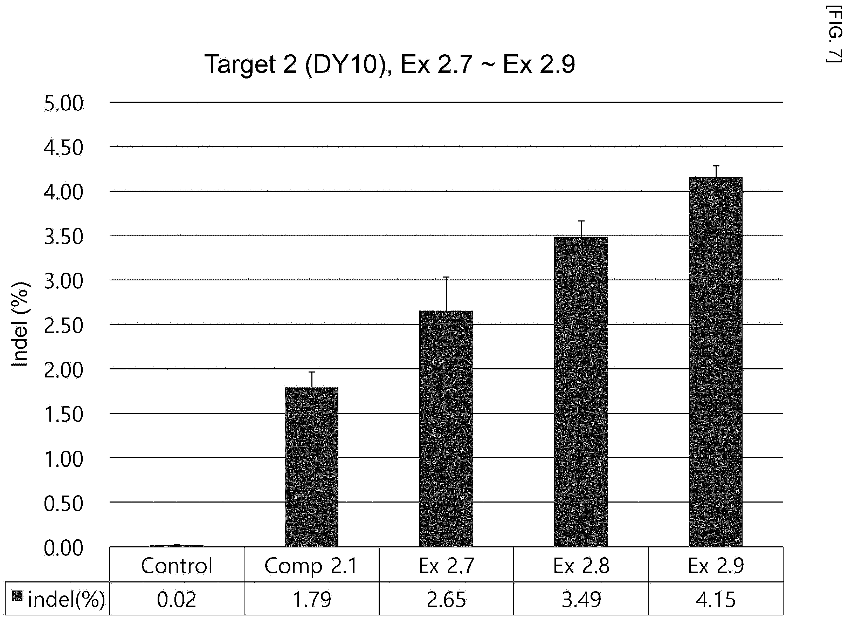

FIG. 7 is a graph showing average indel efficiency for Examples 2.7 to 2.9, target DY10, among the examples disclosed in Experimental Example 2. Here, Ex is an abbreviation for Example, Comp is an abbreviation for Comparative Example, and Control refers to a negative control that is not treated with CRISPR/Cas12f1.

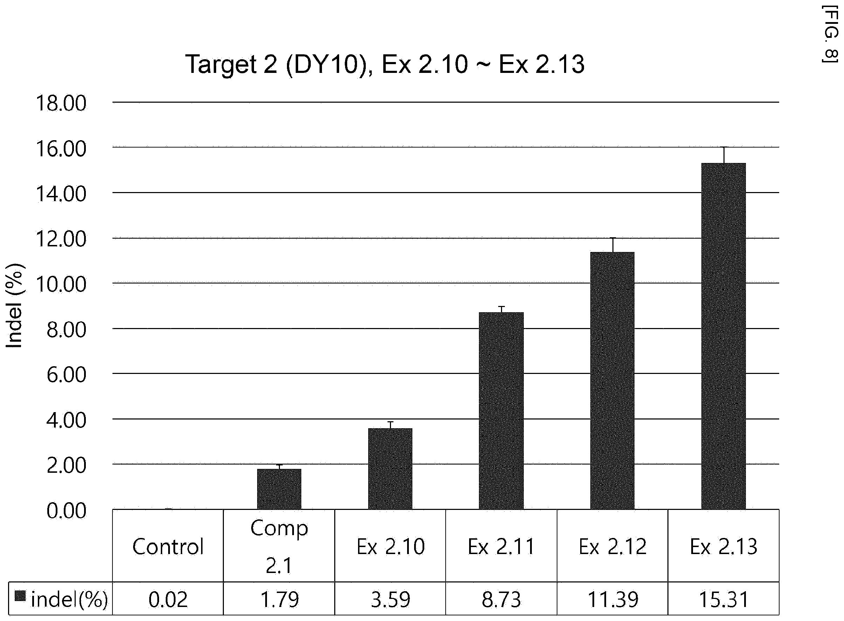

FIG. 8 is a graph showing average indel efficiency for Examples 2.10 to 2.13 targeting DY10 among the examples disclosed in Experimental Example 2. Here, Ex is an abbreviation for Example, Comp is an abbreviation for Comparative Example, and Control refers to a negative control that is not treated with CRISPR/Cas12f1.

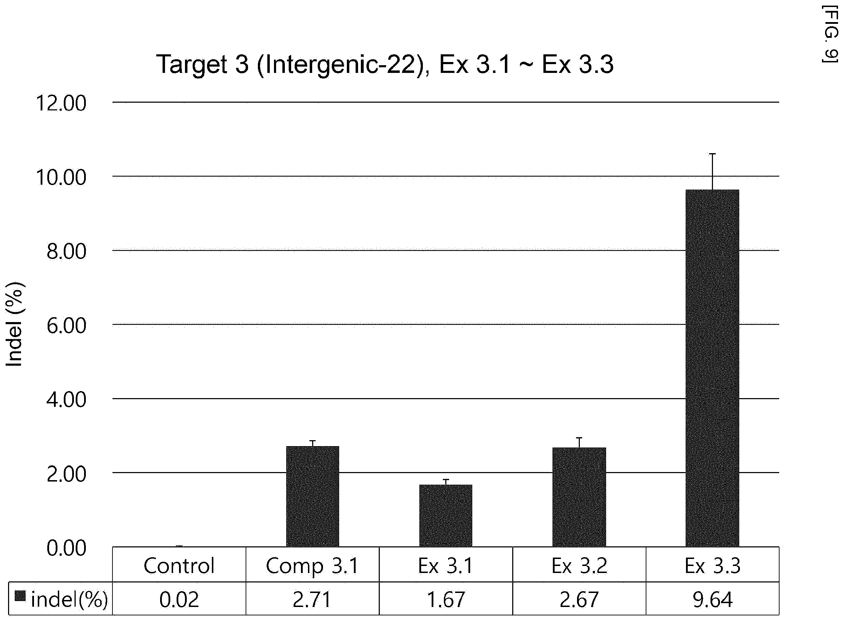

FIG. 9 is a graph showing average indel efficiency for Examples 3.1 to 3.3, which target Intergenic-22, among the examples disclosed in Experimental Example 2. Here, Ex is an abbreviation for Example, Comp is an abbreviation for Comparative Example, and Control refers to a negative control that is not treated with CRISPR/Cas12f1.

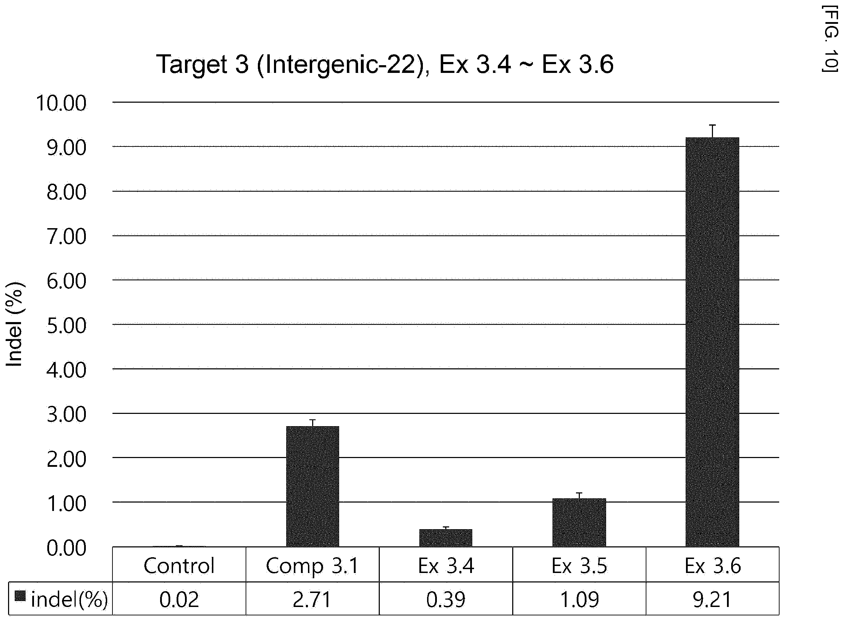

FIG. 10 is a graph showing average indel efficiency for Examples 3.4 to 3.6, which target Intergenic-22, among the examples disclosed in Experimental Example 2. Here, Ex is an abbreviation for Example, Comp is an abbreviation for Comparative Example, and Control refers to a negative control that is not treated with CRISPR/Cas12f1.

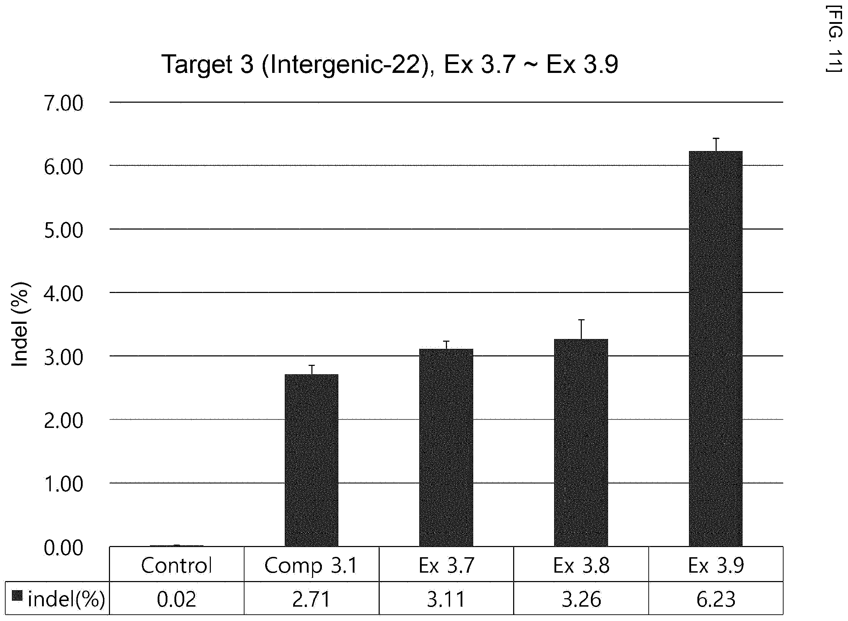

FIG. 11 is a graph showing average indel efficiency for Examples 3.7 to 3.9, which target Intergenic-22, among the examples disclosed in Experimental Example 2. Here, Ex is an abbreviation for Example, Comp is an abbreviation for Comparative Example, and Control refers to a negative control that is not treated with CRISPR/Cas12f1.

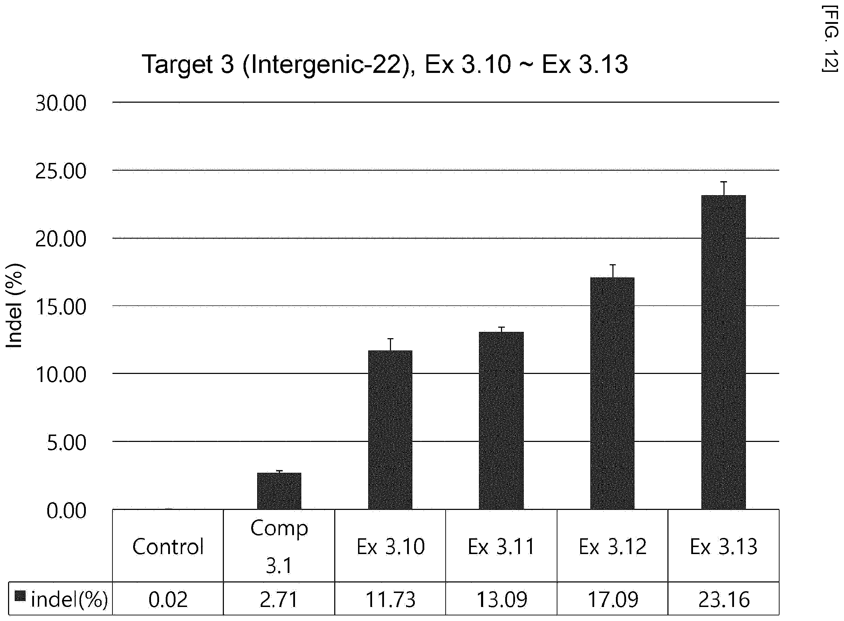

FIG. 12 is a graph showing average indel efficiency for Examples 3.10 to 3.13, which target Intergenic-22, among the examples disclosed in Experimental Example 2. Here, Ex is an abbreviation for Example, Comp is an abbreviation for Comparative Example, and Control refers to a negative control that is not treated with CRISPR/Cas12f1.

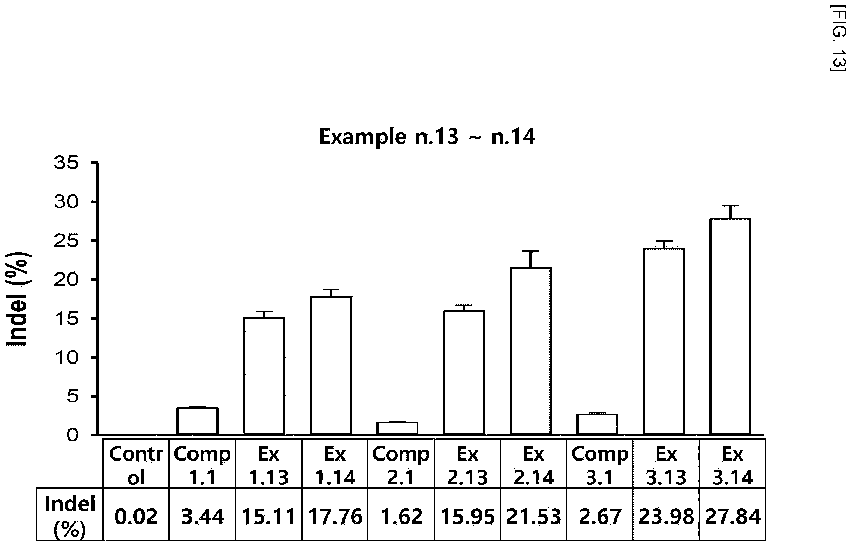

FIG. 13 shows average indel efficiency for Examples 1.13 to 1.14, which target DY2, Examples 2.13 to 2.14, which target DY10, and Examples 3.13 to 3.14, which target Intergenic-22, among the examples disclosed in Experimental Example 2. Here, Ex is an abbreviation for Example, Comp is an abbreviation for Comparative Example, and Control refers to a negative control that is not treated with CRISPR/Cas12f1.

### MODE OF DISCLOSURE

### Definition of Terms

### About

As used herein, the term "about" refers to an amount, level, value, number, frequency, percent, dimension, size, weight or length that varies by approximately 30%, 25%, 20%, 25%, 10%, 9%, 8%, 7%, 6%, 5%, 4%, 3%, 2% or 1% with respect to a reference amount, level, value, number, frequency, percent, dimension, size, weight or length.

### A, T, C, G, and U

As used herein, the symbols A, T, C, G, and U are interpreted as understood by those skilled in the art. It may be properly interpreted as a base, a nucleoside, or a nucleotide in DNA or RNA according to the context and description. For example, in a case where the symbols mean bases, they may be interpreted as adenine (A), thymine (T), cytosine (C), guanine (G), or uracil (U), respectively; in a case where the symbols mean nucleosides, they may be interpreted as adenosine (A), thymidine (T), cytidine (C), guanosine (G) or uridine (U), respectively; and in a case where the symbols mean nucleotides they may be interpreted to mean nucleotides including the respective nucleosides.

### Operably linked

As used herein, the term "operably linked" means that, in gene expression, a particular component is linked to another component so that the particular component can perform its intended function. For example, when a promoter sequence is operably linked to a coding sequence, it means that the promoter is linked thereto so as to affect transcription and/or expression of the coding sequence in a cell. In addition, the term includes all meanings recognized by those skilled in the art and may be appropriately interpreted according to the context.

### Target gene or target nucleic acid

As used herein, "target gene" or "target nucleic acid" basically means a gene or nucleic acid in a cell which becomes a target for gene editing. The target gene or target nucleic acid may be used interchangeably and may refer to the same target. Unless otherwise described, the target gene or target nucleic acid may refer to both a gene and nucleic acid inherent in a target cell or an externallyderived gene or nucleic acid, and is not particularly limited as long as it can be a target for editing. The target gene or target nucleic acid may contain a target sequence or a region adjacent thereto. The target gene or target nucleic acid may be single-stranded DNA, double-stranded DNA, and/or RNA. In addition, the term includes all meanings recognized by those skilled in the art and may be appropriately interpreted according to the context.

### Target sequence or recognition sequence

As used herein, "target sequence" or "recognition sequence" refers to a particular sequence recognized by a CRISPR/Cas complex to cleave a target gene or target nucleic acid. The target sequence may be appropriately selected depending on the purpose. Specifically, the "target sequence" may be a sequence included in the above-described target gene or target nucleic acid and refers to a sequence having complementarity with a spacer sequence included in the guide RNA or the engineered guide RNA provided herein. In general, the spacer sequence is determined in consideration of a sequence of a target gene or target nucleic acid and a protospacer adjacent motif (PAM) sequence recognized by an effector protein of a CRISPR/Cas system. In the present disclosure, the target sequence may refer only to a specific strand complementarily binding to a guide RNA of a CRISPR/Cas complex, or may refer to an entire target double strand including the specific strand portion. The term may be interpreted appropriately depending on the context. In addition, the term includes all meanings recognized by those skilled in the art, and may be appropriately interpreted according to the context.

### Vector

As used herein, unless otherwise specified, the "vector" refers collectively to any material capable of transporting a genetic material into a cell. For example, a vector may be a DNA molecule including a genetic material of interest, for example, a nucleic acid encoding an effector protein of a CRISPR/Cas system, and/or a nucleic acid encoding a guide RNA, however, the vector is not limited thereto. The term includes all meanings recognized by those skilled in the art and may be appropriately interpreted according to the context.

### Naturally occurring

As used herein, the term "naturally occurring" refers to an object that is found in nature and is not artificially modified. The term is used to distinguish it from an "engineered object" obtained by artificial modification. The "naturally occurring" gene, nucleic acid, DNA, RNA, and the like are used as concepts that encompass all genes, nucleic acids, DNA, and RNA in wild type (original form) and mature form (active form). The term includes all meanings recognized by those skilled in the art and should be appropriately interpreted according to the context.

### Engineered

As used herein, the term "engineered" is used to distinguish it from a material, a molecule, or the like whose configuration already exists in nature, and this means that the material, a molecule, or the like has undergone artificial modification For example, the "engineered guide RNA" refers to a guide RNA obtained by applying artificial modification to the configuration of a naturally occurring guide RNA. In addition, the term includes all meanings recognized by those skilled in the art and may be appropriately interpreted according to the context.

### Nuclear localization sequence or signal (NLS)

In a case where a substance outside the cell's nucleus is transported into the nucleus by nuclear transport, the term "NLS" as used herein refers to a peptide of a certain length or a sequence thereof, wherein the peptide is attached to a protein to be transported and acts as a type of "tag." Specifically, the NLS may be, but is not limited to, an NLS sequence derived from: the NLS of an SV40 virus large T-antigen having the amino acid sequence PKKKRKV (SEQ ID NO: 277); the NLS from a nucleoplasmin (for example, the nucleoplasmin bipartite NLS having the sequence KRPAATKKAGQAKKKK (SEQ ID NO: 278)); the c-myc NLS having the amino acid sequence PAAKRVKLD (SEQ ID NO: 279) or RQRRNELKRSP (SEQ ID NO: 280); the hRNPA1 M9 NLS having the sequence NQSSNFGPMKGGNFGGRSSGPYGGGGQYFAKPRNQGGY (SEQ ID NO: 281); the sequence RMRIZFKNKGKDTAELRRRRVEVSVELRKAKKDEQILKRRNV (SEQ ID NO: 282) of an IBB domain from importin alpha; the sequences VSRKRPRP (SEQ ID NO: 283) and PPKKARED (SEQ ID NO: 284) of myoma T protein; the sequence PQPKKKPL (SEQ ID NO: 285) of human p53; the sequenceSALIKKKKKMAP (SEQ ID NO: 286) of mouse c-abl IV; the sequences DRLRR (SEQ ID NO: 287) and PKQKKRK (SEQ ID NO: 288) of influenza virus NS1; the sequence RKLKKKIKKL (SEQ ID NO: 289) of hepatitis virus delta antigen; the sequence REKKKFLKRR (SEQ ID NO: 290) of mouse Mx1 protein; the sequence KRKGDEVDGVDEVAKKKSKK (SEQ ID NO: 291) of human poly (ADP-ribose) polymerase; or the sequence RKCLQAGMNLEARKTKK (SEQ ID NO: 292) of a steroid hormone receptor (human) glucocorticoid. As used herein, the term "NLS" includes all meanings recognized by those skilled in the art and may be appropriately interpreted according to the context.

### Nuclear export sequence, or signal (NES)

In a case where a substance inside the cell's nucleus is transported outside the nucleus by nuclear transport, the term "NES" as used herein refers to a peptide of a certain length or a sequence thereof wherein the peptide is attached to a protein to be transported and acts as a type of "tag." As used herein, the term "NES" includes all meanings recognized by those skilled in the art, and may be appropriately interpreted according to the context.

### Tag

As used herein, the term "tag" refers collectively to a functional domain added to facilitate transport, tracking and/or separation and purification of a peptide or protein. Specifically, the tag includes, but is not limited to, tag proteins such as a histidine (His) tag, a V5 tag, a FLAG tag, an influenza hemagglutinin (HA) tag, an Myc tag, a VSV-G tag, and a thioredoxin (Trx) tag; autofluorescent proteins such as a green fluorescent protein (GFP), a yellow fluorescent protein (YFP), a cyan fluorescent protein (CFP), a blue fluorescent protein (BFP), HcRED, and DsRed; and reporter proteins such as a glutathione-S-transferase (GST), a horseradish peroxidase (HRP), a chloramphenicol acetyltransferase (CAT) betagalactosidase, a beta-glucuronidase, and a luciferase. As used herein, the term "tag" includes all meanings recognized by those skilled in the art and may be appropriately interpreted according to the context.

### Background art - Structure of CRISPR/Cas12f1 complex

### CRISPR/Cas12f system

A CRISPR/Cas12f system belongs to a V-F subtype among type V CRISPR/Cas systems, which is further divided into V-F1 to V-F3 variants. The CRISPR/Cas12f system includes a CRISPR/Cas14 system including Cas14a, Cas14b, and Cas14c variants, among the effector proteins named Cas14 in a previous study (Harrington et al., Programmed DNA destruction by miniature CRISPR-Cas14 enzymes, Science 362, 839-842 (2018)). Among them, the CRISPR/Cas14a system including a Cas14a effector protein is classified as a CRISPR/Cas12f1 system (Makarova et al., Nature Reviews, Microbiology volume 18, 67 (2020)). Recent previous studies (Takeda et al., Structure of the miniature type V-F CRISPR-Cas effector enzyme, Molecular Cell 81, 1-13 (2021), Xiao et al., Structural basis for the dimerization-dependent CRISPR-Cas12f nuclease, bioRxiv (2020)), etc. have revealed a structure of the CRISPR/Cas12f1 complex, which will be briefly described below.

### Structure of CRISPR/Cas12f1 complex

It was found that within the CRISPR/Cas12f1 complex, two molecules of the Cas12f1 protein, which are in a form of a dimer, bind to a guide RNA to form a complex. The Cas12f1 protein is divided into an amino-terminal domain (NTD) and a carboxy-terminal domain (CTD) and has a structure in which the two domains are linked by a linker loop. The NTD consists of wedge (WED), recognition (REC), and zinc finger (ZF) domains, and the CTD consists of another ZF domain and an RuvC domain. The structure of the Cas12f1 dimeric protein may be largely divided into a recognition (REC) lobe and a nuclease (NUC) lobe. The REC lobe consists of: a WED domain, a ZF domain, and a REC domain of one Cas12f1 protein constituting the dimer; and a WED domain, a ZF domain, and a REC domain of the other Cas12f1 protein constituting the dimer. The nuclease lobe consists of: an RuvC domain and a TNB domain of one Cas12f1 protein constituting the dimer; and an RuvC domain and a TNB domain of the other Cas12f1 protein constituting the dimer. All or part of each domain of the Cas12f1 protein respectively recognizes a certain portion of the scaffold region of the Cas12f1 guide RNA and forms a CRISPR/Cas12f1 complex.

### Structure of Cas12f1 guide RNA

In the present disclosure, the Cas12f1 guide RNA may be largely divided into a spacer region and a scaffold region by function. The scaffold region consists of five stems (named Stems 1 to 5) and one pseudoknot (PK).

Generally, it is well known to those skilled in the art that a naturally occurring Cas12f1 guide RNA is divided into a tracrRNA and a crRNA, in which the crRNA may be further divided into a crRNA repeat sequence portion and a spacer. Apart from the above criteria, in the present disclosure, parts of the Cas12f1 guide RNA, which interact with the Cas12f1 protein, are collectively referred to as a scaffold region. The scaffold region is a region comprising a tracrRNA and a portion of a crRNA, and functions to interact with a Cas12f1 protein. A detailed description thereof will be provided in the corresponding section below.

The Cas12f1 guide RNA comprises two structures in which a part of tracrRNA (tracrRNA anti-repeat) and a part of a crRNA repeat portion are complementarily bound to form a duplex, and the structure is named a crRNA repeat-tracrRNA anti-repeat (R:AR) portion. The Stem 5 (R:AR2), and PK (R:AR1) form this crRNA repeat-tracrRNA anti-repeat duplex structure. In the CRISPR/Cas12f1 complex, Stem 1 portion, a part of Stem 2, and Stem 5 (R:AR2) portion in the Cas12f1 guide RNA were found not to interact with the Cas12f1 dimer, and these are called a disordered region.

### Background art - Design of vector expressing CRISPR/Cas system

### Overview of vector design

In order to use the CRISPR/Cas system for gene editing, a method is widely used in the art that introduces a vector, which has sequences encoding respective components of the CRISPR/Cas system, into a cell so that the respective components of the CRISPR/Cas system are expressed in the cell. Hereinafter, the components of the vector, which allow the CRISPR/Cas system to be expressed in a cell, will be described.

### Nucleic acids encoding components of CRISPR/Cas system

Since the purpose of the vector is to express respective components of the CRISPR/Cas system in a cell, a sequence of the vector may comprise one or more nucleic acids encoding the respective components of the CRISPR/Cas system. Specifically, the sequence of the vector comprises nucleic acids encoding a guide RNA and/or a Cas protein which are included in the CRISPR/Cas system to be expressed. Here, a sequence of the vector may comprise nucleic acids encoding an engineered Cas12f1 guide RNA and a codon-optimized Cas protein or a nucleic acid encoding an engineered Cas protein according to the purpose, as well as nucleic acids encoding a wildtype guide RNA and a wildtype Cas protein. Here, the nucleic acid sequence encoding each component may be a DNA sequence.

### Regulatory/control element

In order to express the component in a cell, the vector needs to contain one or more regulatory/control elements. Specifically, the regulatory/control element may include, but is not limited to, a promoter, an enhancer, an intron, a polyadenylation signal, a Kozak consensus sequence, an internal ribosome entry site (IRES), a splice acceptor, a 2A sequence and/or a replication origin. Here, the replication origin may be, but is not limited to, an f1 origin of replication, an SV40 origin of replication, a pMB1 origin of replication, an adeno origin of replication, an AAV origin of replication, and/or a BBV origin of replication.

### Promoter

In order to cause the vector to express its target in a cell, a promoter sequence needs to be operatively linked to a sequence encoding each component so that a transcription factor can be activated in the cell. The promoter sequence may be designed differently depending on the corresponding transcription factor or expression environment and is not limited as long as it may properly express the components of the CRISPR/Cas system in a cell. The promoter sequence may be a promoter that promotes activation of an RNA polymerase (for example, RNA Pol I, Pol II, or Pol III). For example, the promoter may be, but is not limited to, one selected from: an SV40 early promoter, a mouse mammary tumor virus long terminal repeat (LTR) promoter, an adenovirus major late promoter (Ad MLP), a herpes simplex virus (HSV) promoter, a cytomegalovirus (CMV) promoter such as a CMV immediate early promoter region (CMVIE), a rous sarcoma virus (RSV) promoter, a human U6 small nuclear promoter (U6) (Miyagishi et al., Nature Biotechnology 20, 497 - 500 (2002)), an enhanced U6 promoter (e.g., Xia et al., Nucleic Acids Res.2003 Sep 1:31(17)), a human H1 promoter (H1), and a 7SK promoter.

### Termination signal

A sequence, which induces termination of transcriptional activity of the transcription factor is referred to as a termination signal. The termination signal may vary depending on the type of promoter sequence. For example, when the promoter is a U6 or H1 promoter, the promoter recognizes a thymidine repeat sequence (for example, a TTTTTT (T6) sequence) as a termination signal.

### Additionally expressed element

The vector may comprise a nucleic acid sequence encoding an additionally expressed element which those skilled in the art intend to express as necessary, in addition to components of a wildtype CRISPR/Cas system and/or an engineered CRISPR/Cas system. For example, the additionally expressed elements may be one of the tags described in the section "Tag," but are not limited thereto. For example, the additionally expressed element may be a gene that may have a desired additional function, such as a selection marker for selection, for example, a herbicide resistance gene such as glyphosate, glufosinate ammonium or phosphinothricin, or an antibiotic resistance gene such as ampicillin, kanamycin, G418, bleomycin, hygromycin, and chloramphenicol, but is not limited thereto.

### Form of expression vector

The expression vector may be designed in a form of a linear or circular vector.

### Limitations of prior art

The CRISPR/Cas12f1 system is an attractive system for gene editing technology due to its relatively small size. Since it was first reported (Harrington et al., Programmed DNA destruction by miniature CRISPR-Cas14 enzymes, Science 362, 839-842 (2018)), several studies thereon have been conducted (Harrington et al., Programmed DNA destruction by miniature CRISPR-Cas14 enzymes, Science 362, 839-842 (2018), US 2020/0190494 A1). However, the CRISPR/Cas12f1 system shows no or too low gene editing activity in cells (for example, eukaryotic cells), which is an obstacle to its utilization.

As a result of continuing studies, a recent prior literature (Takeda et al., Structure of the miniature type V-F CRISPR-Cas effector enzyme, Molecular Cell 81, 1-13 (2021)) revealed the fact that a Cas12f1 protein forms a dimer in the CRISPR/Cas12f1 system, and a structure of its guide RNA. It was found by the prior literature that there is a so-called disordered region in the guide RNA that does not directly interact with the Cas12f1 protein. In another prior literature (Xiao et al., Structural basis for the dimerization-dependent CRISPR-Cas12f nuclease, bioRxiv (2020)), the disordered region was removed and double-stranded DNA cleavage efficiency of the system was investigated *in vitro.* However, 1) the preceding studies did not show intracellular gene editing activity, nor were related to methods of increasing intracellular gene editing activity (for example, indel generation efficiency), 2) among the studies, there is an experiment in which the disordered region was removed; however, it was reported in this experiment that the cleavage activity was even lowered; moreover, 3) the experiment performed in the above study was performed *in vitro,* and failed to reveal what modifications exhibit intracellular gene editing activity or increase efficiency.

Therefore, enhancing the intracellular gene editing activity of the CRISPR/Cas12f1 system is still an important task.

### Characteristics of engineered Cas12f1 guide RNA

### Overview of engineered Cas12f1 guide RNA

In the present disclosure, there is provided an engineered Cas12f1 guide RNA for increasing intracellular gene editing activity of a CRISPR/Cas12f1 system by overcoming limitations of the prior art. The engineered Cas12f1 guide RNA is a modified form of a naturally occurring guide RNA whose structure is partially modified. The engineered Cas12f1 guide RNA is characterized in that at least a portion of the scaffold region, which serves to interact with a Cas12f1 protein, is modified.

In an embodiment, the engineered Cas12f1 guide RNA may comprise an engineered scaffold region and a spacer. Here, the engineered scaffold region is characterized by being different from a scaffold region of a naturally occurring guide RNA.

### Characteristics of engineered Cas12f1 guide RNA - One or more parts of scaffold region being modified

The engineered Cas12f1 guide RNA provided herein is characterized in that a part of its scaffold region is modified as compared with a naturally occurring guide RNA. The scaffold region is a region comprising a tracrRNA and a part of a crRNA and has a function of interacting with a Cas12f1 protein. The scaffold region will be described in more detail below.

In an embodiment, the engineered Cas12f1 guide RNA may comprise an engineered scaffold region. Here, the engineered scaffold region is obtained by modifying a scaffold region of a naturally occurring guide RNA. Therefore, the engineered scaffold region has a sequence different from the scaffold region of the naturally occurring guide RNA. In an embodiment, the engineered scaffold region may be obtained by removing a part of a scaffold region of a naturally occurring guide RNA. In an embodiment, the engineered scaffold region may be obtained by removing one or more nucleotides from a certain part included in a scaffold region of a naturally occurring guide RNA.

### Effect of engineered Cas12f1 guide RNA

When the engineered Cas12f1 guide RNA provided herein is used in the CRISPR/Cas12f1 system, gene editing activity of the system is dramatically improved in a cell as compared with when a naturally occurring guide RNA is used. The present inventors revealed in detail through experiments what components need to be added to a naturally occurring guide RNA or what modifications need to be applied to its scaffold region, to improve its gene editing efficiency. Use of the engineered Cas12f1 guide RNA makes it possible to edit a gene in a cell with high efficiency by overcoming limitations of the prior art. In addition, the engineered Cas12f1 guide RNA has a length equal to or shorter than a naturally occurring guide RNA, and thus has a high potential for application in the field of gene editing technology. The engineered Cas12f1 guide RNA of the present application makes it possible to fully utilize the advantages of the CRISPR/Cas12f1 system (for example, the advantage of having a very small size) in gene editing technology.

### Uses of engineered Cas12f1 guide RNA

The engineered Cas12f1 guide RNA provided herein may be used for gene editing and/or gene therapy together with a Cas12f1 protein. In addition, the engineered Cas12f1 guide RNA may be used for preparing a composition for gene editing.

### Explanation of Terms - Terms referring to parts of Cas12f1 guide RNA

### Parts of Cas12f1 guide RNA - Overview

It is well known to those skilled in the art that the naturally occurring Cas12f1 guide RNA is divided into a tracrRNA and a crRNA, in which the crRNA may be further divided into a crRNA repeat sequence portion and a spacer.

Apart from the above criteria, in the present disclosure, parts of the Cas12f1 guide RNA, which interacts with a Cas12f1 protein, are collectively referred to as a scaffold region. The scaffold region comprises a tracrRNA and a part of a crRNA and may not refer to a single molecule of RNA. The scaffold region may be further subdivided into a first region, a second region, a third region, a fourth region, a fifth region, and a sixth region (FIG. 1). When the subdivided regions are described with respect to the tracrRNA and crRNA, the tracrRNA comprises the first to fourth regions and the crRNA, in particular the crRNA repeat sequence portion, comprises the fifth to the sixth regions.

The "n-th region" or "naturally occurring n-th region" (n is an integer of between 1 and 6 inclusive) as described below refers to each part of the naturally occurring Cas12f1 guide RNA. Specifically, the "n-th region" may refer to each region corresponding to a structure of a guide RNA transcribed in a prokaryotic cell, and/or a structure of a guide RNA that actually operates in a prokaryotic system (which is in an activated form, not a transcribed form, in a prokaryotic cell). The region in an engineered Cas12f1 guide RNA, which corresponds to the above classification criteria, is generally described as "modified n-th region" or "n-th region of an engineered scaffold region."

However, there may be a case where even an n-th region included in an engineered scaffold region is not modified and thus is identical to a naturally occurring n-th region; and only in that case, the term "n-th region" may be used interchangeably. Here, what is referred to by the "n-th region" (for example, whether it is a region included in an engineered Cas12f1 guide RNA or a region included in a naturally occurring guide RNA) should be appropriately interpreted according to the context.

### tracrRNA, crRNA

As used herein, the terms "tracrRNA" and "crRNA" include all meanings that can be recognized by those skilled in the field of CRISPR/Cas technology. The terms are generally used to refer to respective molecules of a naturally occurring dual guide RNA and may also be used to refer to respective corresponding parts of a single guide RNA in which a tracrRNA and a crRNA are linked by a linker. Unless otherwise specified, in a case of being merely written as "tracrRNA" and "crRNA," the terms refer to a tracrRNA and a crRNA constituting a CRISPR/Cas12f1 system, respectively.

In an embodiment, a sequence of the tracrRNA may be 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUU-3' (SEQ ID NO: 1) or 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAA-3' (SEQ ID NO: 2). In an embodiment, the tracrRNA comprises a first region, a second region, a third region, and a fourth region. In an embodiment, the tracrRNA is one in which the first region, the second region, the third region, and the fourth region are sequentially linked to each other in a 5' to 3' direction.

In an embodiment, a sequence of the crRNA comprises a crRNA repeat sequence and a spacer sequence. Here, the crRNA repeat sequence may be 5'-GAAUGAAGGAAUGCAAC-3' (SEQ ID NO: 3) or 5'-GUUGCAGAACCCGAAUAGACGAAUGAAGGAAUGCAAC-3' (SEQ ID NO: 4). The crRNA repeat sequence comprises a fifth region and a sixth region. The spacer sequence may vary depending on a target sequence, and generally comprises 10 to 50 nucleotides. In an embodiment, the crRNA is one in which the fifth region, the sixth region, and the spacer are sequentially linked to each other in a 5' to 3' direction.

### Scaffold region - Overview

As used herein, the term "scaffold region" refers collectively to the rest of a naturally occurring guide RNA excluding the spacer. Specifically, the scaffold region comprises the tracrRNA, and a part of the crRNA. Specifically, the part of the crRNA may be a crRNA repeat sequence portion. The scaffold region is generally known as a portion capable of interacting with a Cas protein. In the present disclosure, the scaffold region is divided into first to sixth regions for description, and each region will be described in more detail below.

### Scaffold region 1 - First region

As used herein, the term "first region" refers to a region comprising the 5' end of the tracrRNA. The first region may comprise nucleotides forming a stem structure in the CRISPR/Cas12f1 complex and may comprise nucleotides adjacent thereto.

The first region comprises a Stem 1 portion (Takeda et al., Structure of the miniature type V-F CRISPR-Cas effector enzyme, Molecular Cell 81, 1-13 (2021)). The first region may comprise one or more nucleotides adjacent to the Stem 1 portion.

The first region comprises a disordered region that does not interact with a Cas12f1 protein in the CRISPR/Cas12f1 complex.

In an embodiment, the first region may refer to the 1^{st} to 11^{th} nucleotides from the 5' end of the tracrRNA represented by SEQ ID NO: 1 or 2. In an embodiment, a sequence of the first region may be 5'-CUUCACUGAUAAAGUGGAGAA-3' (SEQ ID NO: 9).

### Scaffold region 2 - Second region

As used herein, the term "second region" refers to a region located at the 3' end of the first region in the tracrRNA. The second region may comprise nucleotides forming a stem structure in a CRISPR/Cas12f1 complex, and may comprise nucleotides adjacent thereto. Here, the stem structure is different from the stem included in the first region.

The second region comprises a Stem 2 portion (Takeda et al., Structure of the miniature type V-F CRISPR-Cas effector enzyme, Molecular Cell 81, 1-13 (2021)). The second region may comprise one or more nucleotides adjacent to the Stem 2 portion.

The second region may comprise one or more nucleotides that interact with an RuvC domain of one dimer-forming Cas12f1 protein and/or an RuvC domain of the other dimer-forming Cas12f1 protein in the CRISPR/Cas12f1 complex. The second region comprises a disordered region that does not interact with a Cas12f1 protein in the CRISPR/Cas12f1 complex.

In an embodiment, the second region may refer to the 22^{nd} to 72^{nd} nucleotides from the 5' end of the tracrRNA represented by SEQ ID NO: 1 or 2. In an embodiment, a sequence of the second region may be 5'-CCGCUUCACCAAAAGCUGUCCCUUAGGGGAUUAGAACUUGAGUGAAGGU G-3' (SEQ ID NO: 10).

### Scaffold region 3 - Third region

As used herein, the term "third region" refers to a region located at the 3' end of the second region in a tracrRNA. The third region may comprise nucleotides forming a stem structure in the CRISPR/Cas12f1 complex and nucleotides forming complementary bonds with some nucleotides included in the crRNA and may comprise nucleotides adjacent thereto.

The third region comprises nucleotides, which belong to the tracrRNA, in a Stem 4 portion (Takeda et al., Structure of the miniature type V-F CRISPR-Cas effector enzyme, Molecular Cell 81, 1-13 (2021)) and a Stem 3-PK (R:AR-1) portion (Takeda et al., Structure of the miniature type V-F CRISPR-Cas effector enzyme, Molecular Cell 81, 1-13 (2021)). The third region may comprise one or more nucleotides adjacent to the nucleotides, which belong to the tracrRNA, in the Stem 4 portion and/or the Stem 3-PK (R:AR-1) portion.

The third region comprises one or more nucleotides that interact with a WED domain and/or an RuvC domain of one dimer-forming Cas12f1 protein in the CRISPR/Cas12f1 complex. Here, the nucleotides may be nucleotides, which belong to the tracrRNA, in the Stem 3-PK (R:AR-1) portion.

The third region comprises one or more nucleotides that interact with an RuvC domain of one dimer-forming Cas12f1 protein and/or a REC domain of the other dimer-forming Cas12f1 protein in the CRISPR/Cas12f1 complex. Here, the nucleotides may be nucleotides from the Stem 4 portion.

The third region may comprise one or more nucleotides complementarily binding to one or more nucleotides included in the sixth region of the crRNA.

In an embodiment, the third region may refer to the 72^{nd} to 127^{th} nucleotides from the 5' end of the tracrRNA represented by SEQ ID NO: 1 or 2. In an embodiment, a sequence of the third region may be 5'-GCUGCUUGCAUCAGCCUAAUGUCGAGAAGUGCUUUCUUCGGAAAGUAAC CCUCGA-3' (SEQ ID NO: 11).

### Scaffold region 4 - Fourth region

As used herein, the term "fourth region" refers to a region located at the 3' end of the third region in the tracrRNA. The fourth region may comprise nucleotides capable of forming complementary bonds with some nucleotides included in the crRNA in the CRISPR/Cas12f1 complex and may comprise nucleotides adjacent thereto.

The fourth region comprises nucleotides, which belong to the tracrRNA, in Stem 5 (R:AR-2) (Takeda et al., Structure of the miniature type V-F CRISPR-Cas effector enzyme, Molecular Cell 81, 1-13 (2021)). The fourth region may comprise one or more nucleotides adjacent to the nucleotides, which belong to the tracrRNA, in Stem 5 (R:AR-2).

The fourth region comprises one or more nucleotides that interact with a WED domain and/or a ZF domain of one dimer-forming Cas12f1 protein in the CRISPR/Cas12f1 complex. Here, the nucleotides may be nucleotides, which belong to the tracrRNA, in Stem 5 (R:AR-2).

The fourth region may comprise one or more nucleotides complementarily binding to one or more nucleotides included in the fifth region of the crRNA. The fourth region includes a disordered region that does not interact with a Cas12f1 protein in a CRISPR/Cas12f1 complex.

In an embodiment, the fourth region may refer to the 128^{th} to 140^{th} nucleotides from the 5' end of the tracrRNA represented by SEQ ID NO: 1. In an embodiment, the fourth region may refer to the 128^{th} to 162^{nd} nucleotides from the 5' end of the tracrRNA represented by SEQ ID NO: 2.

In an embodiment, a sequence of the fourth region may be 5'-AACAAAUUCAUUU-3' (SEQ ID NO: 12) or 5'-AACAAAUUCAUUUUUCCUCUCCAAUUCUGCACAA-3' (SEQ ID NO: 13).

### Scaffold region 5 - Fifth region

As used herein, the term "fifth region" refers to a region including the 5' end of the crRNA. The fifth region may comprise nucleotides that form complementary bonds with one or more nucleotides of the fourth region in a CRISPR/Cas12f1 complex and may comprise any nucleotide adjacent thereto.

The fifth region comprises nucleotides, which belong to the crRNA, in a Stem 5 (R:AR-2) portion (Takeda et al., Structure of the miniature type V-F CRISPR-Cas effector enzyme, Molecular Cell 81, 1-13 (2021)). The fifth region may comprise one or more nucleotides adjacent to the nucleotides, which belong to the crRNA, in the Stem 5 (R:AR-2) portion.

The fifth region comprises one or more nucleotides that interact with a WED domain, a REC domain, and/or a ZF domain of one dimer-forming Cas12f1 protein in a CRISPR/Cas12f1 complex. Here, the nucleotides may be nucleotides, which belong to the crRNA, in Stem 5 (R:AR-2).

The fifth region may comprise one or more nucleotides complementarily binding to one or more nucleotides included in the fourth region. The fifth region includes a disordered region that does not interact with a Cas12f1 protein, in the CRISPR/Cas12f1 complex.

In an embodiment, the fifth region may refer to the 1^{st} to 10^{th} nucleotides from the 5' end of the crRNA represented by SEQ ID NO: 3. In an embodiment, the fifth region may refer to the 1^{st} to 30^{th} nucleotides from the 5' end of the crRNA represented by SEQ ID NO: 4. In an embodiment, a sequence of the fifth region may be 5'-GAAUGAAGGA-3' (SEQ ID NO: 14) or 5'-GUUGCAGAACCCGAAUAGACGAAUGAAGGA-3' (SEQ ID NO: 15).

### Scaffold region 6 - Sixth region

As used herein, the term "sixth region" refers to a region located at the 3' end of the fifth region in the crRNA. The sixth region may comprise nucleotides that form complementary bonds with one or more nucleotides of the third region in a CRISPR/Cas12f1 complex and may comprise any nucleotide adjacent thereto.

The sixth region comprises nucleotides, which belong to the crRNA, in a Stem 3-PK (R:AR-1) portion (Takeda et al., Structure of the miniature type V-F CRISPR-Cas effector enzyme, Molecular Cell 81, 1-13 (2021)). The sixth region may comprise any one or more nucleotides adjacent to the nucleotides, which belong to the crRNA, in the Stem 3-PK (R:AR-1) portion.

The sixth region comprises one or more nucleotides that interact with a WED domain, a ZF domain, and/or an RuvC domain of one dimer-forming Cas12f1 protein in the CRISPR/Cas12f1 complex. Here, the nucleotides may be nucleotides, which belong to the crRNA, in Stem 3-PK (R:AR-1).

In an embodiment, the sixth region may refer to the 11^{th} to 17^{th} nucleotides from the 5' end of the crRNA represented by SEQ ID NO: 3. In an embodiment, the sixth region may refer to the 31^{st} to 37^{th} nucleotides from the 5' end of the crRNA represented by SEQ ID NO: 4. In an embodiment, a sequence of the sixth region may be 5'-AUGCAAC-3'.

### Spacer

As used herein, the term "spacer" refers to one or more nucleotides, which hybridize with a target sequence, in a CRISPR/Cas12f1 system. The spacer refers to 10 to 50 consecutive nucleotides near the 3' end of the crRNA of the guide RNA in the CRISPR/Cas12f1 system. The spacer is designed to match a target sequence in the target nucleic acid to be edited using the CRISPR/Cas12f1 system. In other words, the spacer may have a different sequence depending on a target sequence of the target nucleic acid.

### Engineered scaffold region - Overview

### Overview of engineered scaffold region

In the present disclosure, there is provided an engineered scaffold region that can be introduced into a CRISPR/Cas12f1 system to improve gene editing efficiency thereof. The engineered scaffold region improves gene editing efficiency of a CRISPR/Cas12f1 system in which an engineered Cas12f1 guide RNA is used. The engineered scaffold region is characterized in that it is obtained by modifying one or more places in the scaffold region of a naturally occurring Cas12f1 guide RNA (hereinafter, naturally occurring scaffold region) and thus is different therefrom in terms of the sequence and/or structure.

Here, functions of the engineered scaffold region are identical or similar to those of the naturally occurring scaffold region. Specifically, the engineered scaffold region has a function of interacting with a Cas12f1 protein dimer in the CRISPR/Cas12f1 complex.

The engineered scaffold region includes regions corresponding to respective portions of the naturally occurring scaffold region. Specifically, the engineered scaffold region comprises a first region, a second region, a third region, a fourth region, a fifth region, and a sixth region, which respectively correspond to the first to sixth regions included in the naturally occurring scaffold region.

### Modifications to make single guide RNA

As provided herein, the engineered Cas12f1 guide RNA may be a single guide RNA of one molecule. Accordingly, the engineered scaffold region provided herein may be an engineered form of a naturally occurring scaffold region in which one or more of the respective regions are modified, and additionally, the 3' end of the fourth region of the tracrRNA and the 5' end of the fifth region of the crRNA are linked by a linker.

In an embodiment, the engineered scaffold region may be a modified form of a naturally occurring scaffold region in which one or more places are modified and the 3' end of the fourth region and the 5' end of the fifth region are linked by a linker. For example, the linker may be 5'-GAAA-3'.

### Engineered scaffold region 1 - Modification of first region

### Overview of modification of first region

The engineered scaffold region provided herein may be a modified form of a naturally occurring scaffold region in which the first region is modified.

In an embodiment, the engineered scaffold region may comprise a modified first region. Here, the modified first region is obtained by removing one or more nucleotides from the first region of the naturally occurring scaffold region. Here, the removed nucleotide is a nucleotide selected from a region forming a stem structure in a CRISPR/Cas12f1 complex.

In an embodiment, the engineered scaffold region included in the engineered Cas12f1 guide RNA may be a modified form of a naturally occurring scaffold region in which one or more nucleotides are removed from the first region and which may optionally have other modified portions than the first region. In an embodiment, the removed nucleotide may be a nucleotide included in a portion, which forms a stem structure in the CRISPR/Cas12f1 complex, in the naturally occurring first region. In an embodiment, the removed nucleotide may be a nucleotide which belongs to Stem 1 in the naturally occurring first region (Takeda et al., Structure of the miniature type V-F CRISPR-Cas effector enzyme, Molecular Cell 81, 1-13 (2021)). In an embodiment, the removed nucleotide may be a nucleotide which does not interact with a Cas12f1 protein in the CRISPR/Cas12f1 complex in the naturally occurring first region.

In an embodiment, the modified first region comprises a sequence of 5'-A-3' at the 3' end.

In an embodiment, the engineered scaffold region may be a modified form of a naturally occurring scaffold region from which the first region is removed. In other words, the engineered scaffold region may not comprise a region corresponding to the first region of the naturally occurring scaffold region.

### Modification of first region - Removal of some nucleotides

The first region of the engineered scaffold region may be a modified form of the first region of the naturally occurring scaffold region from which one or more nucleotides are removed.

In an embodiment, the modified first region of the engineered scaffold region may be a modified form of the first region of the naturally occurring scaffold region from which 1 to 20 nucleotides at the 5' end are removed. In an embodiment, the modified first region may be a modified form of the first region of the naturally occurring scaffold region from which 1, 2, 3, 4, 5, 6, 7, 8, 9, 10, 11, 12, 13, 14, 15, 16, 17, 18, 19, or 20 consecutive nucleotides at the 5' end are removed. In an embodiment, the modified first region may be a modified form of the first region of the naturally occurring scaffold region from which consecutive nucleotides at the 5' end, the number of which is within a range between two numbers selected from the immediately preceding sentence, are removed. For example, the modified first region may be a modified form of the first region of the naturally occurring scaffold region from which 1 to 3 consecutive nucleotides at the 5' end are removed.

In an embodiment, the modified first region comprises at least one nucleotide, which may be 5'-A-3'.

### Examples of sequence of modified first region

In an embodiment, the modified first region may be selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), and 5'-UUCACUGAUAAAGUGGAGAA-3' (SEQ ID NO: 26).

In an embodiment, a sequence of the engineered scaffold region in which the first region is modified may comprise:
a sequence in which the following sequences are linked to each other in a 5' to 3' direction,
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
SEQ ID NO: 10,
SEQ ID NO: 11, and
SEQ ID NO: 12; and
a sequence in which SEQ ID NO: 14 and 5'-AUGCAAC-3' are linked to each other in a 5' to 3' direction.

In an embodiment, a sequence of the engineered scaffold region in which the first region is modified may comprise:
a sequence selected from the group consisting of 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAUUU-3' (SEQ ID NO: 118), 5'-AACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAUUCAUUU-3' (SEQ ID NO: 119), 5'-GAACCGCUUCACCAAAAGCUGUCCCUUAGGGGAUUAGAACUUGAGUGAA GGUGGGCUGCUUGCAUCAGCCUAAUGUCGAGAAGUGCUUUCUUCGGAA AGUAACCCUCGAAACAAAUUCAUUU-3' (SEQ ID NO: 120), 5'-AGAACCGCUUCACCAAAAGCUGUCCCUUAGGGGAUUAGAACUUGAGUGA AGGUGGGCUGCUUGCAUCAGCCUAAUGUCGAGAAGUGCUUUCUUCGGA AAGUAACCCUCGAAACAAAUUCAUUU-3' (SEQ ID NO: 121), 5'-GAGAACCGCUUCACCAAAAGCUGUCCCUUAGGGGAUUAGAACUUGAGUG AAGGUGGGCUGCUUGCAUCAGCCUAAUGUCGAGAAGUGCUUUCUUCGG AAAGUAACCCUCGAAACAAAUUCAUUU-3' (SEQ ID NO: 122), 5'-GGAGAACCGCUUCACCAAAAGCUGUCCCUUAGGGGAUUAGAACUUGAGU GAAGGUGGGCUGCUUGCAUCAGCCUAAUGUCGAGAAGUGCUUUCUUCG GAAAGUAACCCUCGAAACAAAUUCAUUU-3' (SEQ ID NO: 123), 5'-UGGAGAACCGCUUCACCAAAAGCUGUCCCUUAGGGGAUUAGAACUUGAG UGAAGGUGGGCUGCUUGCAUCAGCCUAAUGUCGAGAAGUGCUUUCUUC GGAAAGUAACCCUCGAAACAAAUUCAUUU-3' (SEQ ID NO: 124), 5'-GUGGAGAACCGCUUCACCAAAAGCUGUCCCUUAGGGGAUUAGAACUUGA GUGAAGGUGGGCUGCUUGCAUCAGCCUAAUGUCGAGAAGUGCUUUCUU CGGAAAGUAACCCUCGAAACAAAUUCAUUU-3' (SEQ ID NO: 125), 5'-AGUGGAGAACCGCUUCACCAAAAGCUGUCCCUUAGGGGAUUAGAACUUG AGUGAAGGUGGGCUGCUUGCAUCAGCCUAAUGUCGAGAAGUGCUUUCU UCGGAAAGUAACCCUCGAAACAAAUUCAUUU-3' (SEQ ID NO: 126), 5'-AAGUGGAGAACCGCUUCACCAAAAGCUGUCCCUUAGGGGAUUAGAACUU GAGUGAAGGUGGGCUGCUUGCAUCAGCCUAAUGUCGAGAAGUGCUUUC UUCGGAAAGUAACCCUCGAAACAAAUUCAUUU-3' (SEQ ID NO: 127), 5'-AAAGUGGAGAACCGCUUCACCAAAAGCUGUCCCUUAGGGGAUUAGAACU UGAGUGAAGGUGGGCUGCUUGCAUCAGCCUAAUGUCGAGAAGUGCUUU CUUCGGAAAGUAACCCUCGAAACAAAUUCAUUU-3' (SEQ ID NO: 128), 5'-UAAAGUGGAGAACCGCUUCACCAAAAGCUGUCCCUUAGGGGAUUAGAAC UUGAGUGAAGGUGGGCUGCUUGCAUCAGCCUAAUGUCGAGAAGUGCUU UCUUCGGAAAGUAACCCUCGAAACAAAUUCAUUU-3' (SEQ ID NO: 129), 5'-AUAAAGUGGAGAACCGCUUCACCAAAAGCUGUCCCUUAGGGGAUUAGAA CUUGAGUGAAGGUGGGCUGCUUGCAUCAGCCUAAUGUCGAGAAGUGCU UUCUUCGGAAAGUAACCCUCGAAACAAAUUCAUUU-3' (SEQ ID NO: 130), 5'-GAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGC UUUCUUCGGAAAGUAACCCUCGAAACAAAUUCAUUU-3' (SEQ ID NO: 131), 5'-UGAUAAAGUGGAGAACCGCUUCACCAAAAGCUGUCCCUUAGGGGAUUAG AACUUGAGUGAAGGUGGGCUGCUUGCAUCAGCCUAAUGUCGAGAAGUG CUUUCUUCGGAAAGUAACCCUCGAAACAAAUUCAUUU-3' (SEQ ID NO: 132), 5'-CUGAUAAAGUGGAGAACCGCUUCACCAAAAGCUGUCCCUUAGGGGAUUA GAACUUGAGUGAAGGUGGGCUGCUUGCAUCAGCCUAAUGUCGAGAAGU GCUUUCUUCGGAAAGUAACCCUCGAAACAAAUUCAUUU-3' (SEQ ID NO: 133), 5'-ACUGAUAAAGUGGAGAACCGCUUCACCAAAAGCUGUCCCUUAGGGGAUU AGAACUUGAGUGAAGGUGGGCUGCUUGCAUCAGCCUAAUGUCGAGAAG UGCUUUCUUCGGAAAGUAACCCUCGAAACAAAUUCAUUU-3' (SEQ ID NO: 134), 5'-CACUGAUAAAGUGGAGAACCGCUUCACCAAAAGCUGUCCCUUAGGGGAU UAGAACUUGAGUGAAGGUGGGCUGCUUGCAUCAGCCUAAUGUCGAGAA GUGCUUUCUUCGGAAAGUAACCCUCGAAACAAAUUCAUUU-3' (SEQ ID NO: 135), 5'-UCACUGAUAAAGUGGAGAACCGCUUCACCAAAAGCUGUCCCUUAGGGGA UUAGAACUUGAGUGAAGGUGGGCUGCUUGCAUCAGCCUAAUGUCGAGA AGUGCUUUCUUCGGAAAGUAACCCUCGAAACAAAUUCAUUU-3' (SEQ ID NO: 136), and 5'-UUCACUGAUAAAGUGGAGAACCGCUUCACCAAAAGCUGUCCCUUAGGGG AUUAGAACUUGAGUGAAGGUGGGCUGCUUGCAUCAGCCUAAUGUCGAG AAGUGCUUUCUUCGGAAAGUAACCCUCGAAACAAAUUCAUUU-3' (SEQ ID NO: 137);
   and
5'-GAAUGAAGGAAUGCAAC-3' (SEQ ID NO: 3).

In an embodiment, a sequence of the engineered scaffold region in which the first region is modified may comprise:
a sequence in which the following sequences are linked to each other in a 5' to 3' direction,
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
SEQ ID NO: 10,
SEQ ID NO: 11, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region is modified may be one in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
SEQ ID NO: 10,
SEQ ID NO: 11,
SEQ ID NO: 12,
a linker,
SEQ ID NO: 14, and
5'-AUGCAAC-3'.

Here, the linker may be 5'-GAAA-3'.

In an embodiment, the engineered scaffold region in which a first region is modified may be a sequence selected from the group consisting of 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAUUCAUUUGAAAGAAUGAAGGAAUGCAAC-3' (SEQ ID NO: 167), 5'-AACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAA GUAACCCUCGAAACAAAUUCAUUUGAAAGAAUGAAGGAAUGCAAC-3' (SEQ ID NO: 168), 5'-GAACCGCUUCACCAAAAGCUGUCCCUUAGGGGAUUAGAACUUGAGUGAA GGUGGGCUGCUUGCAUCAGCCUAAUGUCGAGAAGUGCUUUCUUCGGAA AGUAACCCUCGAAACAAAUUCAUUUGAAAGAAUGAAGGAAUGCAAC-3' (SEQ ID NO: 169), 5'-AGAACCGCUUCACCAAAAGCUGUCCCUUAGGGGAUUAGAACUUGAGUGA AGGUGGGCUGCUUGCAUCAGCCUAAUGUCGAGAAGUGCUUUCUUCGGA AAGUAACCCUCGAAACAAAUUCAUUUGAAAGAAUGAAGGAAUGCAAC-3' (SEQ ID NO: 170), 5'-GAGAACCGCUUCACCAAAAGCUGUCCCUUAGGGGAUUAGAACUUGAGUG AAGGUGGGCUGCUUGCAUCAGCCUAAUGUCGAGAAGUGCUUUCUUCGG AAAGUAACCCUCGAAACAAAUUCAUUUGAAAGAAUGAAGGAAUGCAAC-3' (SEQ ID NO: 171), 5'-GGAGAACCGCUUCACCAAAAGCUGUCCCUUAGGGGAUUAGAACUUGAGU GAAGGUGGGCUGCUUGCAUCAGCCUAAUGUCGAGAAGUGCUUUCUUCG GAAAGUAACCCUCGAAACAAAUUCAUUUGAAAGAAUGAAGGAAUGCAAC-3' (SEQ ID NO: 172), 5'-UGGAGAACCGCUUCACCAAAAGCUGUCCCUUAGGGGAUUAGAACUUGAG UGAAGGUGGGCUGCUUGCAUCAGCCUAAUGUCGAGAAGUGCUUUCUUC GGAAAGUAACCCUCGAAACAAAUUCAUUUGAAAGAAUGAAGGAAUGCAAC -3' (SEQ ID NO: 173), 5'-GUGGAGAACCGCUUCACCAAAAGCUGUCCCUUAGGGGAUUAGAACUUGA GUGAAGGUGGGCUGCUUGCAUCAGCCUAAUGUCGAGAAGUGCUUUCUU CGGAAAGUAACCCUCGAAACAAAUUCAUUUGAAAGAAUGAAGGAAUGCAA C-3' (SEQ ID NO: 174), 5'-AGUGGAGAACCGCUUCACCAAAAGCUGUCCCUUAGGGGAUUAGAACUUG AGUGAAGGUGGGCUGCUUGCAUCAGCCUAAUGUCGAGAAGUGCUUUCU UCGGAAAGUAACCCUCGAAACAAAUUCAUUUGAAAGAAUGAAGGAAUGC AAC-3' (SEQ ID NO: 175), 5'-AAGUGGAGAACCGCUUCACCAAAAGCUGUCCCUUAGGGGAUUAGAACUU GAGUGAAGGUGGGCUGCUUGCAUCAGCCUAAUGUCGAGAAGUGCUUUC UUCGGAAAGUAACCCUCGAAACAAAUUCAUUUGAAAGAAUGAAGGAAUG CAAC-3' (SEQ ID NO: 176), 5'-AAAGUGGAGAACCGCUUCACCAAAAGCUGUCCCUUAGGGGAUUAGAACU UGAGUGAAGGUGGGCUGCUUGCAUCAGCCUAAUGUCGAGAAGUGCUUU CUUCGGAAAGUAACCCUCGAAACAAAUUCAUUUGAAAGAAUGAAGGAAU GCAAC-3' (SEQ ID NO: 177), 5'-UAAAGUGGAGAACCGCUUCACCAAAAGCUGUCCCUUAGGGGAUUAGAAC UUGAGUGAAGGUGGGCUGCUUGCAUCAGCCUAAUGUCGAGAAGUGCUU UCUUCGGAAAGUAACCCUCGAAACAAAUUCAUUUGAAAGAAUGAAGGAA UGCAAC-3' (SEQ ID NO: 178), 5'-AUAAAGUGGAGAACCGCUUCACCAAAAGCUGUCCCUUAGGGGAUUAGAA CUUGAGUGAAGGUGGGCUGCUUGCAUCAGCCUAAUGUCGAGAAGUGCU UUCUUCGGAAAGUAACCCUCGAAACAAAUUCAUUUGAAAGAAUGAAGGA AUGCAAC-3' (SEQ ID NO: 179), 5'-GAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGC UUUCUUCGGAAAGUAACCCUCGAAACAAAUUCAUUUGAAAGAAUGAAGG AAUGCAAC-3' (SEQ ID NO: 180), 5'-UGAUAAAGUGGAGAACCGCUUCACCAAAAGCUGUCCCUUAGGGGAUUAG AACUUGAGUGAAGGUGGGCUGCUUGCAUCAGCCUAAUGUCGAGAAGUG CUUUCUUCGGAAAGUAACCCUCGAAACAAAUUCAUUUGAAAGAAUGAAG GAAUGCAAC-3' (SEQ ID NO: 181), 5'-CUGAUAAAGUGGAGAACCGCUUCACCAAAAGCUGUCCCUUAGGGGAUUA GAACUUGAGUGAAGGUGGGCUGCUUGCAUCAGCCUAAUGUCGAGAAGU GCUUUCUUCGGAAAGUAACCCUCGAAACAAAUUCAUUUGAAAGAAUGAA GGAAUGCAAC-3' (SEQ ID NO: 182), 5'-ACUGAUAAAGUGGAGAACCGCUUCACCAAAAGCUGUCCCUUAGGGGAUU AGAACUUGAGUGAAGGUGGGCUGCUUGCAUCAGCCUAAUGUCGAGAAG UGCUUUCUUCGGAAAGUAACCCUCGAAACAAAUUCAUUUGAAAGAAUGA AGGAAUGCAAC-3' (SEQ ID NO: 183), 5'-CACUGAUAAAGUGGAGAACCGCUUCACCAAAAGCUGUCCCUUAGGGGAU UAGAACUUGAGUGAAGGUGGGCUGCUUGCAUCAGCCUAAUGUCGAGAA GUGCUUUCUUCGGAAAGUAACCCUCGAAACAAAUUCAUUUGAAAGAAUG AAGGAAUGCAAC-3' (SEQ ID NO: 184), 5'-UCACUGAUAAAGUGGAGAACCGCUUCACCAAAAGCUGUCCCUUAGGGGA UUAGAACUUGAGUGAAGGUGGGCUGCUUGCAUCAGCCUAAUGUCGAGA AGUGCUUUCUUCGGAAAGUAACCCUCGAAACAAAUUCAUUUGAAAGAAU GAAGGAAUGCAAC-3' (SEQ ID NO: 185), and 5'-UUCACUGAUAAAGUGGAGAACCGCUUCACCAAAAGCUGUCCCUUAGGGG AUUAGAACUUGAGUGAAGGUGGGCUGCUUGCAUCAGCCUAAUGUCGAG AAGUGCUUUCUUCGGAAAGUAACCCUCGAAACAAAUUCAUUUGAAAGAA UGAAGGAAUGCAAC-3' (SEQ ID NO: 186).

### Engineered scaffold region 2 - Modification of second region

### Overview of modification of second region

The engineered scaffold region included in the engineered guide RNA provided herein may be a modified form of a naturally occurring scaffold region in which the second region is modified.

In an embodiment, the engineered scaffold region may comprise a modified second region. Here, the modified second region is a modified form of the second region of the naturally occurring scaffold region from which one or more nucleotides are removed. Here, the removed nucleotide is a nucleotide selected from a region forming a stem structure in a CRISPR/Cas12f1 complex.

In an embodiment, the engineered scaffold region included in the engineered Cas12f1 guide RNA may have a modified form of a naturally occurring second region from which one or more nucleotides are removed and which may optionally have other modified portions than the second region. In an embodiment, removal of the nucleotides may occur in a portion forming a stem structure in the naturally occurring second region, in which the nucleotides may be removed in complementary pair. In an embodiment, the removed nucleotide may be a nucleotide included in a portion, which forms a stem structure in the CRISPR/Cas12f1 complex, in the naturally occurring second region. In an embodiment, the removed nucleotide may be a nucleotide, which belongs to Stem 2, in the naturally occurring second region (Takeda et al., Structure of the miniature type V-F CRISPR-Cas effector enzyme, Molecular Cell 81, 1-13 (2021)). In an embodiment, the removed nucleotide may be a nucleotide, which does not interact with a Cas12f1 protein in the CRISPR/Cas12f1 complex, in the naturally occurring second region.

In an embodiment, the modified second region has 5'-CCGCUUCAC-3' (SEQ ID NO: 432) at the 5' end. In an embodiment, the modified second region has 5'-UGAGUGAAGG-3' (SEQ ID NO: 433) at the 3' end.

### Modification detail 1 of second region - Removal of some nucleotides

The second region of the engineered scaffold region may be a modified form of a second region of the naturally occurring scaffold region from which one or more nucleotides are removed.

In an embodiment, the modified second region of the engineered scaffold region may be a modified form of a second region of the naturally occurring scaffold region from which 1, 2, 3, 4, 5, 6, 7, 8, 9, 10, 11, 12, 13, 14, 15, 16, 17, 18, 19, 20, 21, 22, 23, 24, 25, 26, or 27 consecutive or nonconsecutive nucleotides are removed. In an embodiment, the modified second region may be a modified form of a second region of the naturally occurring scaffold region from which one or more nucleotides, of the 12^{th} to 24^{th} nucleotides and/or the 27^{th} to 40^{th} from the 5' end based on the sequence of SEQ ID NO: 10, are removed. In an embodiment, the modified second region may be a second region of the naturally occurring scaffold region from which 1, 2, 3, 4, 5, 6, 7, 8, 9, 10, 11, 12, or 13 consecutive nucleotides, of the 12^{th} to 24^{th} nucleotides from the 5' end based on the sequence of SEQ ID NO: 10, are removed. In an embodiment, the modified second region may be a modified form of a second region of the naturally occurring scaffold region from which 1, 2, 3, 4, 5, 6, 7, 8, 9, 10, 11, 12, 13, or 14 consecutive nucleotides, of the 27^{th} to 38^{th} nucleotides from the 5' end based on the sequence of SEQ ID NO: 10, are removed.

In an embodiment, a sequence of the modified second region is characterized by comprising at least 5'-CCGCUUCAC-3' (SEQ ID NO: 432) and 5'-UGAGUGAAGG-3' (SEQ ID NO: 433). Here, a sequence of the modified second region may be one in which 5'-CCGCUUCAC-3' (SEQ ID NO: 432) and 5'-UGAGUGAAGG-3' (SEQ ID NO: 433) are sequentially linked to each other in a 5' to 3' direction, wherein the sequences may be linked by an appropriate intermediate sequence. As an example, the intermediate sequence may be selected from the group consisting of 5'-UUAG-3', 5'-AUUAGU-3', 5'-AAUUAGCU-3', 5'-AAAUUAGACU-3' (SEQ ID NO: 57), 5'-AAAGUUAGAACU-3' (SEQ ID NO: 58), 5'-AAAGCUUAGGAACU-3' (SEQ ID NO: 59), 5'-AAAGCUUUAGAGAACU-3' (SEQ ID NO: 60), 5'-AAAGCUGUUAGUUAGAACU-3' (SEQ ID NO: 61), 5'-AAAGCUGUUAGUAGAACU-3' (SEQ ID NO: 62), 5'-AAAGCUGUUUAGAUUAGAACU-3' (SEQ ID NO: 63), 5'-AAAGCUGUCUUAGGAUUAGAACU-3' (SEQ ID NO: 64), 5'-AAAGCUGUCCUUAGGGAUUAGAACU-3' (SEQ ID NO: 65), 5'-AAAAGCUGUCCCUUAGGGGAUUAGAACUU-3' (SEQ ID NO: 434), and 5'-CAAAAGCUGUCCCUUAGGGGAUUAGAACUUG-3' (SEQ ID NO: 435).

### Modification detail 2 of second region - Removal in complementary pair

The modification of the second region may be removal of one or more pairs of nucleotides that are included in a portion forming a stem structure and complementarily bind to each other.

In an embodiment, the modified second region may be a second region of the naturally occurring scaffold region from which one or more pairs of nucleotides forming complementary pairs in the CRISPR/Cas12f1 complex, of the 12^{th} to 24^{th} nucleotides and/or the 27^{th} to 40^{th} nucleotides from the 5' end based on the sequence of SEQ ID NO: 10, are removed.

In an embodiment, the modified second region may be a modified form of a second region of the naturally occurring scaffold region from which one or more pairs of nucleotides forming complementary pairs and/or one or more nucleotides not forming a complementary pair in the CRISPR/Cas12f1 complex, of the 12^{th} to 24^{th} nucleotides and/or the 27^{th} to 40^{th} nucleotides from the 5' end based on the sequence of SEQ ID NO: ID NO: 10, are removed.

In an embodiment, the modified second region may be a modified form of a second region of the naturally occurring scaffold region from which one or more pairs of nucleotides forming complementary pairs and/or one or more mismatched pairs of nucleotides in the CRISPR/Cas12f1 complex, of the 12^{th} to 24^{th} nucleotides and/or the 27^{th} to 40^{th} nucleotides from the 5' end based on the sequence of SEQ ID NO: 10, are removed.

### Example of sequence of modified second region

In an embodiment, a sequence of the modified second region may be a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUG-3' (SEQ ID NO: 38), 5'-CCGCUUCACCAAUUAGUUGAGUGAAGGUG-3' (SEQ ID NO: 39), 5'-CCGCUUCACCAAAUUAGCUUGAGUGAAGGUG-3' (SEQ ID NO: 40), 5'-CCGCUUCACCAAAAUUAGACUUGAGUGAAGGUG-3' (SEQ ID NO: 41), 5'-CCGCUUCACCAAAAGUUAGAACUUGAGUGAAGGUG-3' (SEQ ID NO: 42), 5'-CCGCUUCACCAAAAGCUUAGGAACUUGAGUGAAGGUG-3' (SEQ ID NO: 43), 5'-CCGCUUCACCAAAAGCUUUAGAGAACUUGAGUGAAGGUG-3' (SEQ ID NO: 44), 5'-CCGCUUCACCAAAAGCUGUUAGUUAGAACUUGAGUGAAGGUG-3' (SEQ ID NO: 45), 5'-CCGCUUCACCAAAAGCUGUUAGUAGAACUUGAGUGAAGGUG-3' (SEQ ID NO: 46), 5'-CCGCUUCACCAAAAGCUGUUUAGAUUAGAACUUGAGUGAAGGUG-3' (SEQ ID NO: 47), 5'-CCGCUUCACCAAAAGCUGUCUUAGGAUUAGAACUUGAGUGAAGGUG-3' (SEQ ID NO: 48), and 5'-CCGCUUCACCAAAAGCUGUCCUUAGGGAUUAGAACUUGAGUGAAGGUG-3' (SEQ ID NO: 49).

In an embodiment, a sequence of the engineered scaffold region in which the second region is modified may comprise:
a sequence in which the following sequences are linked to each other in a 5' to 3' direction,
SEQ ID NO: 9,
a sequence selected from the group consisting of SEQ ID NOS: 38 to 49, and SEQ ID NOS: 430 to 431,
SEQ ID NO: 11, and
SEQ ID NO: 12; and
a sequence in which SEQ ID NO: 14 and 5'-AUGCAAC-3' are linked to each other in a 5' to 3' direction.

In an embodiment, a sequence of the engineered scaffold region in which the second region is modified may comprise:
a sequence selected from the group consisting of 5'-CUUCACUGAUAAAGUGGAGAACCGCUUCACCAUUAGUGAGUGAAGGUGG GCUGCUUGCAUCAGCCUAAUGUCGAGAAGUGCUUUCUUCGGAAAGUAAC CCUCGAAACAAAUUCAUUU-3' (SEQ ID NO: 138), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCAUUU-3' (SEQ ID NO: 139), 5'-CUUCACUGAUAAAGUGGAGAACCGCUUCACCAAAUUAGACUUGAGUGAA GGUGGGCUGCUUGCAUCAGCCUAAUGUCGAGAAGUGCUUUCUUCGGAA AGUAACCCUCGAAACAAAUUCAUUU-3' (SEQ ID NO: 140), 5'-CUUCACUGAUAAAGUGGAGAACCGCUUCACCAAAAGUUAGAACUUGAGU GAAGGUGGGCUGCUUGCAUCAGCCUAAUGUCGAGAAGUGCUUUCUUCG GAAAGUAACCCUCGAAACAAAUUCAUUU-3' (SEQ ID NO: 141), 5'-CUUCACUGAUAAAGUGGAGAACCGCUUCACCAAAAGCUUAGGAACUUGA GUGAAGGUGGGCUGCUUGCAUCAGCCUAAUGUCGAGAAGUGCUUUCUU CGGAAAGUAACCCUCGAAACAAAUUCAUUU-3' (SEQ ID NO: 142), 5'-CUUCACUGAUAAAGUGGAGAACCGCUUCACCAAAAGCUUUAGAGAACUU GAGUGAAGGUGGGCUGCUUGCAUCAGCCUAAUGUCGAGAAGUGCUUUC UUCGGAAAGUAACCCUCGAAACAAAUUCAUUU-3' (SEQ ID NO: 143), 5'-CUUCACUGAUAAAGUGGAGAACCGCUUCACCAAAAGCUGUUAGUUAGAA CUUGAGUGAAGGUGGGCUGCUUGCAUCAGCCUAAUGUCGAGAAGUGCU UUCUUCGGAAAGUAACCCUCGAAACAAAUUCAUUU-3' (SEQ ID NO: 144), 5'-CUUCACUGAUAAAGUGGAGAACCGCUUCACCAAAAGCUGUUAGUAGAAC UUGAGUGAAGGUGGGCUGCUUGCAUCAGCCUAAUGUCGAGAAGUGCUU UCUUCGGAAAGUAACCCUCGAAACAAAUUCAUUU-3' (SEQ ID NO: 145), 5'-CUUCACUGAUAAAGUGGAGAACCGCUUCACCAAAAGCUGUUUAGAUUAG AACUUGAGUGAAGGUGGGCUGCUUGCAUCAGCCUAAUGUCGAGAAGUG CUUUCUUCGGAAAGUAACCCUCGAAACAAAUUCAUUU-3' (SEQ ID NO: 146), 5'-CUUCACUGAUAAAGUGGAGAACCGCUUCACCAAAAGCUGUCUUAGGAUU AGAACUUGAGUGAAGGUGGGCUGCUUGCAUCAGCCUAAUGUCGAGAAG UGCUUUCUUCGGAAAGUAACCCUCGAAACAAAUUCAUUU-3' (SEQ ID NO: 147), and 5'-CUUCACUGAUAAAGUGGAGAACCGCUUCACCAAAAGCUGUCCUUAGGGA UUAGAACUUGAGUGAAGGUGGGCUGCUUGCAUCAGCCUAAUGUCGAGA AGUGCUUUCUUCGGAAAGUAACCCUCGAAACAAAUUCAUUU-3' (SEQ ID NO: 148); and
SEQ ID NO: 3.

In an embodiment, a sequence of the engineered scaffold region in which the second region is modified may comprise:
a sequence in which the following sequences are linked to each other in a 5' to 3' direction:
SEQ ID NO: 9,
a sequence selected from SEQ ID NOS: 38 to 49, and SEQ ID NOS: 430 to 431,
SEQ ID NO: 11, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the second region is modified may be one in which a sequence selected from SEQ ID NO: 9, SEQ ID NOS: 38 to 49, and SEQ ID NOS: 430 to 431, SEQ ID NO: 11, SEQ ID NO: 12, a linker, SEQ ID NO: 14, and 5'-AUGCAAC-3' are linked to each other in a 5' to 3' direction.

Here, the linker may be 5'-GAAA-3'.

In an embodiment, a sequence of the engineered scaffold region in which the second region is modified may be selected from the group consisting of 5'-CUUCACUGAUAAAGUGGAGAACCGCUUCACCAUUAGUGAGUGAAGGUGG GCUGCUUGCAUCAGCCUAAUGUCGAGAAGUGCUUUCUUCGGAAAGUAAC CCUCGAAACAAAUUCAUUUGAAAGAAUGAAGGAAUGCAAC-3' (SEQ ID NO: 187), 5'-CUUCACUGAUAAAGUGGAGAACCGCUUCACCAAUUAGUUGAGUGAAGGU GGGCUGCUUGCAUCAGCCUAAUGUCGAGAAGUGCUUUCUUCGGAAAGU AACCCUCGAAACAAAUUCAUUUGAAAGAAUGAAGGAAUGCAAC-3' (SEQ ID NO: 188), 5'-CUUCACUGAUAAAGUGGAGAACCGCUUCACCAAAUUAGCUUGAGUGAAG GUGGGCUGCUUGCAUCAGCCUAAUGUCGAGAAGUGCUUUCUUCGGAAA GUAACCCUCGAAACAAAUUCAUUUGAAAGAAUGAAGGAAUGCAAC-3' (SEQ ID NO: 189), 5'-CUUCACUGAUAAAGUGGAGAACCGCUUCACCAAAAUUAGACUUGAGUGA AGGUGGGCUGCUUGCAUCAGCCUAAUGUCGAGAAGUGCUUUCUUCGGA AAGUAACCCUCGAAACAAAUUCAUUUGAAAGAAUGAAGGAAUGCAAC-3' (SEQ ID NO: 190), 5'-CUUCACUGAUAAAGUGGAGAACCGCUUCACCAAAAGUUAGAACUUGAGU GAAGGUGGGCUGCUUGCAUCAGCCUAAUGUCGAGAAGUGCUUUCUUCG GAAAGUAACCCUCGAAACAAAUUCAUUUGAAAGAAUGAAGGAAUGCAAC-3' (SEQ ID NO: 191), 5'-CUUCACUGAUAAAGUGGAGAACCGCUUCACCAAAAGCUUAGGAACUUGA GUGAAGGUGGGCUGCUUGCAUCAGCCUAAUGUCGAGAAGUGCUUUCUU CGGAAAGUAACCCUCGAAACAAAUUCAUUUGAAAGAAUGAAGGAAUGCAA C-3' (SEQ ID NO: 192), 5'-CUUCACUGAUAAAGUGGAGAACCGCUUCACCAAAAGCUUUAGAGAACUU GAGUGAAGGUGGGCUGCUUGCAUCAGCCUAAUGUCGAGAAGUGCUUUC UUCGGAAAGUAACCCUCGAAACAAAUUCAUUUGAAAGAAUGAAGGAAUG CAAC-3' (SEQ ID NO: 193), 5'-CUUCACUGAUAAAGUGGAGAACCGCUUCACCAAAAGCUGUUAGUAGAAC UUGAGUGAAGGUGGGCUGCUUGCAUCAGCCUAAUGUCGAGAAGUGCUU UCUUCGGAAAGUAACCCUCGAAACAAAUUCAUUUGAAAGAAUGAAGGAA UGCAAC-3' (SEQ ID NO: 194), 5'-CUUCACUGAUAAAGUGGAGAACCGCUUCACCAAAAGCUGUUAGUUAGAA CUUGAGUGAAGGUGGGCUGCUUGCAUCAGCCUAAUGUCGAGAAGUGCU UUCUUCGGAAAGUAACCCUCGAAACAAAUUCAUUUGAAAGAAUGAAGGA AUGCAAC-3' (SEQ ID NO: 195), 5'-CUUCACUGAUAAAGUGGAGAACCGCUUCACCAAAAGCUGUUUAGAUUAG AACUUGAGUGAAGGUGGGCUGCUUGCAUCAGCCUAAUGUCGAGAAGUG CUUUCUUCGGAAAGUAACCCUCGAAACAAAUUCAUUUGAAAGAAUGAAG GAAUGCAAC-3' (SEQ ID NO: 196), 5'-CUUCACUGAUAAAGUGGAGAACCGCUUCACCAAAAGCUGUCUUAGGAUU AGAACUUGAGUGAAGGUGGGCUGCUUGCAUCAGCCUAAUGUCGAGAAG UGCUUUCUUCGGAAAGUAACCCUCGAAACAAAUUCAUUUGAAAGAAUGA AGGAAUGCAAC-3' (SEQ ID NO: 197), and 5'-CUUCACUGAUAAAGUGGAGAACCGCUUCACCAAAAGCUGUCCUUAGGGA UUAGAACUUGAGUGAAGGUGGGCUGCUUGCAUCAGCCUAAUGUCGAGA AGUGCUUUCUUCGGAAAGUAACCCUCGAAACAAAUUCAUUUGAAAGAAU GAAGGAAUGCAAC-3' (SEQ ID NO: 198).

### Engineered scaffold region 3 - Modification of third region

The third region in the engineered scaffold provided herein is a region containing the Stem 4 portion, which has a key effect on DNA cleavage activity. Therefore, the third region of the engineered scaffold may be the same as the third region of the naturally occurring scaffold region, or may be a third region of a naturally occurring scaffold region which is modified to the extent that a function of the third region is not impaired.

### Engineered scaffold region 4 - Modification of fourth and fifth regions

### Overview of modification of fourth and fifth regions

The engineered scaffold region provided herein may be a modified form of a naturally occurring scaffold region in which the fourth and fifth regions are modified. The fourth and fifth regions comprise parts that hybridize to each other to form a stem in the CRISPR/Cas12f1 complex and thus the corresponding parts may be modified together to constitute an engineered scaffold region.

In an embodiment, the engineered scaffold region may comprise a modified fourth region and/or a modified fifth region.

The modified fourth region is characterized in that it is obtained by removing one or more nucleotides from the fourth region of the naturally occurring scaffold region. The modified fifth region is characterized in that it is obtained by removing one or more nucleotides from the fifth region of the naturally occurring scaffold region.

In an embodiment, an engineered scaffold region included in the engineered Cas12f1 guide RNA may be a modified form of a naturally occurring scaffold region in which one or more nucleotides are removed from the fourth region and/or the fifth region.

In an embodiment, the modified fourth region has 5'-AACAAA-3' at the 5' end. In an embodiment, the modified fifth region has 5'-GGA-3' at the 3' end.

### Modification detail 1 of fourth and fifth regions - Removal of some nucleotides

The fourth region of the engineered scaffold region may be a modified form of a fourth region of the naturally occurring scaffold region from which one or more nucleotides are removed. The fifth region of the engineered scaffold region may be a fifth region of the naturally occurring scaffold region from which one or more nucleotides are removed.

In an embodiment, the modified fourth region of the engineered scaffold region may be a modified form of a fourth region of a naturally occurring scaffold region from which 1, 2, 3, 4, 5, 6 or 7 consecutive or nonconsecutive nucleotides are removed.

In an embodiment, the modified fourth region of the engineered scaffold region may be a modified form of a fourth region of the naturally occurring scaffold region from which 1, 2, 3, 4, 5, 6, 7, 8, 9, 10, 11, 12, 13, 14, 15, 16, 17, 18, 19, 20, 21, 22, 23, 24, 25, 26, 27, or 28 consecutive or nonconsecutive nucleotides are removed.

In an embodiment, the modified fourth region may be a modified form of a fourth region of the naturally occurring scaffold region from which one or more nucleotides, of the 7^{th} to 13^{th} nucleotides from the 5' end based on the sequence of SEQ ID NO: 12, are removed. In an embodiment, the modified fourth region may be a modified form of a fourth region of the naturally occurring scaffold region from which one or more nucleotides, of the 7^{th} to 34^{th} nucleotides from the 5' end based on the sequence of SEQ ID NO: 13, are removed.

In an embodiment, a sequence of the modified fourth region comprises at least 5'-AACAAA-3'.

In an embodiment, the modified fifth region of the engineered scaffold region may be a modified form of a fifth region of the naturally occurring scaffold region from which 1, 2, 3, 4, 5, 6 or 7 consecutive or nonconsecutive nucleotides are removed.

In an embodiment, the modified fifth region of the engineered scaffold region may be a modified form of a fifth region of the naturally occurring scaffold region from which 1, 2, 3, 4, 5, 6, 7, 8, 9, 10, 11, 12, 13, 14, 15, 16, 17, 18, 19, 20, 21, 22, 23, 24, 25, 26, or 27 consecutive or nonconsecutive nucleotides are removed.

In an embodiment, the modified fifth region may be a modified form of a fifth region of the naturally occurring scaffold region from which one or more nucleotides, of the 1^{st} to 7^{th} nucleotides from the 5' end based on the sequence of SEQ ID NO: 14, are removed. In an embodiment, the modified fifth region may be a modified form of a fifth region of the naturally occurring scaffold region from which one or more nucleotides, of the 1^{st} to 27^{th} nucleotides from the 5' end based on the sequence of SEQ ID NO: 15, are removed.

In an embodiment, the modified fifth region comprises 5'-GGA-3'.

### Modification detail 2 of fourth and fifth regions - Removal in complementary pair

The fourth region and the fifth region are known to form a stem by complementarily binding to each other in the CRISPR/Cas12 complex. Since the above-described modifications of the fourth and fifth regions are subject to one or more nucleotides constituting the stem, the modifications of the fourth and fifth regions may be made to remove the nucleotides constituting the stem in complementary pair.

In an embodiment, the modified fourth and fifth regions may be a fourth region and a fifth region of the naturally occurring scaffold region from which one or more pairs of nucleotides forming complementary pairs in the CRISPR/Cas12f1 complex, of the 7^{th} to 13^{th} nucleotides from the 5' end based on the sequence of SEQ ID NO: 12 and the 1^{st} to 7^{th} nucleotides from the 5' end based on the sequence of SEQ ID NO: 14, are removed.

In an embodiment, the modified fourth and fifth regions may be modified forms of a fourth region and a fifth region of the naturally occurring scaffold region from which one or more pairs of nucleotides forming complementary pairs and/or one or more nucleotides not forming a complementary pair in the CRISPR/Cas12f1 complex, of the 7^{th} to 13^{th} nucleotides from the 5' end based on the sequence of SEQ ID NO: 12 and the 1^{st} to 7^{th} nucleotides from the 5' end based on the sequence of SEQ ID NO: 14, are removed.

In an embodiment, the modified fourth and fifth regions may be modified forms of a fourth region and a fifth region of the naturally occurring scaffold region from which one or more pairs of nucleotides forming complementary pairs and/or one or more mismatched pairs of nucleotides in the CRISPR/Cas12f1 complex, of the 7^{th} to 13^{th} nucleotides from the 5' end based on the sequence of SEQ ID NO: 12 and the 1^{st} to 7^{th} nucleotides from the 5' end based on the sequence of SEQ ID NO: 14, are removed.

In an embodiment, the modified fourth and fifth regions may be modified forms of a fourth region and a fifth region of the naturally occurring scaffold region from which one or more pairs of nucleotides forming complementary pairs in the CRISPR/Cas12f1 complex, of the 7^{th} to 34^{th} nucleotides from the 5' end based on the sequence of SEQ ID NO: 13 and the 1^{st} to 27^{th} nucleotides from the 5' end based on the sequence of SEQ ID NO: 15, are removed.

In an embodiment, the modified fourth and fifth regions may be modified forms of a fourth region and a fifth region of the naturally occurring scaffold region from which one or more pairs of nucleotides forming complementary pairs and/or one or more nucleotides not forming a complementary pair in the CRISPR/Cas12f1 complex, of the 7^{th} to 34^{th} nucleotides from the 5' end based on the sequence of SEQ ID NO: 13 and the 1^{st} to 27^{th} nucleotides from the 5' end based on the sequence of SEQ ID NO: 15, are removed.

In an embodiment, the modified fourth and fifth regions may be modified forms of a fourth region and a fifth region of the naturally occurring scaffold region from which one or more pairs of nucleotides forming complementary pairs and/or one or more mismatched pairs of nucleotides in the CRISPR/Cas12f1 complex, of the 7^{th} to 34^{th} nucleotides from the 5' end based on the sequence of SEQ ID NO: 13 and the 1^{st} to 27^{th} nucleotides from the 5' end based on the sequence of SEQ ID NO: 15, are removed.

### Examples of sequences of modified fourth region and fifth regions

In an embodiment, a sequence of the modified fourth region may be selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 66), 5'-AACAAAUUCAU-3' (SEQ ID NO: 67), and 5'-AACAAAUUCAUU-3' (SEQ ID NO: 68).

In an embodiment, a sequence of the modified fourth region may be selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 66), 5'-AACAAAUUCAU-3' (SEQ ID NO: 67), 5'-AACAAAUUCAUU-3' (SEQ ID NO: 68), 5'-AACAAAUUCAUUU-3' (SEQ ID NO: 69), 5'-AACAAAUUCAUUUU-3' (SEQ ID NO: 70), 5'-AACAAAUUCAUUUUU-3' (SEQ ID NO: 71), 5'-AACAAAUUCAUUUUUC-3' (SEQ ID NO: 72), 5'-AACAAAUUCAUUUUUCC-3' (SEQ ID NO: 73), 5'-AACAAAUUCAUUUUUCCU-3' (SEQ ID NO: 74), 5'-AACAAAUUCAUUUUUCCUC-3' (SEQ ID NO: 75), 5'-AACAAAUUCAUUUUUCCUCU-3' (SEQ ID NO: 76), 5'-AACAAAUUCAUUUUUCCUCUC-3' (SEQ ID NO: 77), 5'-AACAAAUUCAUUUUUCCUCUCC-3' (SEQ ID NO: 78), 5'-AACAAAUUCAUUUUUCCUCUCCA-3' (SEQ ID NO: 79), 5'-AACAAAUUCAUUUUUCCUCUCCAA-3' (SEQ ID NO: 80), 5'-AACAAAUUCAUUUUUCCUCUCCAAU-3' (SEQ ID NO: 81), 5'-AACAAAUUCAUUUUUCCUCUCCAAUU-3' (SEQ ID NO: 82), 5'-AACAAAUUCAUUUUUCCUCUCCAAUUC-3' (SEQ ID NO: 83), 5'-AACAAAUUCAUUUUUCCUCUCCAAUUCU-3' (SEQ ID NO: 84), 5'-AACAAAUUCAUUUUUCCUCUCCAAUUCUG-3' (SEQ ID NO: 85), 5'-AACAAAUUCAUUUUUCCUCUCCAAUUCUGC-3' (SEQ ID NO: 86), 5'-AACAAAUUCAUUUUUCCUCUCCAAUUCUGCA-3' (SEQ ID NO: 87), 5'-AAACAAAUUCAUUUUUCCUCUCCAAUUCUGCAC-3' (SEQ ID NO: 88), and 5'-AACAAAUUCAUUUUUCCUCUCCAAUUCUGCACA-3' (SEQ ID NO: 89).

In an embodiment, a sequence of the modified fifth region may be selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3'.

In an embodiment, a sequence of the modified fifth region may be selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', 5'-GAAUGAAGGA-3' (SEQ ID NO: 90), 5'-CGAAUGAAGGA-3' (SEQ ID NO: 91), 5'-ACGAAUGAAGGA-3' (SEQ ID NO: 92), 5'-GACGAAUGAAGGA-3' (SEQ ID NO: 93), 5'-AGACGAAUGAAGGA-3' (SEQ ID NO: 94), 5'-UAGACGAAUGAAGGA-3' (SEQ ID NO: 95), 5'-AUAGACGAAUGAAGGA-3' (SEQ ID NO: 96), 5'-AAUAGACGAAUGAAGGA-3' (SEQ ID NO: 97), 5'-GAAUAGACGAAUGAAGGA-3' (SEQ ID NO: 98), 5'-CGAAUAGACGAAUGAAGGA-3' (SEQ ID NO: 99), 5'-CCGAAUAGACGAAUGAAGGA-3' (SEQ ID NO: 100), 5'-CCCGAAUAGACGAAUGAAGGA-3' (SEQ ID NO: 101), 5'-ACCCGAAUAGACGAAUGAAGGA-3' (SEQ ID NO: 102), 5'-AACCCGAAUAGACGAAUGAAGGA-3' (SEQ ID NO: 103), 5'-GAACCCGAAUAGACGAAUGAAGGA-3' (SEQ ID NO: 104), 5'-AGAACCCGAAUAGACGAAUGAAGGA-3' (SEQ ID NO: 105), 5'-CAGAACCCGAAUAGACGAAUGAAGGA-3' (SEQ ID NO: 106), 5'-GCAGAACCCGAAUAGACGAAUGAAGGA-3' (SEQ ID NO: 107), 5'-UGCAGAACCCGAAUAGACGAAUGAAGGA-3' (SEQ ID NO: 108), and 5'-UUGCAGAACCCGAAUAGACGAAUGAAGGA-3' (SEQ ID NO: 109).

In an embodiment, a sequence of the engineered scaffold region in which the fourth and fifth regions are modified may comprise:
a sequence in which the following sequences are linked to each other in a 5' to 3' direction:
SEQ ID NO: 9,
SEQ ID NO: 10,
SEQ ID NO: 11, and
a sequence selected from the group consisting of 5'-AACAAA-3', 5'-AACAAAU-3', 5'-AACAAAUU-3', 5'-AACAAAUUC-3', and SEQ ID NOS: 66 to 68; and
a sequence in which the following sequences are linked to each other in a 5' to 3' direction,
a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
5'-AUGCAAC-3'.

In an embodiment, a sequence of the engineered scaffold region in which the fourth and fifth regions are modified may comprise:
a sequence selected from the group consisting of 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAA-3' (SEQ ID NO: 149), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAU-3' (SEQ ID NO: 150), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUU-3' (SEQ ID NO: 151), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUC-3' (SEQ ID NO: 152), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCA-3' (SEQ ID NO: 153), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAU-3' (SEQ ID NO: 154), and 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUU-3' (SEQ ID NO: 155); and
a sequence selected from the group consisting of 5'-GGAAUGCAAC-3', 5'-AGGAAUGCAAC-3', 5'-AAGGAAUGCAAC-3', 5'-GAAGGAAUGCAAC-3', 5'-UGAAGGAAUGCAAC-3', 5'-AUGAAGGAAUGCAAC-3', and 5'-AAU GAAGGAAU GCAAC-3'.

In an embodiment, a sequence of the engineered scaffold region in which the fourth and fifth regions are modified may comprise:
a sequence in which the following sequences are linked to each other in a 5' to 3' direction,
SEQ ID NO: 9,
SEQ ID NO: 10,
SEQ ID NO: 11, and
a sequence selected from the group consisting of 5'-AACAAA-3', 5'-AACAAAU-3', 5'-AACAAAUU-3', 5'-AACAAAUUC-3', and SEQ ID NOS: 66 to 89; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', and SEQ ID NOS: 90 to 109, and
   5'-AUGCAAC-3'.

In an embodiment, a sequence of the engineered scaffold region in which the fourth and fifth regions are modified may be one in which the following sequences are linked to each other in a 5' to 3' direction:
SEQ ID NO: 9,
SEQ ID NO: 10,
SEQ ID NO: 11, and
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), and 5'-AACAAAUUCAUUGAAAAAUGAAGGA-3' (SEQ ID NO: 116), and
5'-AUGCAAC-3'.

In an embodiment, a sequence of the engineered scaffold region in which the fourth and fifth regions are modified may be selected from the group consisting of 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAGAAAGGAAUGCAA C-3' (SEQ ID NO: 199), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUGAAAAGGAAUGC AAC-3' (SEQ ID NO: 200), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUGAAAAAGGAAU GCAAC-3' (SEQ ID NO: 201), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCGAAAGAAGGA AUGCAAC-3' (SEQ ID NO: 202), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAGAAAUGAAG GAAUGCAAC-3' (SEQ ID NO: 203), 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUGAAAAUGA AGGAAUGCAAC-3' (SEQ ID NO: 204), and 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUGAAAAAU GAAGGAAUGCAAC-3' (SEQ ID NO: 205).

### Engineered scaffold region 5 - Modification of sixth region

In the engineered scaffold provided herein, the sixth region is a region including nucleotides, which belong to the crRNA in the Stem 3-PK (R:AR-1) portion. As described above, the sixth region comprises one or more nucleotides that interact with a WED domain, a ZF domain, and/or an RuvC domain of one dimer-forming Cas12f1 protein in the CRISPR/Cas12f1 complex. The sixth region of the engineered scaffold may be the same as the sixth region of the naturally occurring scaffold region, or may be a sixth region of the naturally occurring scaffold region which is modified to the extent that a function of the sixth region is not impaired.

### Engineered scaffold region 6 - Combination of respective modifications

### Overview of combination of respective modifications

The engineered scaffold region included in the engineered Cas12f1 guide RNA provided herein may be a modified form of a naturally occurring scaffold region in which one or more of the above-mentioned modifications for respective regions are combined.

In an embodiment, the engineered scaffold region may comprise a modified first region and a modified second region.

In an embodiment, the engineered scaffold region may comprise a modified first region and modified fourth and fifth regions.

In an embodiment, the engineered scaffold region may comprise a modified second region and modified fourth and fifth regions.

In an embodiment, the engineered scaffold region may comprise a modified first region, a modified second region, and modified fourth and fifth regions.

Here, the modified regions are as described above in the section for modification of each of the regions.

### Combination 1 of respective modifications - Modification of first region and modification of second region

In an embodiment, the engineered scaffold region may comprise a modified first region and a modified second region. Here, the modified first region includes all of the modifications described in the section "Engineered scaffold region 1 - Modification of first region." Here, the modified second region includes all of the modifications described in the section "Engineered scaffold region 2 - Modification of second region."

In an embodiment, a sequence of the engineered scaffold region in which the first region and the second region are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
a sequence selected from the group consisting of SEQ ID NOS: 38 to 49, and SEQ ID NOS: 430 to 431,
SEQ ID NO: 11, and
SEQ ID NO: 12; and
a sequence in which SEQ ID NO: 14 and 5'-AUGCAAC-3' are linked to each other in a 5' to 3' direction.

In an embodiment, a sequence of the engineered scaffold region in which the first region and the second region are modified may comprise in a 5' to 3' direction: 
5'-
ACCGCUUCACCAUUAGUGAGUGAAGGUGGGCUGCUUGCAUCAGCCUAA
UGUCGAGAAGUGCUUUCUUCGGAAAGUAACCCUCGAAACAAAUUCAUUU-
3' (SEQ ID NO: 156);
and
5'-GAAUGAAGGAAUGCAAC-3' (SEQ ID NO: 3).

In an embodiment, a sequence of the engineered scaffold region in which the first region and the second region are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
a sequence selected from the group consisting of SEQ ID NOS: 38 to 49, and SEQ ID NOS: 430 to 431,
SEQ ID NO: 11,
SEQ ID NO: 12,
a linker,
SEQ ID NO: 14, and
5'-AUGCAAC-3'.

Here, the linker may be 5'-GAAA-3'.

In an embodiment, a sequence of the engineered scaffold region in which the first region and the second region are modified may be 5'-ACCGCUUCACCAUUAGUGAGUGAAGGUGGGCUGCUUGCAUCAGCCUAA UGUCGAGAAGUGCUUUCUUCGGAAAGUAACCCUCGAAACAAAUUCAUUU GAAAGAAUGAAGGAAUGCAAC-3' (SEQ ID NO: 206).

### Combination 2 of respective modifications - Modification of first region and modification of fourth and fifth regions

In an embodiment, the engineered scaffold region may comprise a modified first region and modified fourth and fifth regions. Here, the modified first region includes all of the modifications described in the section "Engineered scaffold region 1 - Modification of first region." Here, the modified fourth and fifth regions include all of the modifications described in the section "Engineered scaffold region 4 - Modification of fourth and fifth regions."

In an embodiment, a sequence of the engineered scaffold region in which the first region and the fourth and fifth regions are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
SEQ ID NO: 10,
SEQ ID NO: 11, and
a sequence selected from the group consisting of 5'-AACAAA-3', 5'-AACAAAU-3', 5'-AACAAAUU-3', 5'-AACAAAUUC-3', and SEQ ID NOS: 66 to 68; and
a sequence in which the following sequences are linked to each other in a 5' to 3' direction:
a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
5'-AUGCAAC-3'.

In an embodiment, a sequence of the engineered scaffold region in which the first region and the fourth and fifth regions are modified may comprise in a 5' to 3' direction: 
5'-
ACCGCUUCACCAAAAGCUGUCCCUUAGGGGAUUAGAACUUGAGUGAAGG
UGGGCUGCUUGCAUCAGCCUAAUGUCGAGAAGUGCUUUCUUCGGAAAG
UAACCCUCGAAACAAA-3' (SEQ ID NO: 157);
and
5'-GGAAUGCAAC-3' (SEQ ID NO: 160).

In an embodiment, a sequence of the engineered scaffold region in which the first region and the fourth and fifth regions are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
SEQ ID NO: 10,
SEQ ID NO: 11,
a sequence selected from SEQ ID NOS: 110 to 116, and
5'-AUGCAAC-3'.

In an embodiment, a sequence of the engineered scaffold region in which the first region and the fourth and fifth regions are modified may be 5'-ACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAG UAACCCUCGAAACAAAGAAAGGAAUGCAAC-3' (SEQ ID NO: 207).

### Combination 3 of respective modifications - Modification of second region, and modification of fourth and fifth regions

In an embodiment, the engineered scaffold region may comprise a modified second region and modified fourth and fifth regions. Here, the modified second region includes all of the modifications described in the section "Engineered scaffold region 2 - Modification of second region." Here, the modified fourth and fifth regions include all of the modifications described in the section "Engineered scaffold region 4 -Modification of fourth and fifth regions."

In an embodiment, a sequence of the engineered scaffold region in which the second region and the fourth and fifth regions are modified may comprise:
a sequence in which the following sequences are linked to each other in a 5' to 3' direction:
SEQ ID NO: 9,
a sequence selected from the group consisting of SEQ ID NOS: 38 to 49, and SEQ ID NOS: 430 to 431,
SEQ ID NO: 11, and
a sequence selected from the group consisting of 5'-AACAAA-3', 5'-AACAAAU-3', 5'-AACAAAUU-3', 5'-AACAAAUUC-3', and SEQ ID NOS: 66 to 68; and
a sequence in which the following sequences are linked to each other in a 5' to 3' direction:
a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
5'-AUGCAAC-3'.

In an embodiment, a sequence of the engineered scaffold region in which the second region and the fourth and fifth regions are modified may comprise in a 5' to 3' direction: 
5'-
CUUCACUGAUAAAGUGGAGAACCGCUUCACCAUUAGUGAGUGAAGGUGG
GCUGCUUGCAUCAGCCUAAUGUCGAGAAGUGCUUUCUUCGGAAAGUAAC
CCUCGAAACAAA-3' (SEQ ID NO: 158);
and
5'-GGAAUGCAAC-3' (SEQ ID NO: 160).

In an embodiment, a sequence of the engineered scaffold region in which the second region, and the fourth and fifth regions are modified may be a sequence in which the following sequences are linked to each other in a 5' to 3' direction:
SEQ ID NO: 9,
a sequence selected from the group consisting of SEQ ID NOS: 38 to 49, and SEQ ID NOS: 430 to 431,
SEQ ID NO: 11,
a sequence selected from SEQ ID NOS: 110 to 116, and
5'-AUGCAAC-3'.

In an embodiment, a sequence of the engineered scaffold region in which the second region and the fourth and fifth regions are modified may be 5'-CUUCACUGAUAAAGUGGAGAACCGCUUCACCAUUAGUGAGUGAAGGUGG GCUGCUUGCAUCAGCCUAAUGUCGAGAAGUGCUUUCUUCGGAAAGUAAC CCUCGAAACAAAGAAAGGAAUGCAAC-3' (SEQ ID NO: 208).

### Combination 4 of respective modifications - Modification of first region, modification of second region, and modification of fourth and fifth regions

In an embodiment, the engineered scaffold region may comprise a modified first region, a modified second region, and modified fourth and fifth regions. Here, the modified first region includes all of the modifications described in the section "Engineered scaffold region 1 - Modification of first region." Here, the modified second region includes all of the modifications described in the section "Engineered scaffold region 2 - Modification of second region." Here, the modified fourth and fifth regions include all of the modifications described in the section "Engineered scaffold region 4 - Modification of fourth and fifth regions."

In an embodiment, a sequence of the engineered scaffold region in which the first region, the second region, and the fourth and fifth regions are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
a sequence selected from SEQ ID NOS: 38 to 49, and SEQ ID NOS: 430 to 431,
SEQ ID NO: 11, and
a sequence selected from the group consisting of 5'-AACAAA-3', 5'-AACAAAU-3', 5'-AACAAAUU-3', 5'-AACAAAUUC-3', and SEQ ID NOS: 66 to 68; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the first region, the second region, and the fourth and fifth regions are modified may comprise in a 5' to 3' direction: 
5'-
ACCGCUUCACCAUUAGUGAGUGAAGGUGGGCUGCUUGCAUCAGCCUAA
UGUCGAGAAGUGCUUUCUUCGGAAAGUAACCCUCGAAACAAAUUCAUUU-
3' (SEQ ID NO: 159);
and
5'-GGAAUGCAAC-3' (SEQ ID NO: 160).

In an embodiment, a sequence of the engineered scaffold region in which the first region, the second region, and fourth and fifth regions are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOS: 16 to 26,
a sequence selected from the group consisting of SEQ ID NOS: 38 to 49, and SEQ ID NOS: 430 to 431,
SEQ ID NO: 11,
a sequence selected from the group consisting of SEQ ID NOS: 110 to 116, and
5'-AUGCAAC-3'.

In an embodiment, a sequence of the engineered scaffold region in which the first region, the second region, and the fourth and fifth regions are modified may be 5'-ACCGCUUCACCAUUAGUGAGUGAAGGUGGGCUGCUUGCAUCAGCCUAA UGUCGAGAAGUGCUUUCUUCGGAAAGUAACCCUCGAAACAAAGAAAGGA AUGCAAC-3' (SEQ ID NO: 209).

### Combination 5 of respective modifications - Additional modifications of third region and sixth region

As described above, since the third region and the sixth region may also be modified within a range in which their function is not impaired, the engineered scaffold region provided herein may be one in which the third region and/or the sixth region is additionally modified in addition to the modification(s) of the first region, the second region, the fourth region, and/or the fifth region as described above.

### Engineered Scaffold Region 7 - Inclusion of sequence having homology

The engineered scaffold region provided herein comprises a sequence having identity with the sequences of the engineered scaffold region (hereinafter, referred to as the above-described engineered scaffold region) described in the sections of "Engineered scaffold region 1 - Modification of first region," "Engineered scaffold region 2 - Modification of second region," "Engineered scaffold region 3 - Modification of third region," "Engineered scaffold region 4 - Modification of fourth and fifth regions," and "Engineered scaffold region 6 - Combination of respective modifications."

In an embodiment, a sequence of the engineered scaffold region may be a sequence having sequence identity or sequence homology of 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, or 50% to any one of the sequences of the above-described engineered scaffold region. In an embodiment, the scaffold sequence may be a sequence that is identical to any one of the sequences of the above-described engineered scaffold region by a percentage falling within a range between two numbers selected from the immediately preceding sentence. For example, the scaffold sequence may be a sequence that is 90% to 100% identical to any one of the sequences of the above-described engineered scaffold region.

### Engineered Cas12f1 guide RNA

### Overview of engineered Cas12f1 guide RNA

In the present disclosure, there is provided an engineered Cas12f1 guide RNA for increasing gene editing efficiency of the CRISPR/Cas12f1 system in a cell. The engineered Cas12f1 guide RNA comprises an engineered scaffold region and a spacer. Here, the engineered scaffold may be any one of those described in the above-described "Engineered scaffold region."

### Single guide RNA or dual guide RNA

The engineered Cas12f1 guide RNA may be a single guide RNA or a dual guide RNA. The dual guide RNA refers to a guide RNA which consists of two independent RNA molecules of a tracrRNA and a crRNA. The single guide RNA refers to a molecule formed by linking the 3' end of a (engineered) tracrRNA and the 5' end of a (engineered) crRNA through a linker. In other words, the single guide RNA means a molecule obtained by linking the 3' end of a fourth region and the 5' end of a fifth region through a linker, wherein the fourth and fifth regions are included in the engineered scaffold of the dual guide RNA. Here, the respective regions of the engineered scaffold may include all of the modifications, and specific sequences thereof, as described in the sections of "Engineered scaffold region."

### Example 1 of engineered single guide RNA

In an embodiment, the engineered Cas12f1 guide RNA may be one in which an engineered scaffold region and a spacer are sequentially linked to each other in a 5' to 3' direction.

The spacer has a length of 10 to 50 nucleotides and has a sequence complementary to a target sequence.

The engineered scaffold region is one in which a first region, a second region, a third region, a fourth region, a linker, a fifth region, and a sixth region, which correspond to those of the naturally occurring scaffold region, are sequentially linked to each other in a 5' to 3' direction, wherein one or more regions selected from the first region, the second region, the fourth region, and the fifth region are modified as compared with the naturally occurring scaffold region.

As an example, when a first region of the engineered scaffold region is modified, the modified first region may be a modified form of a first region of the naturally occurring scaffold region from which one or more nucleotides are removed. Here, the removed nucleotide may be a nucleotide belonging to Stem 1 in the first region (Takeda et al., Structure of the miniature type V-F CRISPR-Cas effector enzyme, Molecular Cell 81, 1-13 (2021)). Here, a sequence of the modified first region is characterized by comprising 5'-A-3'.

As an example, when a second region of the engineered scaffold region is modified, the modified second region may be a modified form of a first region of the naturally occurring scaffold region from which one or more nucleotides are removed. Here, the removal of the nucleotide may occur in a portion that forms a Stem 2 structure of the second region (Takeda et al., Structure of the miniature type V-F CRISPR-Cas effector enzyme, Molecular Cell 81, 1-13 (2021)), and nucleotides forming pairs complementary to each other may be removed by units of pairs. Here, a sequence of the modified second region is characterized by comprising at least 5'-CCGCUUCAC-3' (SEQ ID NO: 432) and 5'-UGAGUGAAGG-3' (SEQ ID NO: 433). More specifically, a sequence of the modified second region may be one in which 5'-CCGCUUCAC-3' (SEQ ID NO: 432) and 5'-UGAGUGAAGG-3' (SEQ ID NO: 433) are sequentially linked to each other in a 5' to 3' direction, wherein the sequences may be linked by an appropriate intermediate sequence. As an example, the intermediate sequence may be selected from the group consisting of 5'-UUAG-3', 5'-AUUAGU-3', 5'-AAUUAGCU-3', 5'-AAAUUAGACU-3' (SEQ ID NO: 57), 5'-AAAGUUAGAACU-3' (SEQ ID NO: 58), 5'-AAAGCUUAGGAACU-3' (SEQ ID NO: 59), 5'-AAAGCUUUAGAGAACU-3' (SEQ ID NO: 60), 5'-AAAGCUGUUAGUUAGAACU-3' (SEQ ID NO: 61), 5'-AAAGCUGUUAGUAGAACU-3' (SEQ ID NO: 62), 5'-AAAGCUGUUUAGAUUAGAACU-3' (SEQ ID NO: 63), 5'-AAAGCUGUCUUAGGAUUAGAACU-3' (SEQ ID NO: 64), 5'-AAAGCUGUCCUUAGGGAUUAGAACU-3' (SEQ ID NO: 65), 5'-AAAAGCUGUCCCUUAGGGGAUUAGAACUU-3' (SEQ ID NO: 434), and 5'-CAAAAGCUGUCCCUUAGGGGAUUAGAACUUG-3' (SEQ ID NO: 435).

As another example, when the fourth and fifth regions of the engineered scaffold region are modified, the modified fourth and fifth regions may be modified forms of fourth region and/or the fifth region of the naturally occurring scaffold region from which one or more nucleotides are removed. Here, the removal of the nucleotides may occur in a portion that forms a Stem 5 (R:AR-2) structure in the fourth and fifth regions (Takeda et al., Structure of the miniature type V-F CRISPR-Cas effector enzyme, Molecular Cell 81, 1-13 (2021)), and such removal may be done in pairs of nucleotides that are complementary to each other. Here, a sequence of the modified fourth region is characterized by comprising at least 5'-AACAAA-3'. Here, the modified fifth region is characterized by comprising at least 5'-GGA-3'.

### Example 2 of engineered single guide RNA

In an embodiment, the engineered Cas12f1 guide RNA may be one in which an engineered scaffold region and a spacer are sequentially linked to each other in a 5' to 3' direction.

The spacer has a length of 10 to 50 nucleotides and has a sequence complementary to a target sequence.

A sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10); 5'-GCUGCUUGCAUCAGCCUAAUGUCGAGAAGUGCUUUCUUCGGAAAGUAAC CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
5'-AUGCAAC-3',
wherein the sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7).

### Example 3 of engineered single guide RNA

In an embodiment, the engineered Cas12f1 guide RNA may be one in which an engineered scaffold region, and a spacer are sequentially linked to each other in a 5' to 3' direction.

The spacer has a length of 10 to 50 nucleotides and has a sequence complementary to a target sequence. For example, the spacer may have a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides.

A sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
5'-GCUGCUUGCAUCAGCCUAAUGUCGAGAAGUGCUUUCUUCGGAAAGUAAC CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), 5'-AACAAAUUCAUUUGAAAGAAUGAAGGA-3' (SEQ ID NO: 117), 5'-AACAAAUUCAUUUUGAAACGAAUGAAGGA-3' (SEQ ID NO: 293), 5'-AACAAAUUCAUUUUUGAAAACGAAUGAAGGA-3' (SEQ ID NO: 294), 5'-AACAAAUUCAUUUUUCGAAAGACGAAUGAAGGA-3' (SEQ ID NO: 295), 5'-AACAAAUUCAUUUUUCCGAAAAGACGAAUGAAGGA-3' (SEQ ID NO: 296), 5'-AACAAAUUCAUUUUUCCUGAAAUAGACGAAUGAAGGA-3' (SEQ ID NO: 297), 5'-AACAAAUUCAUUUUUCCUCGAAAAUAGACGAAUGAAGGA-3' (SEQ ID NO: 298), 5'-AACAAAUUCAUUUUUCCUCUGAAAAAUAGACGAAUGAAGGA-3' (SEQ ID NO: 299), 5'-AACAAAUUCAUUUUUCCUCUCGAAAGAAUAGACGAAUGAAGGA-3' (SEQ ID NO: 300), 5'-AACAAAUUCAUUUUUCCUCUCCGAAACGAAUAGACGAAUGAAGGA-3' (SEQ ID NO: 301), 5'-AAC AAAU UCAUUUUUCCUCUC C AG AAAC C GAAU AGAC G AAU GAAG G A-3' (SEQ ID NO: 302), 5'-AACAAAUUCAUUUUUCCUCUCCAAGAAACCCGAAUAGACGAAUGAAGGA-3' (SEQ ID NO: 303), 5'-AACAAAUUCAUUUUUCCUCUCCAAUGAAAACCCGAAUAGACGAAUGAAGG A-3' (SEQ ID NO: 304), 5'-AACAAAUUCAUUUUUCCUCUCCAAUUGAAAAACCCGAAUAGACGAAUGAA GGA-3' (SEQ ID NO: 305), 5'-AACAAAUUCAUUUUUCCUCUCCAAUUCGAAAGAACCCGAAUAGACGAAUG AAGGA-3' (SEQ ID NO: 306), 5'-AACAAAUUCAUUUUUCCUCUCCAAUUCUGAAAAGAACCCGAAUAGACGAA UGAAGGA-3' (SEQ ID NO: 307), 5'-AACAAAUUCAUUUUUCCUCUCCAAUUCUGGAAACAGAACCCGAAUAGAC GAAUGAAGGA-3' (SEQ ID NO: 308), 5'-AACAAAUUCAUUUUUCCUCUCCAAUUCUGCGAAAGCAGAACCCGAAUAG ACGAAUGAAGGA-3' (SEQ ID NO: 309), 5'-AACAAAUUCAUUUUUCCUCUCCAAUUCUGCAGAAAUGCAGAACCCGAAUA GACGAAUGAAGGA-3' (SEQ ID NO: 310), 5'-AACAAAUUCAUUUUUCCUCUCCAAUUCUGCACGAAAUUGCAGAACCCGA AUAGACGAAUGAAGGA-3' (SEQ ID NO: 311), 5'-AACAAAUUCAUUUUUCCUCUCCAAUUCUGCACAGAAAGUUGCAGAACCC GAAUAGACGAAUGAAGGA-3' (SEQ ID NO: 312), 5'-AACAAAUUCAUUUUUCCUCUCCAAUUCUGCACAGAAAUUGCAGAACCCG AAUAGACGAAUGAAGGA-3' (SEQ ID NO: 313), and 5'-AACAAAUUCAUUUUUCCUCUCCAAUUCUGCACAAGAAAGUUGCAGAACC CGAAUAGACGAAUGAAGGA-3' (SEQ ID NO: 314); and
5'-AUGCAAC-3',

Here, a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAAGAAAGUUGCAGAACCCGAAUAGACGAAUGAAGGA AUGCAAC-3' (SEQ ID NO: 315).

### Example 4 of engineered single guide RNA

In an embodiment, the engineered Cas12f1 guide RNA may be one in which an engineered scaffold region and a spacer are sequentially linked to each other in a 5' to 3' direction.

The spacer has a length of 10 to 50 nucleotides and has a sequence complementary to a target sequence. For example, the spacer may have a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides.

A sequence of the engineered scaffold region is such that the following sequences are sequentially linked to each other in a 5' to 3' direction:
a first sequence represented by 5'-A-3';
a second sequence represented by 5'-CCGCUUCAC-3' (SEQ ID NO: 432);
a third sequence represented by 5'-UUAG-3';
a fourth sequence represented by 5'-UGAGUGAAGG-3' (SEQ ID NO: 433);
a fifth sequence represented by 5'-GCUGCUUGCAUCAGCCUAAUGUCGAGAAGUGCUUUCUUCGGAAAGUAAC CCUCGA-3' (SEQ ID NO: 11);
a sixth sequence represented by 5'-AACAAA-3';
a linker;
a seventh sequence represented by 5'-GGA-3'; and
an eighth sequence represented by 5'-AUGCAAC-3'.

As an example, the linker may be 5'-GAAA-3'.

As another example, the linker may be selected from the group consisting of 5'-GAAA-3', 5'-UGAAAA-3', 5'-UUGAAAAA-3', 5'-UUCGAAAGAA-3' (SEQ ID NO: 425), 5'-UUCAGAAAUGAA-3' (SEQ ID NO: 426), 5'-UUCAUGAAAAUGAA-3' (SEQ ID NO: 427), 5'-UUCAUUGAAAAAUGAA-3' (SEQ ID NO: 428), and 5'-UUCAUUUGAAAGAAUGAAGGA-3' (SEQ ID NO: 429).

As a specific example of the embodiment, a sequence of the engineered scaffold region may additionally comprise a ninth sequence selected from the group consisting of 5'-A-3', 5'-GA-3', 5'-AGA-3', 5'-GAGA-3', 5'-GGAGA-3', 5'-UGGAGA-3', 5'-GUGGAGA-3', 5'-AGUGGAGA-3', 5'-AAGUGGAGA-3', 5'-AAAGUGGAGA-3' (SEQ ID NO: 27), 5'-UAAAGUGGAGA-3' (SEQ ID NO: 28), 5'-AUAAAGUGGAGA-3' (SEQ ID NO: 29), 5'-GAUAAAGUGGAGA-3' (SEQ ID NO: 30), 5'-UGAUAAAGUGGAGA-3' (SEQ ID NO: 31), 5'-CUGAUAAAGUGGAGA-3' (SEQ ID NO: 32), 5'-ACUGAUAAAGUGGAGA-3' (SEQ ID NO: 33), 5'-CACUGAUAAAGUGGAGA-3' (SEQ ID NO: 34), 5'-UCACUGAUAAAGUGGAGA-3' (SEQ ID NO: 35), 5'-UUCACUGAUAAAGUGGAGA-3' (SEQ ID NO: 36), and 5'-CUUCACUGAUAAAGUGGAGA-3' (SEQ ID NO: 37). Here, the 3' end of the ninth sequence may be linked to the 5' end of the first sequence.

As another specific example of the embodiment, a sequence of the engineered scaffold region may additionally comprise a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 52), 5'-AAAGCUGUCCC-3' (SEQ ID NO: 53), 5'-AAAAGCUGUCCC-3' (SEQ ID NO: 440), and 5'-CAAAAGCUGUCCC-3' (SEQ ID NO: 441). Here, the 3' end of the second sequence and the 5' end of the third sequence may be linked by the tenth sequence.

As yet another specific example of the embodiment, a sequence of the engineered scaffold region may additionally comprise an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 54), 5'-GGAUUAGAACU-3' (SEQ ID NO: 55), 5'-GGGAUUAGAACU-3' (SEQ ID NO: 56), 5'-GGGAUUAGAACUU-3' (SEQ ID NO: 442), and 5'-GGGAUUAGAACUUG-3' (SEQ ID NO: 443). Here, the 3' end of the third sequence and the 5' end of the fourth sequence may be linked by the eleventh sequence.

As still yet another specific example of the embodiment, a sequence of the engineered scaffold region may additionally comprise a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 52), 5'-AAAGCUGUCCC-3' (SEQ ID NO: 53), 5'-AAAAGCUGUCCC-3' (SEQ ID NO: 440), and 5'-CAAAAGCUGUCCC-3' (SEQ ID NO: 441), and an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 54), 5'-GGAUUAGAACU-3' (SEQ ID NO: 55), 5'-GGGAUUAGAACU-3' (SEQ ID NO: 56), 5'-GGGAUUAGAACUU-3' (SEQ ID NO: 442), and 5'-GGGAUUAGAACUUG-3' (SEQ ID NO: 443). Here, the 3' end of the second sequence and the 5' end of the third sequence may be linked by the tenth sequence, and the 3' end of the third sequence and the 5' end of the fourth sequence may be linked by the eleventh sequence.

As an example, when the tenth sequence is 5'-A-3', the eleventh sequence may be 5'-U-3'. As another example, when the tenth sequence is 5'-AA-3', the eleventh sequence may be 5'-CU-3'. As yet another example, when the tenth sequence is 5'-AAA-3', the eleventh sequence may be 5'-ACU-3'. As still yet another example, when the tenth sequence is 5'-AAAG-3', the eleventh sequence may be 5'-AACU-3'. As still yet another example, when the tenth sequence is 5'-AAAGC-3', the eleventh sequence may be 5'-GAACU-3'. As still yet another example, when the tenth sequence is 5'-AAAGCU-3', the eleventh sequence may be 5'-AGAACU-3'. As still yet another example, when the tenth sequence is 5'-AAAGCUG-3', the eleventh sequence may be 5'-UAGAACU-3' or 5'-UUAGAACU-3'. As still yet another example, when the tenth sequence is 5'-AAAGCUGU-3', the eleventh sequence may be 5'-AUUAGAACU-3'. As still yet another example, when the tenth sequence is 5'-AAAGCUGUC-3', the eleventh sequence may be 5'-GAUUAGAACU-3' (SEQ ID NO: 54). As still yet another example, when the tenth sequence is 5'-AAAGCUGUCC-3' (SEQ ID NO: 52), the eleventh sequence may be 5'-GGAUUAGAACU-3' (SEQ ID NO: 55). As still yet another example, when the tenth sequence is 5'-AAAGCUGUCCC-3' (SEQ ID NO: 53), the eleventh sequence may be 5'-GGGAUUAGAACU-3' (SEQ ID NO: 56). As still yet another example, when the tenth sequence is 5'-AAAAGCUGUCCC-3' (SEQ ID NO: 440), the eleventh sequence may be 5'-GGGAUUAGAACUU-3' (SEQ ID NO: 442). As still yet another example, when the tenth sequence is 5'-CAAAAGCUGUCCC-3' (SEQ ID NO: 441), the eleventh sequence may be 5'-GGGAUUAGAACUUG-3' (SEQ ID NO: 443).

As still yet another specific example of the embodiment, a sequence of the engineered scaffold region may additionally comprise a twelfth sequence selected from the group consisting of 5'-U-3', 5'-UU-3', 5'-UUC-3', 5'-UUCA-3', 5'-UUCAU-3', 5'-UUCAUU-3', and 5'-UUCAUUU-3'. Here, the 3' end of the sixth sequence and the 5' end of the linker may be linked by the twelfth sequence.

As still yet another specific example of the embodiment, a sequence of the engineered scaffold region may additionally comprise a thirteenth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3'. Here, the 3' end of the linker and the 5' end of the seventh sequence may be linked to each other by the thirteenth sequence.

As still yet another specific example of the embodiment, a sequence of the engineered scaffold region may additionally comprise a twelfth sequence selected from the group consisting of 5'-U-3', 5'-UU-3', 5'-UUC-3', 5'-UUCA-3', 5'-UUCAU-3', 5'-UUCAUU-3', and 5'-UUCAUUU-3', and a thirteenth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3'. Here, the 3' end of the sixth sequence and the 5' end of the linker may be linked by the twelfth sequence, and the 3' end of the linker and the 5' end of the seventh sequence may be linked by the thirteenth sequence.

As an example, when the twelfth sequence is 5'-U-3', the thirteenth sequence may be 5'-A-3'. As another example, when the twelfth sequence is 5'-UU-3', the thirteenth sequence may be 5'-AA-3'. As yet another example, when the twelfth sequence is 5'-UUC-3', the thirteenth sequence may be 5'-GAA-3'. As still yet another example, when the twelfth sequence is 5'-UUCA-3', the thirteenth sequence may be 5'-UGAA-3'. As still yet another example, when the twelfth sequence is 5'-UUCAU-3', the thirteenth sequence may be 5'-AUGAA-3'. As still yet another example, when the twelfth sequence is 5'-UUCAUU-3', the thirteenth sequence may be 5'-AAUGAA-3'. As still yet another example, when the twelfth sequence is 5'-UUCAUUU-3', the thirteenth sequence may be 5'-GAAUGAA-3'.

### Example 5 of engineered single guide RNA

In an embodiment, the engineered Cas12f1 guide RNA may be one in which an engineered scaffold region and a spacer are sequentially linked to each other in a 5' to 3' direction.

The spacer has a length of 10 to 50 nucleotides and has a sequence complementary to a target sequence. For example, the spacer may have a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides.

A sequence of the engineered scaffold region is such that the following sequences are sequentially linked to each other in a 5' to 3' direction:
a first sequence represented by 5'-A-3';
a second sequence represented by 5'-CCGCUUCAC-3' (SEQ ID NO: 432);
a third sequence represented by 5'-UUAG-3';
a fourth sequence represented by 5'-UGAGUGAAGG-3' (SEQ ID NO: 433);
a fifth sequence represented by 5'-GCUGCUUGCAUCAGCCUAAUGUCGAGAAGUGCUUUCUUCGGAAAGUAAC CCUCGA-3' (SEQ ID NO: 11);
a sixth sequence represented by 5'-AACAAA-3';
a linker;
a seventh sequence represented by 5'-GGA-3'; and
an eighth sequence represented by 5'-AUGCAAC-3'.

In an example, the linker may be 5'-GAAA-3'.

As another example, the linker may be selected from the group consisting of 5'-GAAA-3', 5'-AGAAAG-3', 5'-AAGAAAGU-3', 5'-CAAGAAAGUU-3' (SEQ ID NO: 316), 5'-ACAAGAAAGUUG-3' (SEQ ID NO: 317), 5'-CACAAGAAAGUUGC-3' (SEQ ID NO: 318), 5'-GCACAAGAAAGUUGCA-3' (SEQ ID NO: 319), 5'-UGCACAAGAAAGUUGCAG-3' (SEQ ID NO: 320), 5'-CUGCACAAGAAAGUUGCAGA-3' (SEQ ID NO: 321), 5'-UCUGCACAAGAAAGUUGCAGAA-3' (SEQ ID NO: 322), 5'-UUCUGCACAAGAAAGUUGCAGAAC-3' (SEQ ID NO: 323), 5'-AUUCUGCACAAGAAAGUUGCAGAACC-3' (SEQ ID NO: 324), 5'-AAUUCUGCACAAGAAAGUUGCAGAACCC-3' (SEQ ID NO: 325), 5'-CAAUUCUGCACAAGAAAGUUGCAGAACCCG-3' (SEQ ID NO: 326), 5'-CCAAUUCUGCACAAGAAAGUUGCAGAACCCGA-3' (SEQ ID NO: 327), 5'-UCCAAUUCUGCACAAGAAAGUUGCAGAACCCGAA-3' (SEQ ID NO: 328), 5'-CUCCAAUUCUGCACAAGAAAGUUGCAGAACCCGAAU-3' (SEQ ID NO: 329), 5'-UCUCCAAUUCUGCACAAGAAAGUUGCAGAACCCGAAUA-3' (SEQ ID NO: 330), 5'-CUCUCCAAUUCUGCACAAGAAAGUUGCAGAACCCGAAUAG-3' (SEQ ID NO: 331), 5'-CCUCUCCAAUUCUGCACAAGAAAGUUGCAGAACCCGAAUAGA-3' (SEQ ID NO: 332), 5'-UCCUCUCCAAUUCUGCACAAGAAAGUUGCAGAACCCGAAUAGAC-3' (SEQ ID NO: 333), 5'-UUCCUCUCCAAUUCUGCACAAGAAAGUUGCAGAACCCGAAUAGACG-3' (SEQ ID NO: 334), 5'-UUUCCUCUCCAAUUCUGCACAAGAAAGUUGCAGAACCCGAAUAGACGA-3' (SEQ ID NO: 335), 5'-UUUUCCUCUCCAAUUCUGCACAAGAAAGUUGCAGAACCCGAAUAGACGA A-3' (SEQ ID NO: 336), 5'-UUUUUCCUCUCCAAUUCUGCACAAGAAAGUUGCAGAACCCGAAUAGACG AAU-3' (SEQ ID NO: 337), 5'-AUUUUUCCUCUCCAAUUCUGCACAAGAAAGUUGCAGAACCCGAAUAGAC GAAUG-3' (SEQ ID NO: 338), 5'-CAUUUUUCCUCUCCAAUUCUGCACAAGAAAGUUGCAGAACCCGAAUAGA CGAAUGA-3' (SEQ ID NO: 339), 5'-UCAUUUUUCCUCUCCAAUUCUGCACAAGAAAGUUGCAGAACCCGAAUAG ACGAAUGAA-3' (SEQ ID NO: 340), 5'-UCAUUUUUCCUCUCCAAUUCUGCACAAGAAAGUUGCAGAACCCGAAUAG ACGAAUGA-3' (SEQ ID NO: 341), and 5'-UUCAUUUUUCCUCUCCAAUUCUGCACAAGAAAGUUGCAGAACCCGAAUA GACGAAUGAA-3' (SEQ ID NO: 342).

As a specific example of the embodiment, a sequence of the engineered scaffold region may additionally comprise a ninth sequence selected from the group consisting of 5'-A-3', 5'-GA-3', 5'-AGA-3', 5'-GAGA-3', 5'-GGAGA-3', 5'-UGGAGA-3', 5'-GUGGAGA-3', 5'-AGUGGAGA-3', 5'-AAGUGGAGA-3', 5'-AAAGUGGAGA-3' (SEQ ID NO: 27), 5'-UAAAGUGGAGA-3' (SEQ ID NO: 28), 5'-AUAAAGUGGAGA-3' (SEQ ID NO: 29), 5'-GAUAAAGUGGAGA-3' (SEQ ID NO: 30), 5'-UGAUAAAGUGGAGA-3' (SEQ ID NO: 31), 5'-CUGAUAAAGUGGAGA-3' (SEQ ID NO: 32), 5'-ACUGAUAAAGUGGAGA-3' (SEQ ID NO: 33), 5'-CACUGAUAAAGUGGAGA-3' (SEQ ID NO: 34), 5'-UCACUGAUAAAGUGGAGA-3' (SEQ ID NO: 35), 5'-UUCACUGAUAAAGUGGAGA-3' (SEQ ID NO: 36), and 5'-CUUCACUGAUAAAGUGGAGA-3' (SEQ ID NO: 37). Here, the 3' end of the ninth sequence may be linked to the 5' end of the first sequence.

As another specific example of the embodiment, a sequence of the engineered scaffold region may additionally comprise a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 52), 5'-AAAGCUGUCCC-3' (SEQ ID NO: 53), 5'-AAAAGCUGUCCC-3' (SEQ ID NO: 440), and 5'-CAAAAGCUGUCCC-3' (SEQ ID NO: 441). Here, the 3' end of the second sequence and the 5' end of the third sequence may be linked by the tenth sequence.

As yet another specific example of the embodiment, a sequence of the engineered scaffold region may additionally comprise an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 54), 5'-GGAUUAGAACU-3' (SEQ ID NO: 55), 5'-GGGAUUAGAACU-3' (SEQ ID NO: 56), 5'-GGGAUUAGAACUU-3' (SEQ ID NO: 442), and 5'-GGGAUUAGAACUUG-3' (SEQ ID NO: 443). Here, the 3' end of the third sequence and the 5' end of the fourth sequence may be linked by the eleventh sequence.

As still yet another specific example of the embodiment, a sequence of the engineered scaffold region may additionally comprise a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 52), 5'-AAAGCUGUCCC-3' (SEQ ID NO: 53), 5'-AAAAGCUGUCCC-3' (SEQ ID NO: 440), and 5'-CAAAAGCUGUCCC-3' (SEQ ID NO: 441), and an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 54), 5'-GGAUUAGAACU-3' (SEQ ID NO: 55), 5'-GGGAUUAGAACU-3' (SEQ ID NO: 56), 5'-GGGAUUAGAACUU-3' (SEQ ID NO: 442), and 5'-GGGAUUAGAACUUG-3' (SEQ ID NO: 443). Here, the 3' end of the second sequence and the 5' end of the third sequence may be linked by the tenth sequence, and the 3' end of the third sequence and the 5' end of the fourth sequence may be linked by the eleventh sequence.

As an example, when the tenth sequence is 5'-A-3', the eleventh sequence may be 5'-U-3'. As another example, when the tenth sequence is 5'-AA-3', the eleventh sequence may be 5'-CU-3'. As yet another example, when the tenth sequence is 5'-AAA-3', the eleventh sequence may be 5'-ACU-3'. As still yet another example, when the tenth sequence is 5'-AAAG-3', the eleventh sequence may be 5'-AACU-3'. As still yet another example, when the tenth sequence is 5'-AAAGC-3', the eleventh sequence may be 5'-GAACU-3'. As still yet another example, when the tenth sequence is 5'-AAAGCU-3', the eleventh sequence may be 5'-AGAACU-3'. As still yet another example, when the tenth sequence is 5'-AAAGCUG-3', the eleventh sequence may be 5'-UAGAACU-3' or 5'-UUAGAACU-3'. As still yet another example, when the tenth sequence is 5'-AAAGCUGU-3', the eleventh sequence may be 5'-AUUAGAACU-3'. As still yet another example, when the tenth sequence is 5'-AAAGCUGUC-3', the eleventh sequence may be 5'-GAUUAGAACU-3' (SEQ ID NO: 54). As still yet another example, when the tenth sequence is 5'-AAAGCUGUCC-3' (SEQ ID NO: 52), the eleventh sequence may be 5'-GGAUUAGAACU-3' (SEQ ID NO: 55). As still yet another example, when the tenth sequence is 5'-AAAGCUGUCCC-3' (SEQ ID NO: 53), the eleventh sequence may be 5'-GGGAUUAGAACU-3' (SEQ ID NO: 56). As still yet another example, when the tenth sequence is 5'-AAAAGCUGUCCC-3' (SEQ ID NO: 440), the eleventh sequence may be 5'-GGGAUUAGAACUU-3' (SEQ ID NO: 442). As still yet another example, when the tenth sequence is 5'-CAAAAGCUGUCCC-3' (SEQ ID NO: 441), the eleventh sequence may be 5'-GGGAUUAGAACUUG-3' (SEQ ID NO: 443).

As still yet another specific example of the embodiment, a sequence of the engineered scaffold region may comprise a twelfth sequence selected from the group consisting of 5'-U-3', 5'-UU-3', 5'-UUC-3', 5'-UUCA-3', 5'-UUCAU-3', 5'-UUCAUU-3', 5'-UUCAUUU-3', 5'-UUCAUUUU-3', 5'-UUCAUUUUU-3', 5'-UUCAUUUUUC-3' (SEQ ID NO: 343), 5'-UUCAUUUUUCC-3' (SEQ ID NO: 344), 5'-UUCAUUUUUCCU-3' (SEQ ID NO: 345), 5'-UUCAUUUUUCCUC-3' (SEQ ID NO: 346), 5'-UUCAUUUUUCCUCU-3' (SEQ ID NO: 347), 5'-UUCAUUUUUCCUCUC-3' (SEQ ID NO: 348), 5'-UUCAUUUUUCCUCUCC-3' (SEQ ID NO: 349), 5'-UUCAUUUUUCCUCUCCA-3' (SEQ ID NO: 350), 5'-UUCAUUUUUCCUCUCCAA-3' (SEQ ID NO: 351), 5'-UUCAUUUUUCCUCUCCAAU-3' (SEQ ID NO: 352), 5'-UUCAUUUUUCCUCUCCAAUU-3' (SEQ ID NO: 353), 5'-UUCAUUUUUCCUCUCCAAUUC-3' (SEQ ID NO: 354), 5'-UUCAUUUUUCCUCUCCAAUUCU-3' (SEQ ID NO: 355), 5'-UUCAUUUUUCCUCUCCAAUUCUG-3' (SEQ ID NO: 356), 5'-UUCAUUUUUCCUCUCCAAUUCUGC-3' (SEQ ID NO: 357), 5'-UUCAUUUUUCCUCUCCAAUUCUGCA-3' (SEQ ID NO: 358), 5'-UUCAUUUUUCCUCUCCAAUUCUGCAC-3' (SEQ ID NO: 359), 5'-UUCAUUUUUCCUCUCCAAUUCUGCACA-3' (SEQ ID NO: 360), and 5'-UUCAUUUUUCCUCUCCAAUUCUGCACAA-3' (SEQ ID NO: 361). Here, the 3' end of the sixth sequence and the 5' end of the linker may be linked by the twelfth sequence.

As still yet another specific example of the embodiment, a sequence of the engineered scaffold region may additionally comprise a thirteenth sequence selected from the group consisting of 5'-A-3', 5'-AA-3', 5'-GAA-3', 5'-UGAA-3', 5'-AUGAA-3', 5'-AAUGAA-3', 5'-GAAUGAA-3', 5'-CGAAUGAA-3', 5'-ACGAAUGAA-3', 5'-GACGAAUGAA-3' (SEQ ID NO: 362), 5'-AGACGAAUGAA-3' (SEQ ID NO: 363), 5'-UAGACGAAUGAA-3' (SEQ ID NO: 364), 5'-AUAGACGAAUGAA-3' (SEQ ID NO: 365), 5'-AAUAGACGAAUGAA-3' (SEQ ID NO: 366), 5'-GAAUAGACGAAUGAA-3' (SEQ ID NO: 367), 5'-CGAAUAGACGAAUGAA-3' (SEQ ID NO: 368), 5'-CCGAAUAGACGAAUGAA-3' (SEQ ID NO: 369), 5'-CCCGAAUAGACGAAUGAA-3' (SEQ ID NO: 370), 5'-ACCCGAAUAGACGAAUGAA-3' (SEQ ID NO: 371), 5'-AACCCGAAUAGACGAAUGAA-3' (SEQ ID NO: 372), 5'-GAACCCGAAUAGACGAAUGAA-3' (SEQ ID NO: 373), 5'-AGAACCCGAAUAGACGAAUGAA-3' (SEQ ID NO: 374), 5'-CAGAACCCGAAUAGACGAAUGAA-3' (SEQ ID NO: 375), 5'-GCAGAACCCGAAUAGACGAAUGAA-3' (SEQ ID NO: 376), 5'-UGCAGAACCCGAAUAGACGAAUGAA-3' (SEQ ID NO: 377), 5'-UUGCAGAACCCGAAUAGACGAAUGAA-3' (SEQ ID NO: 378), and 5'-GUUGCAGAACCCGAAUAGACGAAUGAA-3' (SEQ ID NO: 379). Here, the 3' end of the linker and the 5' end of the seventh sequence may be linked by the thirteenth sequence.

As still yet another specific example of the embodiment, a sequence of the engineered scaffold region may additionally comprise a twelfth sequence selected from the group consisting of 5'-U-3', 5'-UU-3', 5'-UUC-3', 5'-UUCA-3', 5'-UUCAU-3', 5'-UUCAUU-3', 5'-UUCAUUU-3', 5'-UUCAUUUU-3', 5'-UUCAUUUUU-3', 5'-UUCAUUUUUC-3' (SEQ ID NO: 343), 5'-UUCAUUUUUCC-3' (SEQ ID NO: 344), 5'-UUCAUUUUUCCU-3' (SEQ ID NO: 345), 5'-UUCAUUUUUCCUC-3' (SEQ ID NO: 346), 5'-UUCAUUUUUCCUCU-3' (SEQ ID NO: 347), 5'-UUCAUUUUUCCUCUC-3' (SEQ ID NO: 348), 5'-UUCAUUUUUCCUCUCC-3' (SEQ ID NO: 349), 5'-UUCAUUUUUCCUCUCCA-3' (SEQ ID NO: 350), 5'-UUCAUUUUUCCUCUCCAA-3' (SEQ ID NO: 351), 5'-UUCAUUUUUCCUCUCCAAU-3' (SEQ ID NO: 352), 5'-UUCAUUUUUCCUCUCCAAUU-3' (SEQ ID NO: 353), 5'-UUCAUUUUUCCUCUCCAAUUC-3' (SEQ ID NO: 354), 5'-UUCAUUUUUCCUCUCCAAUUCU-3' (SEQ ID NO: 355), 5'-UUCAUUUUUCCUCUCCAAUUCUG-3' (SEQ ID NO: 356), 5'-UUCAUUUUUCCUCUCCAAUUCUGC-3' (SEQ ID NO: 357), 5'-UUCAUUUUUCCUCUCCAAUUCUGCA-3' (SEQ ID NO: 358), 5'-UUCAUUUUUCCUCUCCAAUUCUGCAC-3' (SEQ ID NO: 359), 5'-UUCAUUUUUCCUCUCCAAUUCUGCACA-3' (SEQ ID NO: 360), and 5'-UUCAUUUUUCCUCUCCAAUUCUGCACAA-3' (SEQ ID NO: 361), and a thirteenth sequence selected from the group consisting of 5'-A-3', 5'-AA-3', 5'-GAA-3', 5'-UGAA-3', 5'-AUGAA-3', 5'-AAUGAA-3', 5'-GAAUGAA-3', 5'-CGAAUGAA-3', 5'-ACGAAUGAA-3', 5'-GACGAAUGAA-3' (SEQ ID NO: 362), 5'-AGACGAAUGAA-3' (SEQ ID NO: 363), 5'-UAGACGAAUGAA-3' (SEQ ID NO: 364), 5'-AUAGACGAAUGAA-3' (SEQ ID NO: 365), 5'-AAUAGACGAAUGAA-3' (SEQ ID NO: 366), 5'-GAAUAGACGAAUGAA-3' (SEQ ID NO: 367), 5'-CGAAUAGACGAAUGAA-3' (SEQ ID NO: 368), 5'-CCGAAUAGACGAAUGAA-3' (SEQ ID NO: 369), 5'-CCCGAAUAGACGAAUGAA-3' (SEQ ID NO: 370), 5'-ACCCGAAUAGACGAAUGAA-3' (SEQ ID NO: 371), 5'-AACCCGAAUAGACGAAUGAA-3' (SEQ ID NO: 372), 5'-GAACCCGAAUAGACGAAUGAA-3' (SEQ ID NO: 373), 5'-AGAACCCGAAUAGACGAAUGAA-3' (SEQ ID NO: 374), 5'-CAGAACCCGAAUAGACGAAUGAA-3' (SEQ ID NO: 375), 5'-GCAGAACCCGAAUAGACGAAUGAA-3' (SEQ ID NO: 376), 5'-UGCAGAACCCGAAUAGACGAAUGAA-3' (SEQ ID NO: 377), 5'-UUGCAGAACCCGAAUAGACGAAUGAA-3' (SEQ ID NO: 378), and 5'-GUUGCAGAACCCGAAUAGACGAAUGAA-3' (SEQ ID NO: 379). Here, the 3' end of the sixth sequence and the 5' end of the linker may be linked by the twelfth sequence, and the 3' end of the linker and the 5' end of the seventh sequence may be linked by the thirteenth sequence.

As an example, when the twelfth sequence is 5'-U-3', the thirteenth sequence may be 5'-A-3'. As another example, when the twelfth sequence is 5'-UU-3', the thirteenth sequence may be 5'-AA-3'. As yet another example, when the twelfth sequence is 5'-UUC-3', the thirteenth sequence may be 5'-GAA-3'. As still yet another example, when the twelfth sequence is 5'-UUCA-3', the thirteenth sequence may be 5'-UGAA-3'. As still yet another example, when the twelfth sequence is 5'-UUCAU-3', the thirteenth sequence may be 5'-AUGAA-3'. As still yet another example, when the twelfth sequence is 5'-UUCAUU-3', the thirteenth sequence may be 5'-AAUGAA-3'. As still yet another example, when the twelfth sequence is 5'-UUCAUUU-3', the thirteenth sequence may be 5'-GAAUGAA-3'. As still yet another example, when the twelfth sequence is 5'-UUCAUUU-3', the thirteenth sequence may be 5'-GAAUGAA-3'. As still yet another example, when the twelfth sequence is 5'-UUCAUUUUU-3', the thirteenth sequence may be 5'-ACGAAUGAA-3'. As still yet another example, when the twelfth sequence is 5'-UUCAUUUUUC-3', the thirteenth sequence may be 5'-GACGAAUGAA-3'. As still yet another example, when the twelfth sequence is 5'-UUCAUUUUUCC-3', the thirteenth sequence may be 5'-AGACGAAUGAA-3'. As still yet another example, when the twelfth sequence is 5'-UUCAUUUUUCCU-3', the thirteenth sequence may be 5'-UAGACGAAUGAA-3'. As still yet another example, when the twelfth sequence is 5'-UUCAUUUUUCCUC-3', the thirteenth sequence may be 5'-AUAGACGAAUGAA-3'. As still yet another example, when the twelfth sequence is 5'-UUCAUUUUUCCUCU-3', the thirteenth sequence may be 5'-AAUAGACGAAUGAA-3'. As still yet another example, when the twelfth sequence is 5'-UUCAUUUUUCCUCUC-3', the thirteenth sequence may be 5'-GAAUAGACGAAUGAA-3'. As still yet another example, when the twelfth sequence is 5'-UUCAUUUUUCCUCUCC-3', the thirteenth sequence may be 5'-CGAAUAGACGAAUGAA-3'. As still yet another example, when the twelfth sequence is 5'-UUCAUUUUUCCUCUCCA-3', the thirteenth sequence may be 5'-CCGAAUAGACGAAUGAA-3'. As still yet another example, when the twelfth sequence is 5'-UUCAUUUUUCCUCUCCAA-3', the thirteenth sequence may be 5'-CCCGAAUAGACGAAUGAA-3'. As still yet another example, when the twelfth sequence is 5'-UUCAUUUUUCCUCUCCAAU-3', the thirteenth sequence may be 5'-ACCCGAAUAGACGAAUGAA-3'. As still yet another example, when the twelfth sequence is 5'-UUCAUUUUUCCUCUCCAAUU-3', the thirteenth sequence may be 5'-AACCCGAAUAGACGAAUGAA-3'. As still yet another example, when the twelfth sequence is 5'-UUCAUUUUUCCUCUCCAAUUC-3', the thirteenth sequence may be 5'-GAACCCGAAUAGACGAAUGAA-3'. As still yet another example, when the twelfth sequence is 5'-UUCAUUUUUCCUCUCCAAUUCU-3', the thirteenth sequence may be 5'-AGAACCCGAAUAGACGAAUGAA-3'. As still yet another example, when the twelfth sequence is 5'-UUCAUUUUUCCUCUCCAAUUCUG-3', the thirteenth sequence may be 5'-CAGAACCCGAAUAGACGAAUGAA-3'. As still yet another example, when the twelfth sequence is 5'-UUCAUUUUUCCUCUCCAAUUCUGC-3', the thirteenth sequence may be 5'-GCAGAACCCGAAUAGACGAAUGAA-3'. As still yet another example, when the twelfth sequence is 5'-UUCAUUUUUCCUCUCCAAUUCUGCA-3', the thirteenth sequence may be 5'-UGCAGAACCCGAAUAGACGAAUGAA-3'. As still yet another example, when the twelfth sequence is 5'-UUCAUUUUUCCUCUCCAAUUCUGCAC-3', the thirteenth sequence may be 5'-UUGCAGAACCCGAAUAGACGAAUGAA-3'. As still yet another example, when the twelfth sequence is 5'-UUCAUUUUUCCUCUCCAAUUCUGCACA-3', or 5'-UUCAUUUUUCCUCUCCAAUUCUGCACAA-3', the thirteenth sequence may be 5'-GUUGCAGAACCCGAAUAGACGAAUGAA-3'.

### Example of engineered single guide RNA sequence

In an embodiment, the engineered single guide RNA may have a sequence selected from the group consisting of SEQ ID NOS: 210 to 258, SEQ ID NOS: 381 to 393, SEQ ID NOS: 396 to 407, SEQ ID NOS: 409 to 421, and SEQ ID NOS: 436 to 439.

### Example 1 of engineered dual guide RNA

In an embodiment, the engineered Cas12f1 guide RNA may comprise an engineered scaffold region and a spacer.

The spacer has a length of 10 to 50 nucleotides and has a sequence complementary to a target sequence. For example, the spacer may have a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides.

The sequence of the engineered scaffold region comprises in a 5' to 3' direction:
an engineered tracrRNA in which the following sequences are linked to each other:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9),
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10),
5'-GCUGCUUGCAUCAGCCUAAUGUCGAGAAGUGCUUUCUUCGGAAAGUAAC CCUCGA-3' (SEQ ID NO: 11),
   and
a sequence selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 66), 5'-AACAAAUUCAU-3' (SEQ ID NO: 67), 5'-AACAAAUUCAUU-3' (SEQ ID NO: 68), and 5'-AACAAAUUCAUUU-3' (SEQ ID NO: 12); and
an engineered crRNA repeat sequence portion in which the following sequences are linked to each other:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', and 5'-GAAUGAAGGA-3' (SEQ ID NO: 14), and
   5'-AUGCAAC-3',
   wherein the 3' end of the engineered crRNA repeat sequence portion is linked to the 5' end of the spacer.

Here, a sequence of the engineered tracrRNA may be different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUU-3' (SEQ ID NO: 1) and/or the engineered crRNA repeat sequence portion may be different from 5'-GAAUGAAGGAAUGCAAC-3' (SEQ ID NO: 3).

As an example, a sequence of the engineered tracrRNA may be the same as the sequence of SEQ ID NO: 1, and the engineered crRNA repeat sequence portion may be different from the sequence of SEQ ID NO: 3. As another example, a sequence of the engineered tracrRNA may be different from the sequence of SEQ ID NO: 1, and the engineered crRNA repeat sequence portion may be the same as the sequence of SEQ ID NO: 3. As yet another example, a sequence of the engineered tracrRNA may be different from the sequence of SEQ ID NO: 1, and the engineered crRNA repeat sequence portion may be different from the sequence of SEQ ID NO: 3.

As an example, when the engineered tracrRNA comprises 5'-AACAAA-3', the engineered crRNA may comprise 5'-GGA-3'. As another example, when the engineered tracrRNA comprises 5'-AACAAAU-3', the engineered crRNA may comprise 5'-AGGA-3'. As yet another example, when the engineered tracrRNA comprises 5'-AACAAAUU-3', the engineered crRNA may comprise 5'-AAGGA-3'. As still yet another example, when the engineered tracrRNA comprises 5'-AACAAAUUC-3', the engineered crRNA may comprise 5'-GAAGGA-3'. As still yet another example, when the engineered tracrRNA comprises 5'-AACAAAUUCA-3' (SEQ ID NO: 66), the engineered crRNA may comprise 5'-UGAAGGA-3'. As still yet another example, when the engineered tracrRNA comprises 5'-AACAAAUUCAU-3' (SEQ ID NO: 67), the engineered crRNA may comprise 5'-AUGAAGGA-3'. As still yet another example, when the engineered tracrRNA comprises 5'-AACAAAUUCAUU-3' (SEQ ID NO: 68), the engineered crRNA may comprise 5'-AAUGAAGGA-3'. As still yet another example, when the engineered tracrRNA comprises 5'-AACAAAUUCAUUU-3' (SEQ ID NO: 12), the engineered crRNA may comprise 5'-GAAUGAAGGA-3' (SEQ ID NO: 14).

### Example 2 of engineered dual guide RNA

In an embodiment, the engineered Cas12f1 guide RNA may comprise an engineered scaffold region and a spacer.

The spacer has a length of 10 to 50 nucleotides and has a sequence complementary to a target sequence. For example, the spacer may have a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleotides.

A sequence of the engineered scaffold region comprises in a 5' to 3' direction:
an engineered tracrRNA in which the following sequences are linked to each other:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10); 
5'-
GCUGCUUGCAUCAGCCUAAUGUCGAGAAGUGCUUUCUUCGGAAAGUAAC
CCUCGA-3' (SEQ ID NO: 11);
 and
a sequence selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 66), 5'-AACAAAUUCAU-3' (SEQ ID NO: 67), 5'-AACAAAUUCAUU-3' (SEQ ID NO: 68), 5'-AACAAAUUCAUUU-3' (SEQ ID NO: 69), 5'-AACAAAUUCAUUUU-3' (SEQ ID NO: 70), 5'-AACAAAUUCAUUUUU-3' (SEQ ID NO: 71), 5'-AACAAAUUCAUUUUUC-3' (SEQ ID NO: 72), 5'-AACAAAUUCAUUUUUCC-3' (SEQ ID NO: 73), 5'-AACAAAUUCAUUUUUCCU-3' (SEQ ID NO: 74), 5'-AACAAAUUCAUUUUUCCUC-3' (SEQ ID NO: 75), 5'-AACAAAUUCAUUUUUCCUCU-3' (SEQ ID NO: 76), 5'-AACAAAUUCAUUUUUCCUCUC-3' (SEQ ID NO: 77), 5'-AACAAAUUCAUUUUUCCUCUCC-3' (SEQ ID NO: 78), 5'-AACAAAUUCAUUUUUCCUCUCCA-3' (SEQ ID NO: 79), 5'-AACAAAUUCAUUUUUCCUCUCCAA-3' (SEQ ID NO: 80), 5'-AACAAAUUCAUUUUUCCUCUCCAAU-3' (SEQ ID NO: 81), 5'-AACAAAUUCAUUUUUCCUCUCCAAUU-3' (SEQ ID NO: 82), 5'-AACAAAUUCAUUUUUCCUCUCCAAUUC-3' (SEQ ID NO: 83), 5'-AACAAAUUCAUUUUUCCUCUCCAAUUCU-3' (SEQ ID NO: 84), 5'-AACAAAUUCAUUUUUCCUCUCCAAUUCUG-3' (SEQ ID NO: 85), 5'-AACAAAUUCAUUUUUCCUCUCCAAUUCUGC-3' (SEQ ID NO: 86), 5'-AACAAAUUCAUUUUUCCUCUCCAAUUCUGCA-3' (SEQ ID NO: 87), 5'-AAACAAAUUCAUUUUUCCUCUCCAAUUCUGCAC-3' (SEQ ID NO: 88), 5'-AACAAAUUCAUUUUUCCUCUCCAAUUCUGCACA-3' (SEQ ID NO: 89), and 5'-AACAAAUUCAUUUUUCCUCUCCAAUUCUGCACAA-3' (SEQ ID NO: 13); and
a crRNA repeat sequence portion in which the following sequences are linked to each other:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', 5'-GAAUGAAGGA-3' (SEQ ID NO: 90), 5'-CGAAUGAAGGA-3' (SEQ ID NO: 91), 5'-ACGAAUGAAGGA-3' (SEQ ID NO: 92), 5'-GACGAAUGAAGGA-3' (SEQ ID NO: 93), 5'-AGACGAAUGAAGGA-3' (SEQ ID NO: 94), 5'-UAGACGAAUGAAGGA-3' (SEQ ID NO: 95), 5'-AUAGACGAAUGAAGGA-3' (SEQ ID NO: 96), 5'-AAUAGACGAAUGAAGGA-3' (SEQ ID NO: 97), 5'-GAAUAGACGAAUGAAGGA-3' (SEQ ID NO: 98), 5'-CGAAUAGACGAAUGAAGGA-3' (SEQ ID NO: 99), 5'-CCGAAUAGACGAAUGAAGGA-3' (SEQ ID NO: 100), 5'-CCCGAAUAGACGAAUGAAGGA-3' (SEQ ID NO: 101), 5'-ACCCGAAUAGACGAAUGAAGGA-3' (SEQ ID NO: 102), 5'-AACCCGAAUAGACGAAUGAAGGA-3' (SEQ ID NO: 103), 5'-GAACCCGAAUAGACGAAUGAAGGA-3' (SEQ ID NO: 104), 5'-AGAACCCGAAUAGACGAAUGAAGGA-3' (SEQ ID NO: 105), 5'-CAGAACCCGAAUAGACGAAUGAAGGA-3' (SEQ ID NO: 106), 5'-GCAGAACCCGAAUAGACGAAUGAAGGA-3' (SEQ ID NO: 107), 5'-UGCAGAACCCGAAUAGACGAAUGAAGGA-3' (SEQ ID NO: 108), 5'-UUGCAGAACCCGAAUAGACGAAUGAAGGA-3' (SEQ ID NO: 109), and 5'-GUUGCAGAACCCGAAUAGACGAAUGAAGGA-3' (SEQ ID NO: 15), and
   5'-AUGCAAC-3',
   wherein the 3' end of the engineered crRNA repeat sequence portion is linked to the 5' end of the spacer.

Here, a sequence of the engineered tracrRNA may be different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAA-3' (SEQ ID NO: 2) and/or the engineered crRNA repeat sequence portion may be different from 5'-GUUGCAGAACCCGAAUAGACGAAUGAAGGAAUGCAAC-3' (SEQ ID NO: 4).

As an example, a sequence of the engineered tracrRNA may be the same as the sequence of SEQ ID NO: 2, and the engineered crRNA repeat sequence portion may be different from the sequence of SEQ ID NO: 4. As another example, a sequence of the engineered tracrRNA may be different from the sequence of SEQ ID NO: 2, and the engineered crRNA repeat sequence portion may be the same as the sequence of SEQ ID NO: 4. As yet another example, a sequence of the engineered tracrRNA may be different from the sequence of SEQ ID NO: 2, and the engineered crRNA repeat sequence portion may be the same as the sequence of SEQ ID NO: 4.

As an example, when the engineered tracrRNA comprises 5'-AACAAA-3', the engineered crRNA may comprise 5'-GGA-3'. As another example, when the engineered tracrRNA comprises 5'-AACAAAU-3', the engineered crRNA may comprise 5'-AGGA-3'. In yet another example, when the engineered tracrRNA comprises 5'-AACAAAUU-3', the engineered crRNA may comprise 5'-AAGGA-3'. As still yet another example, when the engineered tracrRNA comprises 5'-AACAAAUUC-3', the engineered crRNA may comprise 5'-GAAGGA-3'. As still yet another example, when the engineered tracrRNA comprises 5'-AACAAAUUCA-3' (SEQ ID NO: 66), the engineered crRNA may comprise 5'-UGAAGGA-3'. As still yet another example, when the engineered tracrRNA comprises 5'-AACAAAUUCAU-3' (SEQ ID NO: 67), the engineered crRNA may comprise 5'-AUGAAGGA-3'. As still yet another example, when the engineered tracrRNA comprises 5'-AACAAAUUCAUU-3' (SEQ ID NO: 68), the engineered crRNA may comprise 5'-AAUGAAGGA-3'. As still yet another example, when the engineered tracrRNA comprises 5'-AACAAAUUCAUUU-3' (SEQ ID NO: 69), the engineered crRNA may comprise 5'-GAAUGAAGGA-3' (SEQ ID NO: 90). As still yet another example, when the engineered tracrRNA comprises 5'-AACAAAUUCAUUUU-3' (SEQ ID NO: 70), the engineered crRNA may comprise 5'-CGAAUGAAGGA-3' (SEQ ID NO: 91). As still yet another example, when the engineered tracrRNA comprises 5'-AACAAAUUCAUUUUU-3' (SEQ ID NO: 71), the engineered crRNA may comprise 5'-ACGAAUGAAGGA-3' (SEQ ID NO: 92). As still yet another example, when the engineered tracrRNA comprises 5'-AACAAAUUCAUUUUUC-3' (SEQ ID NO: 72), the engineered crRNA may comprise 5'-GACGAAUGAAGGA-3' (SEQ ID NO: 93). As still yet another example, when the engineered tracrRNA comprises 5'-AACAAAUUCAUUUUUCC-3' (SEQ ID NO: 73), the engineered crRNA may comprise 5'-AGACGAAUGAAGGA-3' (SEQ ID NO: 94). As still yet another example, when the engineered tracrRNA comprises 5'-AACAAAUUCAUUUUUCCU-3' (SEQ ID NO: 74), the engineered crRNA may comprise 5'-UAGACGAAUGAAGGA-3' (SEQ ID NO: 95). As still yet another example, when the engineered tracrRNA comprises 5'-AACAAAUUCAUUUUUCCUC-3' (SEQ ID NO: 75), the engineered crRNA may comprise 5'-AUAGACGAAUGAAGGA-3' (SEQ ID NO: 96). As still yet another example, when the engineered tracrRNA comprises 5'-AACAAAUUCAUUUUUCCUCU-3' (SEQ ID NO: 76), the engineered crRNA may comprise 5'-AAUAGACGAAUGAAGGA-3' (SEQ ID NO: 97). As still yet another example, when the engineered tracrRNA comprises 5'-AACAAAUUCAUUUUUCCUCUC-3' (SEQ ID NO: 77), the engineered crRNA may comprise 5'-GAAUAGACGAAUGAAGGA-3' (SEQ ID NO: 98). As still yet another example, when the engineered tracrRNA comprises 5'-AACAAAUUCAUUUUUCCUCUCC-3' (SEQ ID NO: 78), the engineered crRNA may comprise 5'-CGAAUAGACGAAUGAAGGA-3' (SEQ ID NO: 99). As still yet another example, when the engineered tracrRNA comprises 5'-AACAAAUUCAUUUUUCCUCUCCA-3' (SEQ ID NO: 79), the engineered crRNA may comprise 5'-CCGAAUAGACGAAUGAAGGA-3' (SEQ ID NO: 100). As still yet another example, when the engineered tracrRNA comprises 5'-AACAAAUUCAUUUUUCCUCUCCAA-3' (SEQ ID NO: 80), the engineered crRNA may comprise 5'-CCCGAAUAGACGAAUGAAGGA-3' (SEQ ID NO: 101). As still yet another example, when the engineered tracrRNA comprises 5'-AACAAAUUCAUUUUUCCUCUCCAAU-3' (SEQ ID NO: 81), the engineered crRNA may comprise 5'-ACCCGAAUAGACGAAUGAAGGA-3' (SEQ ID NO: 102). As still yet another example, when the engineered tracrRNA comprises 5'-AACAAAUUCAUUUUUCCUCUCCAAUU-3' (SEQ ID NO: 82), the engineered crRNA may comprise 5'-AACCCGAAUAGACGAAUGAAGGA-3' (SEQ ID NO: 103). As still yet another example, when the engineered tracrRNA comprises 5'-AACAAAUUCAUUUUUCCUCUCCAAUUC-3' (SEQ ID NO: 83), the engineered crRNA may comprise 5'-GAACCCGAAUAGACGAAUGAAGGA-3' (SEQ ID NO: 104). As still yet another example, when the engineered tracrRNA comprises 5'-AACAAAUUCAUUUUUCCUCUCCAAUUCU-3' (SEQ ID NO: 84), the engineered crRNA may comprise 5'-AGAACCCGAAUAGACGAAUGAAGGA-3' (SEQ ID NO: 105). As still yet another example, when the engineered tracrRNA comprises 5'-AACAAAUUCAUUUUUCCUCUCCAAUUCUG-3' (SEQ ID NO: 85), the engineered crRNA may comprise 5'-CAGAACCCGAAUAGACGAAUGAAGGA-3' (SEQ ID NO: 106). As still yet another example, when the engineered tracrRNA comprises 5'-AACAAAUUCAUUUUUCCUCUCCAAUUCUGC-3' (SEQ ID NO: 86), the engineered crRNA may comprise 5'-GCAGAACCCGAAUAGACGAAUGAAGGA-3' (SEQ ID NO: 107). As still yet another example, when the engineered tracrRNA comprises 5'-AACAAAUUCAUUUUUCCUCUCCAAUUCUGCA-3' (SEQ ID NO: 87), the engineered crRNA may comprise 5'-UGCAGAACCCGAAUAGACGAAUGAAGGA-3' (SEQ ID NO: 108). As still yet another example, when the engineered tracrRNA comprises 5'-AAACAAAUUCAUUUUUCCUCUCCAAUUCUGCAC-3' (SEQ ID NO: 88), the engineered crRNA may comprise 5'-UUGCAGAACCCGAAUAGACGAAUGAAGGA-3' (SEQ ID NO: 109). As still yet another example, when the engineered tracrRNA comprises 5'-AACAAAUUCAUUUUUCCUCUCCAAUUCUGCACA-3' (SEQ ID NO: 89) or 5'-AACAAAUUCAUUUUUCCUCUCCAAUUCUGCACAA-3' (SEQ ID NO: 13), the engineered crRNA may comprise 5'-GUUGCAGAACCCGAAUAGACGAAUGAAGGA-3' (SEQ ID NO: 15).

### Engineered CRISPR/Cas12f1 complex

### Overview of CRISPR/Cas12f1 complex

In the present disclosure, there is provided an engineered CRISPR/Cas12f1 complex. The engineered CRISPR/Cas12f1 complex comprises a Cas12f1 protein and an engineered Cas12f1 guide RNA. Here, the engineered Cas12f1 guide RNA is as described in the section "Engineered Cas12f1 guide RNA."

In an embodiment, there is provided herein an engineered CRISPR/Cas12f1 complex capable of editing a target sequence-containing nucleic acid, the complex comprising a Cas12f1 protein and an engineered Cas12f1 guide RNA. Here, the engineered Cas12f1 guide RNA may be any one of those described in the section "Engineered Cas12f1 guide RNA."

### Cas12f1 protein - Overview

The engineered CRISPR/Cas12f1 complex provided herein comprises a Cas12f1 protein. Basically, the Cas12f1 protein may be a wildtype Cas12f1 protein occurring in nature. The sequence encoding the Cas12f1 protein may be a human codon-optimized Cas12f1 sequence for the wildtype Cas12f1 protein. In addition, the Cas12f1 protein may have the same function as the wildtype Cas12f1 protein occurring in nature. However, unless specifically limited, the term "Cas12f1 protein" as used herein may refer not only to a wildtype or codon-optimized Cas12f1 protein, but also encompass a modified Cas12f1 protein to a Cas12f1 fusion protein. In addition, the "Cas12f1 protein" may refer collectively not only to those having the same function as the wildtype Cas12f1 protein occurring in nature, but to those in which all or a part of the function is modified, those in which all or a part of the function is lost, and/or those to which an additional function is added. A meaning of the "Cas12f1 protein" may be appropriately interpreted according to the context and is interpreted in the broadest sense except for special cases. Hereinafter, a structure or function of the Cas12f1 protein will be described in detail.

### Cas12f1 protein - Wildtype Cas12f1 protein

The engineered CRISPR/Cas12f1 complex provided herein may comprise a Cas12f1 protein.

In an embodiment, the Cas12f1 protein may be a wildtype Cas12f1 protein. In an embodiment, the Cas12f1 protein may be derived from the Cas14 family (Harrington et al., Programmed DNA destruction by miniature CRISPR-Cas14 enzymes, Science 362, 839-842 (2018)). In an embodiment, the Cas12f1 protein may be a Cas14a protein derived from an uncultured archaeon (Harrington et al., Programmed DNA destruction by miniature CRISPR-Cas14 enzymes, Science 362, 839-842 (2018)). In an embodiment, the Cas12f1 protein may be a Cas14a1 protein.

### Cas12f1 protein - Modified Cas12f1 protein

The engineered CRISPR/Cas12f1 complex provided herein may comprise a modified Cas12f1 protein. The modified Cas12f1 refers to a modified form of a sequence of a wildtype or codon-optimized Cas12f1 protein, at least some of which are modified. Modification of the Cas12f1 protein may be made in its individual amino acid unit or its functional domain unit.

In an embodiment, modification of the protein may be made by individual substitution, deletion, and/or addition of one or more amino acids, peptides, polypeptides, proteins, and/or domains in the sequence of the wildtype or codon-optimized Cas12f1 protein. In an embodiment, the Cas12f1 protein may be a wildtype Cas12f1 protein whose RuvC domain includes substitution, deletion, and/or addition of one or more amino acids, peptides, and/or polypeptides.

### Cas12f1 protein - Cas12f1 fusion protein

The engineered CRISPR/Cas12f1 complex provided herein may comprise a Cas12f1 fusion protein. Here, the Cas12f1 fusion protein refers to a protein in which an additional amino acid, peptide, polypeptide, protein, and/or domain is fused to a wildtype or modified Cas12f1 protein.

In an embodiment, the Cas12f1 protein may be one in which a base editor and/or a reverse transcriptase is fused to a wildtype Cas12f1 protein. In an embodiment, the base editor may be an adenosine deaminase, and/or a cytidine deaminase. In an embodiment, the reverse transcriptase may be Moloney murine leukemia virus (M-MLV) reverse transcriptase, and/or a variant thereof. Here, the Cas12f1 protein fused with the reverse transcriptase may function as a prime editor.

In an embodiment, the Cas12f1 protein may be one in which various enzymes, which can be involved in a gene expression process in a cell, are fused to a wildtype Cas12f1 protein. Here, the Cas12f1 protein fused with the enzyme may cause various quantitative and qualitative changes in gene expression in a cell. In an embodiment, the enzyme may be VP64, DNMT, TET, KRAB, DHAC, LSD, and/or p300.

### Cas12f1 protein - Alteration of function

The Cas12f1 protein included in the engineered CRISPR/Cas12f1 complex provided herein may have the same function as a wildtype Cas12f1 protein. The Cas12f1 protein included in the engineered CRISPR/Cas12f1 complex provided herein may have an altered function as compared with a wildtype Cas12f1 protein. Specifically, the alteration may be modification of all or a part of the functions, loss of all or a part of the functions, and/or addition of an additional function. In an embodiment, the Cas12f1 protein is not particularly limited as long as such alteration can be applied to a Cas protein of a CRISPR/Cas system by those skilled in the art. Here, the alteration may be made using a known technique in the art.

In an embodiment, the Cas12f1 protein may be a Cas12f1 protein that is altered to cleave only one strand of the double strand of a target nucleic acid. Moreover, the Cas12f1 protein may be a Cas12f1 protein altered so that it is able to cleave only one strand of the double strand of a target nucleic acid, and to perform base editing or prime editing on the uncleaved strand. In an embodiment, the Cas12f1 protein may be a Cas12f1 protein altered so that it is unable to cleave neither strand of the double strand of a target nucleic acid. Furthermore, the Cas12f1 protein may be a Cas12f1 protein altered so that it is unable to cleave neither strand of the double strand of a target nucleic acid, and is able to perform base editing, prime editing, or a function of regulating gene expression on the target nucleic acid.

### Cas12f1 protein - Examples of other modifications

In an embodiment, the Cas12f1 protein may comprise a nuclear localization sequence (NLS) or a nuclear export sequence (NES). Specifically, the NLS may be any one of those exemplified in the section for NLS among "Definitions of terms," but is not limited thereto. In an embodiment, the Cas12f1 protein may comprise a tag. Specifically, the tag may be any one of those exemplified in the section for tag in "Definition of terms," but is not limited thereto.

### Cas12f1 protein - PAM sequence

Two conditions are required for a CRISPR/Cas12f1 complex to cleave a target gene or target nucleic acid.

First, there has to be a nucleotide sequence of a certain length, which can be recognized by a Cas12f1 protein, in the target gene or target nucleic acid. Here, the nucleotide sequence of a certain length recognized by the Cas12f1 protein is referred to as a protospacer adjacent motif (PAM) sequence. The PAM sequence is a distinctive sequence determined according to the Cas12f1 protein. Second, there has to be a sequence, which is capable of complementarily binding to a spacer sequence included in the guide RNA, around the PAM sequence of a certain length.

When these two conditions are satisfied, that is, 1) the Cas12f1 protein recognizes the PAM sequence of a certain length, and 2) the spacer sequence portion complementarily binds to a sequence around the PAM sequence, the Cas12f1 protein/guide RNA complex (CRISPR/Cas12f1 complex) cleaves a target gene or target nucleic acid. Therefore, when determining a target sequence of the CRISPR/Cas12f1 complex, there is a constraint that the target sequence has to be determined within sequences adjacent to the PAM sequence.

### Cas12f1 protein - Examples of PAM sequence

In an embodiment, a PAM sequence of the Cas12f1 protein may be a T-rich sequence. In an embodiment, a PAM sequence of the Cas12f1 protein may be THTN, in a 5' to 3' direction. Here, N may be one of deoxythymidine (T), deoxyadenosine (A), deoxycytidine (C), or deoxyguanosine (G), and H may be one of deoxythymidine (T), deoxyadenosine (A), and deoxycytidine (C). In an embodiment, a PAM sequence of the Cas12f1 protein may be TTTN in a 5' to 3' direction. Here, N is one of deoxythymidine (T), deoxyadenosine (A), deoxycytidine (C), or deoxyguanosine (G). In an embodiment, a PAM sequence of the Cas12f1 protein may be TTTA, TTTT, TTTC, or TTTG in a 5' to 3' direction. In an embodiment, a PAM sequence of the Cas12f1 protein may be TATA, TATT, TATC, or TATG in a 5' to 3' direction. In an embodiment, a PAM sequence of the Cas12f1 protein may be TCTA, TCTT, TCTC, or TCTG in a 5' to 3' direction. In an embodiment, a PAM sequence of the Cas12f1 protein may be TTTA or TTTG in a 5' to 3' direction. In an embodiment, a PAM sequence of the Cas12f1 protein may be different from a PAM sequence of the wildtype Cas12f1 protein.

### Cas12f1 protein - Examples of sequence

In an embodiment, the Cas12f1 protein may have an amino acid sequence selected from the group consisting of SEQ ID NOS: 259 to 266.

In an embodiment, a DNA sequence encoding the Cas12f1 protein may be a human codon-optimized sequence.

In an embodiment, a DNA sequence encoding the Cas12f1 protein may be a DNA sequence selected from the group consisting of SEQ ID NOS: 267 to 276.

### Engineered Cas12f1 guide RNA

The engineered Cas12f1 guide RNA constituting the CRISPR/Cas12f1 complex provided herein has the same characteristics and structure as described in the section "Engineered Cas12f1 guide RNA."

### CRISPR/Cas12f1 complex - Examples of structure

In an embodiment, the Cas12f1 protein may have an amino acid sequence selected from SEQ ID NOS: 259 to 262, and the engineered Cas12f1 guide RNA may have a sequence selected from the group consisting of SEQ ID NOS: 210 to 258, SEQ ID NOS: 381 to 393, SEQ ID NOS: 396 to 407, and SEQ ID NOS: 409 to 421. Here, the Cas12f1 protein and the engineered Cas12f1 guide RNA may be combined to form a CRISPR/Cas12f1 complex.

In an embodiment, the Cas12f1 protein may have an amino acid sequence selected from the group consisting of SEQ ID NOS: 263 to 266, and the engineered Cas12f1 guide RNA may have a sequence selected from SEQ ID NOS: 210 to 258, SEQ ID NOS: 381 to 393, SEQ ID NOS: 396 to 407, and SEQ ID NOS: 409 to 421. Here, the CRISPR/Cas12f1 complex formed by combination of the Cas12f1 protein with the engineered Cas12f1 guide RNA may have a base editing function.

### Vector for expressing respective components of CRISPR/Cas12f1 system

### Overview of vector

In the present disclosure, there is provided a vector for expressing components of a CRISPR/Cas12f1 system. The vector is constructed to express a Cas12f1 protein, and/or an engineered Cas12f1 guide RNA. A sequence of the vector may comprise a nucleic acid sequence encoding one of the components of the CRISPR/Cas12f1 system or may comprise a nucleic acid sequence encoding two or more of the components thereof. A sequence of the vector comprises a nucleic acid sequence encoding the Cas12f1 protein and/or a nucleic acid sequence encoding the engineered Cas12f1 guide RNA. A sequence of the vector comprises one or more promoter sequences. The promoter is operatively linked with a nucleic acid sequence encoding the Cas12f1 protein and/or a nucleic acid sequence encoding the engineered Cas12f1 guide RNA, so that transcription of the nucleic acid sequence(s) in a cell can be promoted. The Cas12f1 protein has the same characteristics and structure as the Cas12f1 protein, the modified Cas12f1 protein, and/or the Cas12f1 fusion protein as described in the section "Engineered CRISPR/Cas12f1 complex." The engineered Cas12f1 guide RNA has the same characteristics and composition as the engineered Cas12f1 guide RNA as described in the section "Engineered Cas12f1 guide RNA."

A sequence of the vector may comprise a nucleic acid sequence encoding the Cas12f1 protein and/or a nucleic acid sequence encoding the engineered Cas12f1 guide RNA. In an embodiment, a sequence of the vector may comprise a first sequence comprising a nucleic acid sequence encoding the Cas12f1 protein, and a second sequence comprising a nucleic acid sequence encoding the engineered Cas12f1 guide RNA. The sequence of the vector comprises a promoter sequence for expressing a nucleic acid sequence encoding the Cas12f1 protein in a cell, and a promoter sequence for expressing a nucleic acid sequence encoding the engineered Cas12f1 guide RNA in a cell, wherein each of the promoters is operably linked to each target to be expressed. In an embodiment, a sequence of the vector may comprise a first promoter sequence operably linked to the first sequence, and a second promoter sequence operably linked to the second sequence.

A sequence of the vector may comprise a nucleic acid sequence encoding the Cas12f1 protein and/or a nucleic acid sequence encoding two or more engineered Cas12f1 guide RNAs that are different from each other. In an embodiment, a sequence of the vector may comprise a first sequence comprising a nucleic acid sequence encoding the Cas12f1 protein, a second sequence comprising a nucleic acid sequence encoding a first engineered Cas12f1 guide RNA, and a third sequence comprising a nucleic acid sequence encoding a second engineered Cas12f1 guide RNA. Furthermore, the sequence of the vector may comprise a first promoter sequence operably linked to the first sequence, a second promoter sequence operably linked to the second sequence, and a third promoter sequence operably linked to the third sequence.

In the vector, the nucleic acid sequence encoding each component may be a DNA sequence.

### Target to be expressed - Cas12f1 protein

The vector may be constructed to express a Cas12f1 protein. Here, the Cas12f1 protein may have the same structure and characteristics as each of those described in the section "Engineered CRISPR/Cas12f1 complex."

In an embodiment, the vector may be constructed to express a wildtype Cas12f1 protein. Here, the wildtype Cas12f1 protein may be Cas14a1. In an embodiment, the vector may be constructed to express a Cas12f1 protein altered so that it cleaves only one strand of the double strand of a target nucleic acid. Furthermore, the modified Cas12f1 protein may be an altered Cas12f1 protein so that it is able to cleave only one strand of the double strand of a target nucleic acid and is able to perform base editing or prime editing on the uncleaved strand. In an embodiment, the Cas12f1 protein may be altered so that it is unable to cleave neither strand of the double strand of a target nucleic acid. Furthermore, the Cas12f1 protein may be altered so that it is unable to cleave neither strand of the double strand of a target nucleic acid and is able to perform base editing, prime editing, or a function of regulating gene expression on a target nucleic acid.

### Target to be expressed - Engineered Cas12f1 guide RNA

The vector may be constructed to express an engineered Cas12f1 guide RNA. The engineered Cas12f1 guide RNA may have the same characteristics and composition as the engineered Cas12f1 guide RNA as described in the section "Engineered Cas12f1 guide RNA." The vector may be constructed to express two or more engineered Cas12f1 guide RNAs that are different from each other.

### Target to be expressed - Additional component

The vector may be constructed to express an additional component such as an NLS and a tag protein in addition to the above-described targets to be expressed. In an embodiment, the additional component may be expressed independently of the Cas12f1 protein, the modified Cas12f1 protein, and/or the engineered Cas12f1 guide RNA. In another embodiment, the additional component may be expressed in conjunction with the Cas12f1 protein, the modified Cas12f1 protein, and/or the engineered Cas12f1 guide RNA. Here, the additional component may be a component that is generally expressed when it is intended to express a CRISPR/Cas system. In this regard, reference may be made to the prior art. The additional component may be one or more of the components as described in the section "Background art - Design of vector expressing CRISPR/Cas system ."

### Component of vector - Sequence for expressing Cas12f1 protein

A sequence of the vector may comprise a nucleic acid sequence encoding the Cas12f1 protein. Here, the Cas12f1 protein may have the same structure and characteristics as each of those described in the section "Engineered CRISPR/Cas12f1 complex."

In an embodiment, a sequence of the vector may comprise a sequence encoding a wildtype Cas12f1 protein. Here, the wildtype Cas12f1 protein may be Cas14a1. In an embodiment, a sequence of the vector may comprise a human codon-optimized nucleic acid sequence encoding a Cas12f1 protein. Here, the human codon-optimized nucleic acid sequence encoding a Cas12f1 protein may be a human codon-optimized nucleic acid sequence encoding a Cas14a1 protein. In an embodiment, a sequence of the vector may comprise a sequence encoding a modified Cas12f1 protein or a Cas12f1 fusion protein. In an embodiment, a sequence of the vector may comprise a sequence encoding a Cas12f1 fusion protein altered so that it is able to cleave only one strand of the double strand of a target nucleic acid, and is able to perform base editing or prime editing on the uncleaved strand. In an embodiment, a sequence of the vector may comprise a sequence encoding a Cas12f1 fusion protein altered so that it is unable to cleave neither strand of the double strand of a target nucleic acid, and is able to perform base editing, prime editing, or a function of regulating gene expression on the uncleaved strand.

### Component of vector - Sequence for expressing engineered Cas12f1 guide RNA

In an embodiment, a sequence of the vector may comprise a sequence encoding an engineered Cas12f1 guide RNA. For example, a sequence of the vector may comprise a sequence selected from the group consisting of SEQ ID NOS: 210 to 258, SEQ ID NOS: 381 to 393, SEQ ID NOS: 395 to 407, SEQ ID NOS: 409 to 421, and SEQ ID NOS: 436 to 439.

In an embodiment, a sequence of the vector may comprise a sequence encoding two or more engineered Cas12f1 guide RNAs that are different from each other. For example, a sequence of the vector may comprise a sequence encoding a first engineered Cas12f1 guide RNA and a sequence encoding a second engineered Cas12f1 guide RNA, each of which is selected from the group consisting of SEQ ID NOS: 210 to 258, SEQ ID NOS: 381 to 393, SEQ ID NOS: 395 to 407, SEQ ID NOS: 409 to 421, and SEQ ID NOS: 436 to 439.

### Component of vector - Promoter sequence

A sequence of the vector may comprise a promoter sequence operably linked to a sequence encoding each component. Specifically, the promoter sequence may be one of the promoters disclosed in the promoter part of the section "Background art - Design of vector expressing CRISPR/Cas system ", but is not limited thereto.

In an embodiment, a sequence of the vector may comprise a sequence encoding a Cas12f1 protein and a promoter sequence. Here, the promoter sequence is operably linked to the sequence encoding a Cas12f1 protein. In an embodiment, a sequence of the vector may comprise a sequence encoding an engineered Cas12f1 guide RNA and a promoter sequence. Here, the promoter sequence may be operably linked to the sequence encoding an engineered Cas12f1 guide RNA. In an embodiment, the vector sequence may comprise a sequence encoding a Cas12f1 protein, a sequence encoding an engineered Cas12f1 guide RNA, and a promoter sequence. Here, the promoter sequence is operatively linked to the sequence encoding a Cas12f1 protein and the sequence encoding an engineered Cas12f1 guide RNA sequence, wherein a transcription factor activated by the promoter sequence causes expression of the Cas12f1 protein and the engineered Cas12f1 guide RNA.

### Component of vector - Possible to comprise two or more promoter sequences

In an embodiment, a sequence of the vector may comprise a first sequence encoding a Cas12f1 protein, a first promoter sequence, a second sequence encoding an engineered Cas12f1 guide RNA, and a second promoter sequence. Here, the first promoter sequence is operably linked to the first sequence, the second promoter sequence is operatively linked to the second sequence, transcription of the first sequence is induced by the first promoter sequence, wherein transcription of the second sequence is induced by the second promoter sequence. Here, the first promoter and the second promoter may be the same type of promoters. Here, the first promoter and the second promoter may be different kinds of promoters.

In an embodiment, a sequence of the vector may comprise a first sequence encoding a Cas12f1 protein, a first promoter sequence, a second sequence encoding a first engineered Cas12f1 guide RNA, a second promoter sequence, a third sequence encoding a second engineered Cas12f1 guide RNA, and a third promoter sequence. Here, the first promoter sequence is operatively linked to the first sequence, the second promoter sequence is operably linked to the second sequence, and the third promoter sequence is operably linked to the third sequence, wherein transcription of the first sequence is induced by the first promoter sequence, transcription of the second sequence is induced by the second promoter sequence, and transcription of the third sequence is induced by the third promoter sequence. Here, the second promoter and the third promoter may be the same type of promoters. Specifically, the second promoter sequence and the third promoter sequence may be a U6 promoter sequence, but are not limited thereto. Here, the second promoter and the third promoter may be different types of promoters. Specifically, the second promoter may be a U6 promoter sequence, and the third promoter may be an H1 promoter sequence, but these promoters are not limited thereto.

### Component of vector - Termination signal

The vector may comprise a termination signal operably linked to the promoter sequence. Here, the termination signal may be one of the termination signals disclosed in the termination signal portion of the section "Background art - Design of vector expressing CRISPR/Cas system ", but is not limited thereto. The termination signal may vary depending on the type of promoter sequence.

In an embodiment, when a sequence of the vector comprises a U6 promoter sequence, a thymidine repeat sequence operably linked to the U6 promoter sequence may serve as a termination signal. In an embodiment, the thymidine repeat sequence may be a sequence in which five or more thymidines are continuously linked. In an embodiment, when a sequence of the vector comprises an H1 promoter sequence, a thymidine repeat sequence operably linked to the H1 promoter sequence may serve as a termination signal. In an embodiment, the thymidine repeat sequence may be a sequence in which five or more thymidines are continuously linked.

### Component of vector - Other components

A sequence of the vector may comprise a component necessary according to the purpose in addition to the above components.

In an embodiment, a sequence of the vector may comprise a sequence of a regulatory/control component, and/or a sequence of an additional component. In an embodiment, the additional element may be added for the purpose of distinguishing transfected cells from non-transfected cells. Here, each of the sequence of the regulatory/control element and the additional component may be one of those disclosed in the section "Background art - Design of vector expressing CRISPR/Cas system ", but is not limited thereto.

### Type of vector - Viral vector

The vector may be a viral vector.

In an embodiment, the viral vector may be at least one selected from the group consisting of a retrovirus, a lentivirus, an adenovirus, an adeno-associated virus, a vaccinia virus, a poxvirus, and a herpes simplex virus. In an embodiment, the viral vector may be an adeno-associated virus.

### Type of vector - Non-viral vector

The vector may be a non-viral vector. In an embodiment, the non-viral vector may be one or more selected from the group consisting of a plasmid, a phage, naked DNA, a DNA complex, and mRNA. In an embodiment, the plasmid may be selected from the group consisting of pcDNA series, pSC101, pG1796, pACYC177, ColE1, pKT230, pME290, pBR322, pUC8/9, pUC6, pBD9, pHC79, pIJ61, pLAFR1, pHV14, pGEX series, pET series, and pUC19. In an embodiment, the phage may be selected from the group consisting of λgt4λB, λ-Charon, λΔz1, and M13. In an embodiment, the vector may be a PCR amplicon.

### Form of vector - Circular or linear vector

The vector may have a circular or linear form. When the vector is a linear vector, RNA transcription is terminated at the 3' end thereof even if a sequence of the linear vector does not separately comprise a termination signal. In contrast, when the vector is a circular vector, RNA transcription is not terminated unless the circular vector sequence separately comprises a termination signal. Therefore, when the vector is used in a form of a circular vector, a termination signal corresponding to a transcription factor related to each promoter sequence has to be included in order for the vector to express an intended target.

In an embodiment, the vector may be a linear vector. In an embodiment, the vector may be a linear amplicon. In an embodiment, the vector may be a linear vector comprising a sequence selected from the group consisting of SEQ ID NOS: 267 to 276 and SEQ ID NOS: 210 to 258, SEQ ID NOS: 381 to 393, SEQ ID NOS: 396 to 407, and SEQ ID NOS: 409 to 421. In an embodiment, the vector may be a circular vector. In an embodiment, the vector may be a circular vector comprising a sequence selected from the group consisting of SEQ ID NOS: 267 to 276 and SEQ ID NOS: 210 to 258, SEQ ID NOS: 381 to 393, SEQ ID NOS: 396 to 407, and SEQ ID NOS: 409 to 421.

### Vector - Example of sequence

In an embodiment, a sequence of the vector may comprise a sequence selected from the group consisting of SEQ ID NOS: 267 to 276, SEQ ID NOS: 210 to 258, SEQ ID NOS: 381 to 393, SEQ ID NOS: 396 to 407, and SEQ ID NOS: 409 to 421.

### Chemical modification of nucleic acid

In the present disclosure, there is provided a component that comprises or consists of a nucleic acid such as an engineered crRNA or a nucleic acid encoding the same, an engineered Cas12f1 guide RNA or a nucleic acid encoding the same, and/or a vector for expressing components of a CRISPR/Cas12f1 system. Here, the "nucleic acid" in the component may be naturally occurring DNA or RNA, or a modified nucleic acid in which a part of or all of a constituent nucleic acid is chemically modified. In an embodiment, the constituent nucleic acid may be naturally occurring DNA and/or RNA. In an embodiment, the constituent nucleic acid may be one in which one or more nucleotides are chemically modified. Here, the chemical modification includes all modifications of a nucleic acid known to those skilled in the art. Specifically, the chemical modification may include all modifications of a nucleic acid as described in WO 2019/089820 A1, but is not limited thereto.

### Gene editing method using engineered Cas12f1 guide RNA

### Overview of gene editing method

As disclosed herein, there is provided a method of editing a target gene or target nucleic acid in a target cell by using an engineered crRNA. The target gene or target nucleic acid contains a target sequence. The target nucleic acid may be single-stranded DNA, double-stranded DNA, and/or RNA. The gene editing method comprises delivering an engineered Cas12f1 guide RNA and a Cas12f1 protein, or nucleic acids, each of which encodes each of them, into a target cell including a target gene or target nucleic acid. As a result, an engineered CRISPR/Cas12f1 complex is introduced into the target cell, or formation of an engineered CRISPR/Cas12f1 complex is induced, so that the target gene is edited by the engineered CRISPR/Cas12f1 complex. The engineered Cas12f1 guide RNA has the same characteristics and structure as described in the section "Engineered Cas12f1 guide RNA." The Cas12f1 protein has the same characteristics and structure as the Cas12f1 protein and/or the modified Cas12f1 protein as described in the section "Engineered CRISPR/Cas12f1 complex."

In an embodiment, the gene editing method may comprise delivering a Cas12f1 protein or a nucleic acid encoding the same, and an engineered Cas12f1 guide RNA or a nucleic acid encoding the same into a target cell.

Here, the engineered Cas12f1 guide RNA may comprise an engineered scaffold region and a spacer.

Here, the engineered scaffold region has the same characteristics and structure as each of those described in any one of the above-described "Engineered scaffold region" sections. As an example, the engineered scaffold region may be represented by a sequence selected from the group consisting of SEQ ID NOS: 168 to 187. As another example, the engineered scaffold region may be represented by a sequence selected from the group consisting of ID NOS: 187 to 198. In another example, the engineered scaffold region may be represented by a sequence selected from the group consisting of SEQ ID NOS: 199 to 205. As yet another example, the engineered scaffold region may be represented by a sequence selected from the group consisting of SEQ ID NOS: 206 to 209.

Here, the spacer sequence may complementarily bind to a target gene or target nucleic acid included in the target cell.

### Target cell

In an embodiment, the target cell may be a prokaryotic cell. In an embodiment, the target cell may be a eukaryotic cell. Specifically, the eukaryotic cell may be, but is not limited to, a plant cell, an animal cell, and/or a human cell.

### Determination of target sequence

A target gene or target nucleic acid and a target sequence to be edited by a CRISPR/Cas12f1 complex may be determined in consideration of the purpose of gene editing, environment of a target cell, a PAM sequence recognized by a Cas12f1 protein, and/or other variables. Here, a method of determining the target sequence is not particularly limited as long as it is capable of determining a target sequence of an appropriate length, and a technique known in the art may be used therefor.

### Determination of spacer sequence according to target sequence

Once the target sequence is determined, a spacer sequence corresponding thereto is designed. The spacer sequence is designed as a sequence capable of complementarily binding to the target sequence. In an embodiment, the spacer sequence may be designed as a sequence capable of complementarily binding to the target gene. In an embodiment, the spacer sequence may be designed to be capable of complementarily binding to the target nucleic acid. In an embodiment, the spacer sequence may be designed as a sequence complementary to a target sequence included in a target strand sequence of the target nucleic acid. In an embodiment, the spacer sequence is designed as an RNA sequence corresponding to a DNA sequence of a protospacer included in a non-target strand sequence of the target nucleic acid. Specifically, the spacer sequence is designed to have the same nucleotide sequence as the protospacer sequence, except that every thymidine included in the nucleotide sequence is substituted by a uridine.

In an embodiment, the spacer has a length of 10 to 50 nucleotides. For example, the spacer has a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides. Preferably, the spacer has a length of 17, 18, 19, 20, 21, 22, 23, 24 or 25 nucleotides.

### Complementarity between target sequence and spacer sequence

In an embodiment, the spacer sequence may be complementary to the target sequence by 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. In an embodiment, the spacer sequence may be a sequence complementary to the target sequence within a range between numbers selected from the immediately preceding sentence. As an example, the spacer sequence may be a sequence that is 60% to 90% complementary to the target sequence. As another example, the spacer sequence may be a sequence that is 90% to 100% complementary to the target sequence.

### Number of mismatches between target sequence and spacer sequence

In an embodiment, the spacer sequence may be a sequence that is complementary to the target sequence and has 0, 1, 2, 3, 4, 5, 6, 7, 8, 9, or 10 mismatches therewith. In an embodiment, the spacer sequence may have mismatches within a numerical range selected from the immediately preceding sentence. As an example, the spacer sequence may have 0, 1, 2, 3, 4, or 5 mismatches with the target sequence. As another example, the spacer sequence may have 6 to 10 mismatches with the target sequence.

### Use of CRISPR/Cas12f1 complex

The gene editing method provided herein utilizes the fact that the engineered CRISPR/Cas12f1 complex has activity of cleaving a gene or nucleic acid in a target-specific manner. The engineered CRISPR/Cas12f1 complex has the same characteristics and composition as the engineered CRISPR/Cas12f1 complex as described in the section "Engineered CRISPR/Cas12f1 complex."

### Delivery of respective components of CRISPR/Cas12f1 complex into cell

The gene editing method provided herein comprises bringing an engineered CRISPR/Cas12f1 complex in contact with a target gene or target nucleic acid in a target cell. Accordingly, in order to induce the engineered CRISPR/Cas12f1 complex to come in contact with the target gene or target nucleic acid, the gene editing method comprises delivering respective components of the engineered CRISPR/Cas12f1 complex into a target cell. In an embodiment, the gene editing method may comprise delivering into a target cell an engineered Cas12f1 guide RNA or a nucleic acid encoding the same and a Cas12f1 protein or a nucleic acid encoding the same. In an embodiment, the gene editing method may comprise delivering an engineered Cas12f1 guide RNA and a Cas12f1 protein into a target cell. In an embodiment, the gene editing method may comprise delivering a nucleic acid encoding an engineered Cas12f1 guide RNA and a Cas12f1 protein into a target cell. In an embodiment, the gene editing method may comprise delivering an engineered Cas12f1 guide RNA and a nucleic acid encoding a Cas12f1 protein into a target cell. In an embodiment, the gene editing method may comprise delivering a nucleic acid encoding an engineered Cas12f1 guide RNA and a nucleic acid encoding a Cas12f1 protein into a target cell. The engineered Cas12f1 guide RNA or the nucleic acid encoding the same, and the Cas12f1 protein or the nucleic acid encoding the same may be delivered into the target cell in various forms of delivery using various delivery methods.

### Form of delivery - RNP

As the form of delivery, a ribonucleoprotein particle (RNP), in which an engineered Cas12f1 guide RNA and a Cas12f1 protein are bound to each other, may be used. In an embodiment, the gene editing method may comprise introducing, into a target cell, a CRISPR/Cas12f1 complex in which the engineered Cas12f1 guide RNA and the Cas12f1 protein are bound to each other.

### Form of delivery - Non-viral vector

As another form of delivery, a non-viral vector comprising a nucleic acid sequence encoding an engineered Cas12f1 guide RNA and a nucleic acid sequence encoding a Cas12f1 protein may be used. In an embodiment, the gene editing method may comprise introducing, into a target cell, a non-viral vector comprising a nucleic acid sequence encoding an engineered Cas12f1 guide RNA and a nucleic acid sequence encoding a Cas12f1 protein. Specifically, the non-viral vector may be a plasmid, naked DNA, a DNA complex, or mRNA, but is not limited thereto. In another embodiment, the gene editing method comprises introducing into a target cell, a first non-viral vector comprising a nucleic acid sequence encoding an engineered Cas12f1 guide RNA, and a second non-viral vector comprising a nucleic acid sequence encoding a Cas12f1 protein. Specifically, each of the first non-viral vector and the second non-viral vector may be one selected from the group consisting of a plasmid, naked DNA, a DNA complex, and mRNA, but is not limited thereto.

### Form of delivery - Viral vector

As another form of delivery, a viral vector comprising a nucleic acid sequence encoding an engineered Cas12f1 guide RNA and a nucleic acid sequence encoding a Cas12f1 protein may be used. In an embodiment, the gene editing method may comprise introducing, into a target cell, a viral vector comprising a nucleic acid sequence encoding an engineered Cas12f1 guide RNA and a nucleic acid sequence encoding a Cas12f1 protein. Specifically, the viral vector may be one selected from a retrovirus, a lentivirus, an adenovirus, an adeno-associated virus, a vaccinia virus, a poxvirus, and a herpes simplex virus, but is not limited thereto. In an embodiment, the viral vector may be an adeno-associated virus.

In another embodiment, the gene editing method may comprise introducing into a target cell, a first viral vector comprising a nucleic acid sequence encoding an engineered Cas12f1 guide RNA, and a second viral vector comprising a nucleic acid sequence encoding a Cas12f1 protein. Specifically, each of the first viral vector and the second viral vector may be one selected from a retrovirus, a lentivirus, an adenovirus, an adeno-associated virus, a vaccinia virus, a poxvirus, and a herpes simplex virus, but is not limited thereto.

### Delivery method - Common means of delivery

The delivery method is not particularly limited as long as it is capable of delivering, into a cell, an engineered Cas12f1 guide RNA or a nucleic acid encoding the same, and a Cas12f1 protein or a nucleic acid encoding the same in an appropriate form of delivery. In an embodiment, the delivery method may be electroporation, gene gun, sonoporation, magnetofection, and/or transient cell compression or squeezing.

### Delivery method - Nanoparticles

The delivery method may be delivering at least one component, which is included in the CRISPR/Cas12f1 system, using nanoparticles. Here, the delivery method may be a method known in the art which can be appropriately selected by those skilled in the art. For example, the nanoparticle delivery method may be a method disclosed in WO 2019/089820 A1, but is not limited thereto.

In an embodiment, the delivery method may be delivering, using nanoparticles, a Cas12f1 protein or a nucleic acid encoding the same and/or an engineered Cas12f1 guide RNA or a nucleic acid encoding the same. In an embodiment, the delivery method may be delivering, using nanoparticles, a Cas12f1 protein or a nucleic acid encoding the same, a first engineered Cas12f1 guide RNA or a nucleic acid encoding the same, and/or a second engineered Cas12f1 guide RNA or a nucleic acid encoding the same. Here, the delivery method may be a cationic liposome method, a lithium acetate-dimethyl sulfoxide (DMSO) method, lipid-mediated transfection, calcium phosphate precipitation, lipofection, polyethyleneimine (PEI)-mediated transfection, diethylaminoethyl (DEAE)-dextran-mediated transfection, and/or nanoparticle-mediated nucleic acid delivery (see Panyam et., al Adv Drug Deliv Rev. 2012 Sep 13. pii: S0169-409X(12)00283-9. doi: 10.1016/j.addr.2012.09.023), but is not limited thereto. Here, the component of the CRISPR/Cas12f1 system may be in a form of an RNP, a non-viral vector, and/or a viral vector. For example, each of the components of the CRISPR/Cas12f1 system may be in a form of mRNA encoding the same, but is not limited thereto.

### Form and method of delivery - Combination being possible

The gene editing method comprises delivering, into a target cell, an engineered Cas12f1 guide RNA or a nucleic acid encoding the same, and a Cas12f1 protein or a nucleic acid encoding the same into a cell, wherein delivery forms and/or delivery methods of respective components may be the same or different from each other. In an embodiment, the gene editing method may comprise delivering an engineered Cas12f1 guide RNA or a nucleic acid encoding the same in a first form of delivery, and delivering a Cas12f1 protein or a nucleic acid encoding the same in a second form of delivery. Here, each of the first form of delivery, and the second form of delivery may be any one of the above-described forms of delivery. In an embodiment, the gene editing method may comprise delivering an engineered Cas12f1 guide RNA or a nucleic acid encoding the same in a first form of delivery, and delivering a Cas12f1 protein or a nucleic acid encoding the same in a second form of delivery. Here, each of the first form of delivery, and the second form of delivery may be any one of the above-described forms of delivery.

### Order of delivery

The gene editing method comprises delivering, into a cell, an engineered Cas12f1 guide RNA or a nucleic acid encoding the same, and a Cas12f1 protein or a nucleic acid encoding the same into a cell, wherein the components may be delivered into the cell simultaneously or sequentially with a time interval.

In an embodiment, the gene editing method may comprise delivering, into a target cell, an engineered Cas12f1 guide RNA or a nucleic acid encoding the same and a Cas12f1 protein or a nucleic acid encoding the same simultaneously. In an embodiment, the gene editing method may comprise delivering an engineered Cas12f1 guide RNA or a nucleic acid encoding the same into a cell, and then delivering a Cas12f1 protein or a nucleic acid encoding the same into the cell. In an embodiment, the gene editing method may comprise delivering a Cas12f1 protein or a nucleic acid encoding the same into a cell, and then delivering an engineered Cas12f1 guide RNA into the cell. In an embodiment, the gene editing method may comprise delivering a nucleic acid encoding a Cas12f1 protein into a cell, and then delivering an engineered Cas12f1 guide RNA into the cell.

### Delivery of plurality of engineered Cas12f1 guide RNAs or nucleic acids encoding same

The gene editing method provided herein may comprise delivering, into a target cell, a Cas12f1 protein or a nucleic acid encoding the same, and two or more engineered Cas12f1 guide RNAs or nucleic acids encoding the same. By using the above method, two or more CRISPR/Cas12f1 complexes, which target different sequences, may be introduced into a target cell or formed in a target cell. As a result, the method enables editing of two or more different target genes or target nucleic acids included in the cell. In an embodiment, the gene editing method comprises delivering, into a target cell including a target gene or target nucleic acid, a Cas12f1 protein or a nucleic acid encoding the same, a first engineered Cas12f1 guide RNA or a nucleic acid encoding the same, and a second engineered Cas12f1 guide RNA or a nucleic acid encoding the same. Here, each of the components may be delivered into the cell using one or more of the above-described forms of delivery and methods of delivery. Here, two or more of the components may be delivered simultaneously or sequentially into the cell.

### Bringing CRISPR/Cas12f1 complex in contact with target nucleic acid

In the gene editing method provided herein, editing of a target gene or target nucleic acid in a target cell is performed as an engineered CRISPR/Cas12f1 complex comes in contact with the target gene or target nucleic acid. Accordingly, the gene editing method may comprise bringing the engineered CRISPR/Cas12f1 complex in contact with the target gene or target nucleic acid in the target cell or inducing the engineered CRISPR/Cas12f1 complex to come in contact therewith. In an embodiment, the gene editing method may comprise bringing an engineered CRISPR/Cas12f1 complex in contact with a target nucleic acid in a target cell. In an embodiment, the gene editing method may comprise inducing an engineered CRISPR/Cas12f1 complex to come in contact with a target nucleic acid in a target cell. Here, the induction method is not particularly limited as long as it allows the engineered CRISPR/Cas12f1 complex to come in contact with the target nucleic acid in the cell. In an embodiment, the induction may be achieved by delivering, into a cell, an engineered Cas12f1 guide RNA or a nucleic acid encoding the same, and a Cas12f1 protein or a nucleic acid encoding the same.

### Results of gene editing - Indel

As a result of performing the gene editing method provided herein, indel may occur in a target gene or target nucleic acid. Here, the indel may occur inside and/or outside of a target sequence portion and/or a protospacer sequence portion. The indel refers to a mutation caused by deletion of some nucleotides in the nucleotide sequence of the nucleic acid, insertion of any nucleotide thereinto, and/or both of the deletion and the insertion, before the gene editing. In general, when an indel occurs in a target gene or target nucleic acid sequence, the gene or nucleic acid is inactivated. In an embodiment, as a result of performing the gene editing method, deletion and/or addition of one or more nucleotides may occur in the target gene or target nucleic acid.

### Results of gene editing - Base editing

As a result of performing the gene editing method provided herein, base editing may occur in a target gene or target nucleic acid. Base editing means intentionally altering one or more specific nucleotides in a nucleic acid, unlike the indel caused by deletion or addition of any nucleotide in a target gene or target nucleic acid. In other words, the base editing causes an intended point mutation at a specific location in the target gene or target nucleic acid. In an embodiment, as a result of performing the gene editing method, substitution of one or more nucleotides by other nucleotides may occur in the target gene or target nucleic acid.

### Results of gene editing - Insertion

As a result of performing the gene editing method provided herein, a knock-in may occur in a target gene or target nucleic acid. The knock-in means inserting an additional nucleic acid sequence into a target gene or target nucleic acid sequence. To cause the knock-in, a donor including the additional nucleic acid sequence is further required in addition to the CRISPR/Cas12f1 complex. When the CRISPR/Cas12f1 complex cleaves a target gene or target nucleic acid in a cell, repair of the cleaved target gene or target nucleic acid occurs. Here, the donor participates in the repair process so that the additional nucleic acid sequence can be inserted into the target gene or target nucleic acid. In an embodiment, the gene editing method may further comprise introducing a donor into a target cell. For example, the donor comprises an exogeneous DNA sequence to be inserted into an intracellular genome and induces the exogeneous DNA sequence to be inserted into the target gene or target nucleic acid. Here, when delivering the donor into the target cell, the above-described forms of delivery and/or delivery methods may be used.

### Results of gene editing - Deletion

As a result of performing the gene editing method provided herein, all or a part of a target gene or target nucleic acid sequence may be deleted. The deletion means removing a part of the nucleotide sequence over a certain length or longer in the target gene or target nucleic acid. The deletion refers to an effect capable of completely removing a specific region of a gene, for example, a first exon region, as compared with an effect of the above-described indel.

In an embodiment, the gene editing method comprises introducing, into a target cell including a target gene or target nucleic acid, a Cas12f1 protein or a nucleic acid encoding the same, a first engineered Cas12f1 guide RNA or a nucleic acid encoding the same, and a second engineered Cas12f1 guide RNA or a nucleic acid encoding the same. Thus, as a result of the gene editing, removal of a specific sequence portion in the target gene or target nucleic acid occurs.

### Examples of gene editing method

In an embodiment, the gene editing method may comprise delivering, into a eukaryotic cell, a CRISPR/Cas12f1 complex in a form of a ribonucleoprotein particle in which an engineered Cas12f1 guide RNA and a Cas12f1 protein are bound to each other. Here, the delivery may be achieved by electroporation or lipofection.

In an embodiment, the gene editing method may comprise delivering, into a eukaryotic cell, a nucleic acid encoding an engineered Cas12f1 guide RNA and a nucleic acid encoding a Cas12f1 protein. Here, the delivery may be achieved by electroporation or lipofection.

In an embodiment, the gene editing method may comprise delivering, into a eukaryotic cell, an adeno-associated viral (AAV) vector comprising a nucleic acid sequence encoding an engineered Cas12f1 guide RNA and a nucleic acid sequence encoding a Cas12f1 protein.

In an embodiment, the gene editing method may comprise delivering, into a eukaryotic cell, an adeno-associated viral (AAV) vector comprising a nucleic acid sequence encoding a first engineered Cas12f1 guide RNA, a nucleic acid sequence encoding a second engineered Cas12f1 guide RNA, and a nucleic acid sequence encoding a Cas12f1 protein.

### Possible examples of present disclosure

### Example 1. sqRNA ver. A, MF

An engineered guide RNA for a CRISPR/Cas12f1 system, comprising:
an engineered scaffold region; and
a spacer;
wherein in the engineered guide RNA, the engineered scaffold region and the spacer are sequentially linked to each other in a 5' to 3' direction,
the spacer comprises 10 nucleotides to 50 nucleotides, and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
the sequence of the engineered scaffold region is such that the following sequences are sequentially linked to each other in a 5' to 3' direction:
   a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
   a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
   5'-GCUGCUUGCAUCAGCCUAAUGUCGAGAAGUGCUUUCUUCGGAAAGUAAC CCUCGA-3' (SEQ ID NO: 11);
   a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
   5'-AUGCAAC-3'.

### Example 2. sqRNA ver.A, WT

An engineered guide RNA for the CRISPR/Cas12f1 system, comprising:
an engineered scaffold region; and
a spacer,
wherein in the engineered guide RNA, the engineered scaffold region and the spacer are sequentially linked to each other in a 5' to 3' direction,
the spacer comprises 10 nucleotides to 50 nucleotides, and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAAGAAAGUUGCAGAACCCGAAUAGACGAAUGAAGGA AUGCAAC-3' (SEQ ID NO: 315), and
the sequence of the engineered scaffold region is such that the following sequences are sequentially linked to each other in a 5' to 3' direction:
   a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
   a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

   a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), 5'-AACAAAUUCAUUUGAAAGAAUGAAGGA-3' (SEQ ID NO: 117), 5'-AACAAAUUCAUUUUGAAACGAAUGAAGGA-3' (SEQ ID NO: 293), 5'-AACAAAUUCAUUUUUGAAAACGAAUGAAGGA-3' (SEQ ID NO: 294), 5'-AACAAAUUCAUUUUUCGAAAGACGAAUGAAGGA-3' (SEQ ID NO: 295), 5'-AACAAAUUCAUUUUUCCGAAAAGACGAAUGAAGGA-3' (SEQ ID NO: 296), 5'-AACAAAUUCAUUUUUCCUGAAAUAGACGAAUGAAGGA-3' (SEQ ID NO: 297), 5'-AACAAAUUCAUUUUUCCUCGAAAAUAGACGAAUGAAGGA-3' (SEQ ID NO: 298), 5'-AACAAAUUCAUUUUUCCUCUGAAAAAUAGACGAAUGAAGGA-3' (SEQ ID NO: 299), 5'-AACAAAUUCAUUUUUCCUCUCGAAAGAAUAGACGAAUGAAGGA-3' (SEQ ID NO: 300), 5'-AACAAAUUCAUUUUUCCUCUCCGAAACGAAUAGACGAAUGAAGGA-3' (SEQ ID NO: 301), 5'-AACAAAUUCAUUUUUCCUCUCCAGAAACCGAAUAGACGAAUGAAGGA-3' (SEQ ID NO: 302), 5'-AACAAAUUCAUUUUUCCUCUCCAAGAAACCCGAAUAGACGAAUGAAGGA-3' (SEQ ID NO: 303), 5'-AACAAAUUCAUUUUUCCUCUCCAAUGAAAACCCGAAUAGACGAAUGAAGG A-3' (SEQ ID NO: 304), 5'-AACAAAUUCAUUUUUCCUCUCCAAUUGAAAAACCCGAAUAGACGAAUGAA GGA-3' (SEQ ID NO: 305), 5'-AACAAAUUCAUUUUUCCUCUCCAAUUCGAAAGAACCCGAAUAGACGAAUG AAGGA-3' (SEQ ID NO: 306), 5'-AACAAAUUCAUUUUUCCUCUCCAAUUCUGAAAAGAACCCGAAUAGACGAA UGAAGGA-3' (SEQ ID NO: 307), 5'-AACAAAUUCAUUUUUCCUCUCCAAUUCUGGAAACAGAACCCGAAUAGAC GAAUGAAGGA-3' (SEQ ID NO: 308), 5'-AACAAAUUCAUUUUUCCUCUCCAAUUCUGCGAAAGCAGAACCCGAAUAG ACGAAUGAAGGA-3' (SEQ ID NO: 309), 5'-AACAAAUUCAUUUUUCCUCUCCAAUUCUGCAGAAAUGCAGAACCCGAAUA GACGAAUGAAGGA-3' (SEQ ID NO: 310), 5'-AACAAAUUCAUUUUUCCUCUCCAAUUCUGCACGAAAUUGCAGAACCCGA AUAGACGAAUGAAGGA-3' (SEQ ID NO: 311), 5'-AACAAAUUCAUUUUUCCUCUCCAAUUCUGCACAGAAAGUUGCAGAACCC GAAUAGACGAAUGAAGGA-3' (SEQ ID NO: 312), 5'-AACAAAUUCAUUUUUCCUCUCCAAUUCUGCACAGAAAUUGCAGAACCCG AAUAGACGAAUGAAGGA-3' (SEQ ID NO: 313), and 5'-AACAAAUUCAUUUUUCCUCUCCAAUUCUGCACAAGAAAGUUGCAGAACC CGAAUAGACGAAUGAAGGA-3' (SEQ ID NO: 314); and
   5'-AUGCAAC-3'.

### Example 3. sgRNA ver. A, v4.0

The engineered guide RNA of Example 1, wherein the sequence of the scaffold region is such that the following sequences are sequentially linked to each other in a 5' to 3' direction:
5'-A-3';

5'-
CCGCUUCACCAAAAGCUGUCCCUUAGGGGAUUAGAACUUGAGUGAAGGU
GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

5'-AACAAAGAAAGGA-3' (SEQ ID NO: 110); and
5'-AUGCAAC-3'.

### Example 4. sgRNA ver.A, v4.1

The engineered guide RNA of Example 1, wherein the sequence of the scaffold region is such that the following sequences are sequentially linked to each other in a 5' to 3' direction:
5'-A-3';
5'-CCGCUUCACUUAGAGUGAAGGUGG-3' (SEQ ID NO: 430);

5'-
GCUGCUUGCAUCAGCCUAAUGUCGAGAAGUGCUUUCUUCGGAAAGUAAC
CCUCGA-3' (SEQ ID NO: 11);

5'-AACAAAGAAAGGA-3' (SEQ ID NO: 110); and
5'-AUGCAAC-3'.

### Example 5. sgRNA ver.B

An engineered guide RNA for the CRISPR/Cas12f1 system, comprising:
an engineered scaffold region; and
a spacer,
wherein in the engineered guide RNA, the engineered scaffold region and the spacer are sequentially linked to each other in a 5' to 3' direction,
the spacer has a length of 10 to 50 nucleotides, and has a sequence complementary to a target sequence, and
a sequence of the engineered scaffold region is such that the following sequences are sequentially linked to each other in a 5' to 3' direction:
   a first sequence represented by 5'-A-3';
   a second sequence represented by 5'-CCGCUUCAC-3' (SEQ ID NO: 432);
   a third sequence represented by 5'-UUAG-3';
   a fourth sequence represented by 5'-AGUGAAGGUGG-3' (SEQ ID NO: 433);
   a fifth sequence represented by 5'-GCUGCUUGCAUCAGCCUAAUGUCGAGAAGUGCUUUCUUCGGAAAGUAAC CCUCGA-3' (SEQ ID NO: 11);
   a sixth sequence represented by 5'-AACAAA-3';
   a linker;
   a seventh sequence represented by 5'-GGA-3'; and
   an eighth sequence represented by 5'-AUGCAAC-3'.

### Example 6. sgRNA ver.B, Linker

The engineered guide RNA of Example 5, wherein the linker is 5'-GAAA-3'.

### Example 7. sgRNA ver.B, Linker variations

The engineered guide RNA of Example 5, wherein the linker is selected from the group consisting of 5'-GAAA-3', 5'-UGAAAA-3', 5'-UUGAAAAA-3', 5'-UUCGAAAGAA-3' (SEQ ID NO: 425), 5'-UUCAGAAAUGAA-3' (SEQ ID NO: 426), 5'-UUCAUGAAAAUGAA-3' (SEQ ID NO: 427), and 5'-UUCAUUGAAAAAUGAA-3' (SEQ ID NO: 428).

### Example 8. sgRNA ver.B, + 1st region variations

The engineered guide RNA of Example 5, wherein the sequence of the engineered scaffold region further comprises a ninth sequence selected from the group consisting of 5'-A-3', 5'-GA-3', 5'-AGA-3', 5'-GAGA-3', 5'-GGAGA-3', 5'-UGGAGA-3', 5'-GUGGAGA-3', 5'-AGUGGAGA-3', 5'-AAGUGGAGA-3', 5'-AAAGUGGAGA-3' (SEQ ID NO: 27), 5'-UAAAGUGGAGA-3' (SEQ ID NO: 28), 5'-AUAAAGUGGAGA-3' (SEQ ID NO: 29), 5'-GAUAAAGUGGAGA-3' (SEQ ID NO: 30), 5'-UGAUAAAGUGGAGA-3' (SEQ ID NO: 31), 5'-CUGAUAAAGUGGAGA-3' (SEQ ID NO: 32), 5'-ACUGAUAAAGUGGAGA-3' (SEQ ID NO: 33), 5'-CACUGAUAAAGUGGAGA-3' (SEQ ID NO: 34), 5'-UCACUGAUAAAGUGGAGA-3' (SEQ ID NO: 35), 5'-UUCACUGAUAAAGUGGAGA-3' (SEQ ID NO: 36), and 5'-CUUCACUGAUAAAGUGGAGA-3' (SEQ ID NO: 37), and the 3' end of the ninth sequence is linked to the 5' end of the first sequence.

### Example 9. sgRNA ver.B, + 2nd region variations

The engineered guide RNA of Example 5, wherein the sequence of the engineered scaffold region further comprises:a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 52), and 5'-AAAGCUGUCCC-3' (SEQ ID NO: 53); and
an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 54), 5'-GGAUUAGAACU-3' (SEQ ID NO: 55), and 5'-GGGAUUAGAACU-3' (SEQ ID NO: 56),
wherein the 3' end of the second sequence and the 5' end of the third sequence are linked to each other by the tenth sequence, and
the 3' end of the third sequence and the 5' end of the fourth sequence are linked to each other by the eleventh sequence.

### Example 10. sgRNA ver.B, + 2nd region examples

The engineered guide RNA of Example 9, wherein
when the tenth sequence is 5'-A-3', the eleventh sequence is 5'-U-3',
when the tenth sequence is 5'-AA-3', the eleventh sequence is 5'-CU-3',
when the tenth sequence is 5'-AAA-3', the eleventh sequence is 5'-ACU-3',
when the tenth sequence is 5'-AAAG-3', the eleventh sequence is 5'-AACU-3',
when the tenth sequence is 5'-AAAGC-3', the eleventh sequence is 5'-GAACU-3',
when the tenth sequence is 5'-AAAGCU-3', the eleventh sequence is 5'-AGAACU-3',
when the tenth sequence is 5'-AAAGCUG-3', the eleventh sequence is 5'-UAGAACU-3', or 5'-UUAGAACU-3',
when the tenth sequence is 5'-AAAGCUGU-3', the eleventh sequence is 5'-AUUAGAACU-3',
when the tenth sequence is 5'-AAAGCUGUC-3', the eleventh sequence is 5'-GAUUAGAACU-3' (SEQ ID NO:54),
when the tenth sequence is 5'-AAAGCUGUCC-3' (SEQ ID NO: 52), the eleventh sequence is 5'-GGAUUAGAACU-3' (SEQ ID NO:55),
when the tenth sequence is 5'-AAAGCUGUCCC-3' (SEQ ID NO: 53), the eleventh sequence is 5'-GGGAUUAGAACU-3' (SEQ ID NO:56),
when the tenth sequence is 5'-AAAAGCUGUCCC-3' (SEQ ID NO: 440), the eleventh sequence may be 5'-GGGAUUAGAACUU-3' (SEQ ID NO: 442), or
when the tenth sequence is 5'-CAAAAGCUGUCCC-3' (the group consisting of ID NO: 441), the eleventh sequence is '5'-GGGAUUAGAACUU'-3' (SEQ ID NO: 443).

### Example 11. sgRNA ver.B, v4.0

The engineered guide RNA of Example 10, wherein the tenth sequence i' 5'-CAAAAGCUGUCC'-3' (SEQ ID NO: 441), and the eleventh sequence i' 5'-GGGAUUAGAACUU'-3' (SEQ ID NO: 443).

### Example 12. sgRNA ver.B, + 4th &5th region variations

The engineered guide RNA of Example 5, wherein the sequence of the engineered scaffold region further comprises:
a twelfth sequence selected from the group consisting of 5'-U-3', 5'-UU-3', 5'-UUC-3', 5'-UUCA-3', 5'-UUCAU-3', 5'-UUCAUU-3', and 5'-UUCAUUU-3'; and
a thirteenth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3',
wherein the 3' end of the sixth sequence and the 5' end of the linker are linked to each other by the twelfth sequence, and
the 3' end of the third sequence and the 5' end of the seventh sequence are linked to each other by the thirteenth sequence.

### Example 13. sgRNA ver.B, 4th & 5th region examples

The engineered guide RNA of Example 12, wherein
when the twelfth sequence is 5'-U-3', the thirteenth sequence is 5'-A-3',
when the twelfth sequence is 5'-UU-3', the thirteenth sequence is 5'-AA-3',
when the twelfth sequence is 5'-UUC-3', the thirteenth sequence is 5'-GAA-3',
when the twelfth sequence is 5'-UUCA-3', the thirteenth sequence is 5'-UGAA-3',
when the twelfth sequence is 5'-UUCAU-3', the thirteenth sequence is 5'-AUGAA-3',
when the twelfth sequence is 5'-UUCAUU-3', the thirteenth sequence is 5'-AAUGAA-3', or
when the twelfth sequence is 5'-UUCAUUU-3', the thirteenth sequence is 5'-GAAU GAA-3' .

### Example 14. Dual gRNA

An engineered guide RNA for the CRISPR/Cas12f1 system, comprising:
an engineered scaffold region; and
a spacer;
wherein the spacer has a length of 10 to 50 nucleotides, and has a sequence complementary to a target sequence,
a sequence of the engineered scaffold region comprises in a 5' to 3' direction:
   an engineered tracrRNA in which the following sequences are linked to each other:
   a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9),
   a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10), 
5'-
GCUGCUUGCAUCAGCCUAAUGUCGAGAAGUGCUUUCUUCGGAAAGUAAC
CCUCGA-3' (SEQ ID NO: 11),
   and
   a sequence selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 66), 5'-AACAAAUUCAU-3' (SEQ ID NO: 67), 5'-AACAAAUUCAUU-3' (SEQ ID NO: 68), and 5'-AACAAAUUCAUUU-3' (SEQ ID NO: 12); and
   an engineered crRNA repeat sequence portion in which the following sequences are linked to each other:
      a sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', and 5'-GAAUGAAGGA-3' (SEQ ID NO: 14), and
      5'-AUGCAAC-3',
      wherein the 3' end of the engineered crRNA repeat sequence is linked to the 5' end of the spacer, and
      wherein one in which a sequence of the engineered tracrRNA is the same as 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUU-3' (SEQ ID NO: 1) and the engineered crRNA repeat sequence is the same as 5'-GAAUGAAGGAAUGCAAC-3' (SEQ ID NO: 3) is excluded.

### Example 15. complex, ver. A

An engineered CRISPR/Cas12f1 complex capable of editing a target sequence-containing nucleic acid, comprising:
a Cas12f1 protein; and
an engineered guide RNA,
wherein the engineered guide RNA comprises:
   an engineered scaffold region; and
   a spacer;
   wherein in the engineered guide RNA, the engineered scaffold region and the spacer are sequentially linked to each other in a 5' to 3' direction,
   the spacer comprises 10 nucleotides to 50 nucleotides, and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
      5'-AUGCAAC-3'.

### Example 16. Complex, ver. A, v4.0

The engineered CRISPR/Cas12f1 complex of Example 15, wherein the sequence of the scaffold region included in the engineered guide RNA is such that the following sequences are sequentially linked to each other in a 5' to 3' direction:
5'-A-3';

5'-
CCGCUUCACCAAAAGCUGUCCCUUAGGGGAUUAGAACUUGAGUGAAGGU
GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

5'-AACAAAGAAAGGA-3' (SEQ ID NO: 110); and
5'-AUGCAAC-3'.

### Example 17. Complex, ver. A, v4.1

The engineered CRISPR/Cas12f1 complex of Example 15, wherein the sequence of the scaffold region included in the engineered guide RNA is such that the following sequences are sequentially linked to each other in a 5' to 3' direction:
5'-A-3';
5'-CCGCUUCACUUAGAGUGAAGGUGG-3' (SEQ ID NO: 430);

5'-
GCUGCUUGCAUCAGCCUAAUGUCGAGAAGUGCUUUCUUCGGAAAGUAAC
CCUCGA-3' (SEQ ID NO: 11);

5'-AACAAAGAAAGGA-3' (SEQ ID NO: 110); and
5'-AUGCAAC-3'.

### Example 18. Complex, ver. B

An engineered CRISPR/Cas12f1 complex capable of editing a target sequence-containing nucleic acid, comprising:
a Cas12f1 protein; and
the engineered guide RNA of any one of Examples 5 to 13.

### Example 19. Vector, ver. A

A vector capable of expressing respective components of a CRISPR/Cas12f1 system, comprising:
a first sequence comprising a nucleic acid sequence encoding a Cas12f1 protein;
a first promoter sequence operably linked to the first sequence;
a second sequence comprising a nucleic acid sequence encoding the engineered guide RNA; and
a second promoter sequence operably linked to the second sequence,
wherein the engineered guide RNA comprises:
   an engineered scaffold region; and
   a spacer;
   wherein in the engineered guide RNA, the engineered scaffold region and the spacer are sequentially linked to each other in a 5' to 3' direction,
   the spacer comprises 10 nucleotides to 50 nucleotides, and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
      5'-AUGCAAC-3'.

### Example 20. Vector, ver. A, v4.0

The vector of Example 19, wherein the sequence of the scaffold region included in the engineered guide RNA is such that the following sequences are sequentially linked to each other in a 5' to 3' direction:
5'-A-3';

5'-
CCGCUUCACCAAAAGCUGUCCCUUAGGGGAUUAGAACUUGAGUGAAGGU
GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

5'-AACAAAGAAAGGA-3' (SEQ ID NO: 110); and
5'-AUGCAAC-3'.

### Example 21. Vector, ver. A, v4.1

The vector of Example 19, wherein the sequence of the scaffold region included in the engineered guide RNA is such that the following sequences are sequentially linked to each other in a 5' to 3' direction:
5'-A-3';
5'-CCGCUUCACUUAGAGUGAAGGUGG-3' (SEQ ID NO: 430);

5'-
GCUGCUUGCAUCAGCCUAAUGUCGAGAAGUGCUUUCUUCGGAAAGUAAC
CCUCGA-3' (SEQ ID NO: 11);

5'-AACAAAGAAAGGA-3' (SEQ ID NO: 110); and
5'-AUGCAAC-3'.

### Example 22. Vector, ver.A, viral or plasmid

The vector of Example 19, wherein the vector is at least one selected from the group consisting of a plasmid, a retrovirus, a lentivirus, an adenovirus, an adeno-associated virus, a vaccinia virus, a poxvirus, and a herpes simplex virus.

### Example 23. Vector, ver. B

A vector capable of expressing respective components of a CRISPR/Cas12f1 system, comprising:
a first sequence comprising a nucleic acid sequence encoding a Cas12f1 protein;
a first promoter sequence operably linked to the first sequence;
a second sequence comprising a nucleic acid sequence encoding the engineered guide RNA of any one of Examples 5 to 13; and
a second promoter sequence operably linked to the second sequence.

### Example 24. Editing method, ver.A, MF

A method of editing a target sequence-containing nucleic acid in a cell, comprising:
delivering, into the cell, a Cas12f1 protein or a nucleic acid encoding the same, and an engineered guide RNA or a nucleic acid encoding the same,
which allows a CRISPR/Cas12f1 complex to be formed in the cell, and
wherein the CRISPR/Cas12f1 complex is capable of editing the target sequence-containing nucleic acid,
the engineered guide RNA comprises:
   an engineered scaffold region; and
   a spacer;
   wherein in the engineered guide RNA, the engineered scaffold region and the spacer are sequentially linked to each other in a 5' to 3' direction,
   the spacer comprises 10 nucleotides to 50 nucleotides, and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
      5'-AUGCAAC-3'.

### Example 25. Editing method, ver. A, WT

A method of editing a target sequence-containing nucleic acid in a cell, comprising:
delivering, into the cell, a Cas12f1 protein or a nucleic acid encoding the same, and an engineered guide RNA or a nucleic acid encoding the same,
which allows a CRISPR/Cas12f1 complex to be formed in the cell, and
wherein the CRISPR/Cas12f1 complex is capable of editing the target sequence-containing nucleic acid,
the engineered guide RNA comprises:
   an engineered scaffold region; and
   a spacer,
   wherein in the engineered guide RNA, the engineered scaffold region and the spacer are sequentially linked to each other in a 5' to 3' direction,
   the spacer comprises 10 nucleotides to 50 nucleotides, and has a sequence complementary to a target sequence,
   a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAAGAAAGUUGCAGAACCCGAAUAGACGAAUGAAGGA AUGCAAC-3' (SEQ ID NO: 315), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
      a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);

5'-
GCUGCUUGCAUCAGCCUAAUGUCGAGAAGUGCUUUCUUCGGAAAGUAAC
CCUCGA-3' (SEQ ID NO: 11);

      a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), 5'-AACAAAUUCAUUUGAAAGAAUGAAGGA-3' (SEQ ID NO: 117), 5'-AACAAAUUCAUUUUGAAACGAAUGAAGGA-3' (SEQ ID NO: 293), 5'-AACAAAUUCAUUUUUGAAAACGAAUGAAGGA-3' (SEQ ID NO: 294), 5'-AACAAAUUCAUUUUUCGAAAGACGAAUGAAGGA-3' (SEQ ID NO: 295), 5'-AACAAAUUCAUUUUUCCGAAAAGACGAAUGAAGGA-3' (SEQ ID NO: 296), 5'-AACAAAUUCAUUUUUCCUGAAAUAGACGAAUGAAGGA-3' (SEQ ID NO: 297), 5'-AACAAAUUCAUUUUUCCUCGAAAAUAGACGAAUGAAGGA-3' (SEQ ID NO: 298), 5'-AACAAAUUCAUUUUUCCUCUGAAAAAUAGACGAAUGAAGGA-3' (SEQ ID NO: 299), 5'-AACAAAUUCAUUUUUCCUCUCGAAAGAAUAGACGAAUGAAGGA-3' (SEQ ID NO: 300), 5'-AACAAAUUCAUUUUUCCUCUCCGAAACGAAUAGACGAAUGAAGGA-3' (SEQ ID NO: 301), 5'-AACAAAUUCAUUUUUCCUCUCCAGAAACCGAAUAGACGAAUGAAGGA-3' (SEQ ID NO: 302), 5'-AACAAAUUCAUUUUUCCUCUCCAAGAAACCCGAAUAGACGAAUGAAGGA-3' (SEQ ID NO: 303), 5'-AACAAAUUCAUUUUUCCUCUCCAAUGAAAACCCGAAUAGACGAAUGAAGG A-3' (SEQ ID NO: 304), 5'-AACAAAUUCAUUUUUCCUCUCCAAUUGAAAAACCCGAAUAGACGAAUGAA GGA-3' (SEQ ID NO: 305), 5'-AACAAAUUCAUUUUUCCUCUCCAAUUCGAAAGAACCCGAAUAGACGAAUG AAGGA-3' (SEQ ID NO: 306), 5'-AACAAAUUCAUUUUUCCUCUCCAAUUCUGAAAAGAACCCGAAUAGACGAA UGAAGGA-3' (SEQ ID NO: 307), 5'-AACAAAUUCAUUUUUCCUCUCCAAUUCUGGAAACAGAACCCGAAUAGAC GAAUGAAGGA-3' (SEQ ID NO: 308), 5'-AACAAAUUCAUUUUUCCUCUCCAAUUCUGCGAAAGCAGAACCCGAAUAG ACGAAUGAAGGA-3' (SEQ ID NO: 309), 5'-AACAAAUUCAUUUUUCCUCUCCAAUUCUGCAGAAAUGCAGAACCCGAAUA GACGAAUGAAGGA-3' (SEQ ID NO: 310), 5'-AACAAAUUCAUUUUUCCUCUCCAAUUCUGCACGAAAUUGCAGAACCCGA AUAGACGAAUGAAGGA-3' (SEQ ID NO: 311), 5'-AACAAAUUCAUUUUUCCUCUCCAAUUCUGCACAGAAAGUUGCAGAACCC GAAUAGACGAAUGAAGGA-3' (SEQ ID NO: 312), 5'-AACAAAUUCAUUUUUCCUCUCCAAUUCUGCACAGAAAUUGCAGAACCCG AAUAGACGAAUGAAGGA-3' (SEQ ID NO: 313), and 5'-AACAAAUUCAUUUUUCCUCUCCAAUUCUGCACAAGAAAGUUGCAGAACC CGAAUAGACGAAUGAAGGA-3' (SEQ ID NO: 314); and
      5'-AUGCAAC-3'.

### Example 26. Editing method, ver. A, introducing RNP

The method of any one of Example 24 or Example 25, wherein the delivery is achieved by introducing, in the cell, the Cas12f1 protein and the engineered guide RNA as a CRISPR/Cas12f1 complex.

### Example 27. Editing method, ver. A, introducing vector

The method of Example 24, wherein the delivery is achieved by introducing, into the cell, a vector comprising a nucleic acid encoding the Cas12f1 protein and a nucleic acid encoding the engineered guide RNA.

### Example 28. Editing method, ver.A, editing eukaryotic cell

The method of any one of Example 24 or Example 25, wherein the cell is a eukaryotic cell.

### Example 29. Editing method, ver. A, vector limitation

The vector of Example 27, wherein the vector is at least one selected from the group consisting of a plasmid, a retrovirus, a lentivirus, an adenovirus, an adeno-associated virus, a vaccinia virus, a poxvirus, and a herpes simplex virus.

### Example 30. Editing method, ver.B

A method of editing a target sequence-containing nucleic acid in a cell, comprising:
delivering, into the cell, a Cas12f1 protein or a nucleic acid encoding the same, and the engineered guide RNA any one of Examples 5 to 13 or a nucleic acid encoding the same,
which allows a CRISPR/Cas12f1 complex to be formed in the cell, and
wherein the CRISPR/Cas12f1 complex is capable of editing the target sequence-containing nucleic acid.

### Example 31. sqRNA, ver.A, DNA

DNA encoding the engineered guide RNA of any one of Examples 1 to 2.

### Example 32. sqRNA, ver.B, DNA

DNA encoding the engineered guide RNA of any one of Examples 5 to 13.

### Example 33. Dual guide RNA, DNA

DNA encoding the engineered guide RNA of Example 14.

Hereinafter, the present disclosure will be described in more detail through experimental examples and examples. These examples are only for illustrating the present disclosure, and it would be obvious to those skilled in the art that a scope of the disclosure is not to be construed as being limited by these examples.

### Experimental Example 1. Preparation of materials for experiment

### Experimental Example 1. 1. Design and construction of plasmid vector

A Cas12f1 gene was codon-optimized for expression in human cells, and the optimized sequence was synthesized for vector construction. Finally, to the Cas12f1 protein-encoding sequence were added a chicken β-actin promoter, a nuclear localization signal sequence at the 5'-end and the 3'-end, and a sequence encoding an enhanced green fluorescent protein (eGFP) linked by a self-cleaving T2A peptide. An amino acid sequence of the Cas12f1 protein and a DNA sequence encoding the same are shown in Table 01.

**[Table 01]**

| **Label** | **Sequence(N-terminal to C-terminal/5' to 3')** | **SEQ ID NO** |
|---|---|---|
| Cas12f1 + NLS(N/C-terminal) seq uence (peptide) | MPKKKRKVAKNTITKTLKLRIVRPYNSAEVEKIVADEKNNREKIALEKNKDKVKEACSKHLKVAAYCTTQVERNACLFCKARKLDDKFYQKLRGQFPDAVFWQEISEIFRQLQKQA AEIYNQSLIELYYEIFIKGKGIANASSVEHYLSDVCYTRAAELFKNAAIASGLRSKIKSNFRLKELKNMKSGLPTTKSDNFPIPLVKQKGGQYTGFEISNHNSDFIIKIPFGRWQVKKEI DKYRPWEKFDFEQVQKSPKPISLLLSTQRRKRNKGWSKDEGTEAEIKKVMNGDYQTSYIEVKRGSKIGEKSAWMLNLSIDVPKIDKGVDPSIIGGIDVGVKSPLVCAINNAFSRYSI SDNDLFHFNKKMFARRRILLKKNRHKRAGHGAKNKLKPITILTEKSERFRKKLIERWACEIADFFIKNKVGTVQMENLESMKRKEDSYFNIRLRGFWPYAEMQNKIEFKLKQYGIE IRKVAPNNTSKTCSKCGHLNNYFNFEYRKKNKFPHFKCEKCNFKENADYNAALNISNPKLKSTKEEPPKKKRKV | 262 |
| Cas12f1 + NLS(N/C-terminal) sequence (DNA) | ATGccaaagaagaagcggaaagtcGCCAAGAACACAATTACAAAGACACTGAAGCTGAGGATCGTGAGACCATACAACAGCGCTGAGGTCGAGAAGATTGTGGCTGATGAAA AGAACAACAGGGAAAAGATCGCCCTCGAGAAGAACAAGGATAAGGTGAAGGAGGCCTGCTCTAAGCACCTGAAAGTGGCCGCCTACTGCACCACACAGGTGGAGAGG AACGCCTGTCTGTTTTGTAAAGCTCGGAAGCTGGATGATAAGTTTTACCAGAAGCTGCGGGGCCAGTTCCCCGATGCCGTCTTTTGGCAGGAGATTAGCGAGATCTTCAGA CAGCTGCAGAAGCAGGCCGCCGAGATCTACAACCAGAGCCTGATCGAGCTCTACTACGAGATCTTCATCAAGGGCAAGGGCATTGCCAACGCCTCCTCCGTGGAGCACTA CCTGAGCGACGTGTGCTACACAAGAGCCGCCGAGCTCTTTAAGAACGCCGCTATCGCTTCCGGGCTGAGGAGCAAGATTAAGAGTAACTTCCGGCTCAAGGAGCTGAAG AACATGAAGAGCGGCCTGCCCACTACAAAGAGCGACAACTTCCCAATTCCACTGGTGAAGCAGAAGGGGGGCCAGTACACAGGGTTCGAGATTTCCAACCACAACAGC GACTTTATTATTAAGATCCCCTTTGGCAGGTGGCAGGTCAAGAAGGAGATTGACAAGTACAGGCCCTGGGAGAAGTTTGATTTCGAGCAGGTGCAGAAGAGCCCCAAGC CTATTTCCCTGCTGCTGTCCACACAGCGGCGGAAGAGGAACAAGGGGTGGTCTAAGGATGAGGGGACCGAGGCCGAGATTAAGAAAGTGATGAACGGCGACTACCAG ACAAGCTACATCGAGGTCAAGCGGGGCAGTAAGATTGGCGAGAAGAGCGCCTGGATGCTGAACCTGAGCATTGACGTGCCAAAGATTGATAAGGGCGTGGATCCCAGC ATCATCGGAGGGATCGATGTGGGGGTCAAGAGCCCCCTCGTGTGCGCCATCAACAACGCCTTCAGCAGGTACAGCATCTCCGATAACGACCTGTTCCACTTTAACAAGAA GATGTTCGCCCGGCGGAGGATTTTGCTCAAGAAGAACCGGCACAAGCGGGCCGGACACGGGGCCAAGAACAAGCTCAAGCCCATCACTATCCTGACCGAGAAGAGCGA GAGGTTCAGGAAGAAGCTCATCGAGAGATGGGCCTGCGAGATCGCCGATTTCTTTATTAAGAACAAGGTCGGAACAGTGCAGATGGAGAACCTCGAGAGCATGAAGAG GAAGGAGGATTCCTACTTCAACATTCGGCTGAGGGGGTTCTGGCCCTACGCTGAGATGCAGAACAAGATTGAGTTTAAGCTGAAGCAGTACGGGATTGAGATCCGGAAG GTGGCCCCCAACAACACCAGCAAGACCTGCAGCAAGTGCGGGCACCTCAACAACTACTTCAACTTCGAGTACCGGAAGAAGAACAAGTTCCCACACTTCAAGTGCGAGA AGTGCAACTTTAAGGAGAACGCCGATTACAACGCCGCCCTGAACATCAGCAACCCTAAGCTGAAGAGCACTAAGGAGGAGCCccaaagaagaagcggaaagtc | 272 |

A template DNA encoding a (engineered) Cas12f1 guide RNA was synthesized and cloned into a pTwist Amp plasmid vector (Twist Bioscience). When necessary, the vector was used as a template for amplifying the sequence encoding the guide RNA using a U6-complementary forward primer and a protospacer-complementary reverse primer. Using a Gibson assembly, an oligonucleotide encoding the engineered Cas12f1 guide RNA was cloned into the vector comprising the codon-optimized Cas12f1 gene, so that a vector for an engineered CRISPR/Cas12f1 system was constructed.

### Experimental Example 1.2. Engineering of Cas12f1 guide RNA

Modification of the second region, the fourth and fifth regions of the engineered scaffold region of the engineered Cas12f1 guide RNA was performed by cloning synthetic oligonucleotides, each of which delivers a modified sequence (Macrogen) into a linearized guide RNA-encoding vector, using Apo I and BamH I restriction enzymes. Modification of the first region of the engineered scaffold region of the engineered Cas12f1 guide RNA was performed by PCR amplification of a canonical or engineered template plasmid vector using a forward primer targeting the 5' end of the tracrRNA, and a reverse primer targeting the U6 promoter region. The PCR amplification was performed using a Q5 Hot Start high-fidelity DNA polymerase (NEB), and ligation of the PCR products was performed using a KLD Enzyme Mix (NEB). The ligated PCR product was transformed into DH5α E. coli cells. Mutagenesis was identified by a Sanger sequencing analysis. The modified plasmid vector was purified using a NucleoBond ^{®} Xtra Midi EF kit (MN). 1 microgram of the purified plasmid was used as a template for mRNA synthesis using T7 RNA polymerase (NEB) and NTPs (Jena Bioscience). The engineered Cas12f1 guide RNA prepared above was purified using a Monarch^{®} RNA cleanup kit (NEB), aliquoted into cryogenic vials and stored in liquid nitrogen.

### Experimental Example 1.3. Cell culture and transfection

HEK293 T cells (LentX-293T, Takara) were cultured under a condition of 5% of CO₂ in Dulbecco's modified eagle medium (DMEM) supplemented with 10% heat-inactivated fetal bovine serum (FBS) (Corning) and penicillin/streptomycin. Cell transfection was performed by electroporation or lipofection. For the electroporation, each 2 µg to 5 µg of the plasmid vector encoding the Cas12f1 protein and DNA encoding the guide RNA (or the engineered guide RNA) produced in Experimental Example 1.2 were transfected into 4X10⁵ HEK- 293 T cells using a Neon transfection system (Invitrogen). The electroporation was performed under conditions of 1300V, 10 mA, and 3 pulses. For the lipofection, 6 µL to 15 µL of FuGene reagent (Promega) was mixed for 15 minutes with 2 µg to 5 µg of the plasmid vector encoding a Cas12f1 protein and 1.5 µg to 5 µg of the PCR amplicon. The mixture (300 µL) was added to 1.5 ml DMEM medium plated with 1×10⁶ cells 1 day before transfection. The cells were cultured in the presence of the mixture for 1 day to 10 days. After culturing, the cells were collected, and genomic DNA of the cells was manually isolated using a PureHelix^{™} genomic DNA preparation kit (NanoHelix) or a Maxwell RSC Cultured cells DNA Kit (Promega).

### Experimental Example 1. 4. Measurement of intercellular indel efficiency

PCR was performed using target-specific primers in the presence of KAPA HiFi HotStart DNA polymerase (Roche) on a region including a protospacer in the genomic DNA isolated from HEK-293 T cells. The amplification was performed following the manufacturer's instructions. The PCR amplicon, which is a resulting product of the amplification and contains Illumina TruSeq HT dual indexes, was subjected to 150-bp paired end sequencing using Illumina iSeq 100. Indel frequencies were calculated using MAUND. The MAUND is provided at https://github.com/ibs-cge/maund.

### Experimental Example 1.5. Quantitave real-time PCR

A guide RNA (or engineered guide RNA) or a genomic DNA was each extracted from HEK293 T cells using a RNeasy Miniprep kit (Qiagen), a Maxwell RSC miRNA Tissue Kit (Promega), or a DNeasy Blood & Tissue Kit (Qiagen). To quantify the guide RNA, ligation of an RNA-specific primer was performed and cDNA was synthesized using a crRNA-specific primer. The cDNA was then used as a template for quantitative real-time PCR. The real-time PCR was analyzed using a KAFA SYBR FAST qPCR Master Mix (2X) Kit (KAPAbiosystems).

### Experimental Example 1.6. Statistical analysis

For each experimental example, the experiment was performed three times, and an average of the respective values was used for analysis.

### Experimental Example 2. Indel efficiency comparison between engineered CRISPR/Cas12f1 systems

In order to measure indel efficiency of an engineered CRISPR/Cas12f1 system using an engineered Cas12f1 guide RNA, each of the examples was prepared by Experimental Examples 1.1 to 1.2. The target sequences used for the experiments are shown in Table 02 below.

**[Table 02]**

| **Target Label** | **Protospacer sequence (5' to 3')** | **SEQ ID NO** |
|---|---|---|
| DY2 | CACACACACAGTGGGCTACC | 422 |
| DY10 | CATCCCCAGGACACACACAC | 423 |
| Intergenic-22 | AGAACACATACCCCTGGGCC | 424 |

Sequences of the engineered Cas12f1 guide RNAs used in respective examples are shown in Table 03 to Table 08.

**[Table 03]**

| **Label** | **Target** | **PAM(5' to 3')** | **Engineered Cas12f1 guide RNA sequence (5' to 3')** | **SEQ ID NO** |
|---|---|---|---|---|
| Comparative Example 1.1 | DY2 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCA UUUGAAAGAAUGAAGGAAUGCAACCACACACACAGUGGGCUACC | 380 |
| Example 1.1 | DY2 | TTTG | GAUAAAGUGGAGAACCGCUUCACCAAAAGCUGUCCCUUAGGGGAUUAGAACUUGAGUGAAGGUGGGCU GCUUGCAUCAGCCUAAUGUCGAGAAGUGCUUUCUUCGGAAAGUAACCCUCGAAACAAAUUCAUUUGAAA GAAUGAAGGAAUGCAACCACACACACAGUGGGCUACC | 381 |
| Example 1.2 | DY2 | TTTG | UGGAGAACCGCUUCACCAAAAGCUGUCCCUUAGGGGAUUAGAACUUGAGUGAAGGUGGGCUGCUUGCA UCAGCCUAAUGUCGAGAAGUGCUUUCUUCGGAAAGUAACCCUCGAAACAAAUUCAUUUGAAAGAAUGA AGGAAUGCAACCACACACACAGUGGGCUACC | 382 |
| Example 1.3 | DY2 | TTTG | ACCGCUUCACCAAAAGCUGUCCCUUAGGGGAUUAGAACUUGAGUGAAGGUGGGCUGCUUGCAUCAGCCU AAUGUCGAGAAGUGCUUUCUUCGGAAAGUAACCCUCGAAACAAAUUCAUUUGAAAGAAUGAAGGAAUG CAACCACACACACAGUGGGCUACC | 383 |
| Example 1.4 | DY2 | TTTG | CUUCACUGAUAAAGUGGAGAACCGCUUCACCAAAAGCUGUUUAGAUUAGAACUUGAGUGAAGGUGGGC UGCUUGCAUCAGCCUAAUGUCGAGAAGUGCUUUCUUCGGAAAGUAACCCUCGAAACAAAUUCAUUUGAA AGAAUGAAGGAAUGCAACCACACACACAGUGGGCUACC | 384 |
| Example 1.5 | DY2 | TTTG | CUUCACUGAUAAAGUGGAGAACCGCUUCACCAAAAGCUUUAGAGAACUUGAGUGAAGGUGGGCUGCUU GCAUCAGCCUAAUGUCGAGAAGUGCUUUCUUCGGAAAGUAACCCUCGAAACAAAUUCAUUUGAAAGAA UGAAGGAAUGCAACCACACACACAGUGGGCUACC | 385 |
| Example 1.6 | DY2 | TTTG | CUUCACUGAUAAAGUGGAGAACCGCUUCACCAUUAGUGAGUGAAGGUGGGCUGCUUGCAUCAGCCUAAU GUCGAGAAGUGCUUUCUUCGGAAAGUAACCCUCGAAACAAAUUCAUUUGAAAGAAUGAAGGAAUGCAA CCACACACACAGUGGGCUACC | 386 |

**[Table 04]**

| **Label** | **Target** | **PAM(5'to 3')** | **Engineered Cas12f1 guide RNA sequence (5' to 3')** | **SEQ ID NO** |
|---|---|---|---|---|
| Example 1.7 | DY2 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCA UGAAAAUGAAGGAAUGCAACCACACACACAGUGGGCUACC | 387 |
| Example 1.8 | DY2 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCG AAAGAAGGAAUGCAACCACACACACAGUGGGCUACC | 388 |
| Example 1.9 | DY2 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAGAAA GGAAUGCAACCACACACACAGUGGGCUACC | 389 |
| Example 1.10 | DY2 | TTTG | ACCGCUUCACCAAUUAGUUGAGUGAAGGUGGGCUGCUUGCAUCAGCCUAAUGUCGAGAAGUGCUUUCUU CGGAAAGUAACCCUCGAAACAAAUUCAUUUGAAAGAAUGAAGGAAUGCAACCACACACACAGUGGGCUA CC | 390 |
| Example 1.11 | DY2 | TTTG | ACCGCUUCACCAAAAGCUGUCCCUUAGGGGAUUAGAACUUGAGUGAAGGUGGGCUGCUUGCAUCAGCCU AAUGUCGAGAAGUGCUUUCUUCGGAAAGUAACCCUCGAAACAAAGAAAGGAAUGCAACCACACACACAG UGGGCUACC | 391 |
| Example 1.12 | DY2 | TTTG | CUUCACUGAUAAAGUGGAGAACCGCUUCACCAAUUAGUUGAGUGAAGGUGGGCUGCUUGCAUCAGCCUA AUGUCGAGAAGUGCUUUCUUCGGAAAGUAACCCUCGAAACAAAGAAAGGAAUGCAACCACACACACAGU GGGCUACC | 392 |
| Example 1.13 | DY2 | TTTG | ACCGCUUCACCAAUUAGUUGAGUGAAGGUGGGCUGCUUGCAUCAGCCUAAUGUCGAGAAGUGCUUUCUU CGGAAAGUAACCCUCGAAACAAAGAAAGGAAUGCAACCACACACACAGUGGGCUACC | 393 |
| Example 1.14 | DY2 | TTTG | ACCGCUUCACUUAGAGUGAAGGUGGGCUGCUUGCAUCAGCCUAAUGUCGAGAAGUGCUUUCUUCGGAAA GUAACCCUCGAAACAAAGAAAGGAAUGCAACCACACACACAGUGGGCUACC | 436 |

**[Table 05]**

| **Label** | **Target** | **PAM(5' to 3')** | **Engineered Cas12f1 guide RNA sequnce (5' to 3')** | **SEQ ID NO** |
|---|---|---|---|---|
| Comparative **E**xample 2.1 | DY10 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCA UUUGAAAGAAUGAAGGAAUGCAACCAUCCCCAGGACACACACAC | 394 |
| Example 2.1 | DY10 | TTTG | GAUAAAGUGGA GAACCGCUUCACCAAAAGCUGUCCCUUAGGGGAUUAGAACUUGAGUGAAGGUGGGCU GCUUGCAUCAGCCUAAUGUCGAGAAGUGCUUUCUUCGGAAAGUAACCCUCGAAACAAA UUCAUUUGAAA GAAUGAAGGAA UGCAACCA UCCCCAGGACACACACAC | 395 |
| Example 2.2 | DY10 | TTTG | UGGAGAACCGCUUCACCAAAAGCUGUCCCUUAGGGGAUUAGAACUUGAGUGAAGGUGGGCUGCUUGCA UCAGCCUAAUGUCGAGAAGUGCUUUCUUCGGAAAGUAACCCUCGAAACAAAUUCAUUUGAAAGAAUGA AGGAAUGCAACCAUCCCCAGGACACACACAC | 396 |
| Example 2.3 | DY10 | TTTG | ACCGCUUCACCAAAAGCUGUCCCUUAGGGGAUUAGAACUUGAGUGAAGGUGGGCUGCUUGCAUCAGCCU AAUGUCGAGAAGUGCUUUCUUCGGAAAGUAACCCUCGAAACAAAUUCAUUUGAAAGAAUGAAGGAAUG CAACCAUCCCCAGGACACACACAC | 397 |
| Example 2.4 | DY10 | TTTG | CUUCACUGAUAAAGUGGAGAACCGCUUCACCAAAAGCUGUUUAGAUUAGAACUUGAGUGAAGGUGGGC UGCUUGCAUCAGCCUAAUGUCGAGAAGUGCUUUCUUCGGAAAGUAACCCUCGAAACAAAUUCAUUUGAA AGAAUGAAGGAAUGCAACCAUCCCCAGGACACACACAC | 398 |
| Example 2.5 | DY10 | TTTG | CUUCACUGAUAAAGUGGAGAACCGCUUCACCAAAAGCUUUAGAGAACUUGAGUGAAGGUGGGCUGCUU GCAUCAGCCUAAUGUCGAGAAGUGCUUUCUUCGGAAAGUAACCCUCGAAACAAAUUCAUUUGAAAGAA UGAAGGAAUGCAACCAUCCCCAGGACACACACAC | 399 |
| Example 7.6 | DY10 | TTTG | CUUCACUGAUAAAGUGGAGAACCGCUUCACCAAUUAGUUGAGUGAAGGUGGGCUGCUUGCAUCAGCCUA AUGUCGAGAAGUGCUUUCUUCGGAAAGUAACCCUCGAAACAAAUUCAUUUGAAAGAAUGAAGGAAUGC AACCAUCCCCAGGACACACACAC | 400 |

**[Table 06]**

| **Label** | **Target** | **PAM(5' to 3')** | **Engineered Cas12f1 guide RNA sequence (5' to 3')** | **SEQ ID NO** |
|---|---|---|---|---|
| Example 27 | DY10 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCA UGAAAAUGAAGGAAUGCAACCAUCCCCAGGACACACACAC | 401 |
| Example 2.8 | DY10 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCG AAAGAAGGAAUGCAACCAUCCCCAGGACACACACAC | 402 |
| Example 2.9 | DY10 | TTTG | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAGAAA GGAAUGCAACCAUCCCCAGGACACACACAC | 403 |
| Example 2.10 | DY10 | TTTG | ACCGCUUCACCAAUUAGUUGAGUGAAGGUGGGCUGCUUGCAUCAGCCUAAUGUCGAGAAGUGCUUUCUU CGGAAAGUAACCCUCGAAACAAAUUCAUUUGAAAGAAUGAAGGAAUGCAACCAUCCCCAGGACACACAC AC | 404 |
| Example 1.11 | DY10 | TTTG | ACCGCUUCACCAAAAGCUGUCCCUUAGGGGAUUAGAACUUGAGUGAAGGUGGGCUGCUUGCAUCAGCCU AAUGUCGAGAAGUGCUUUCUUCGGAAAGUAACCCUCGAAACAAAGAAAGGAAUGCAACCAUCCCCAGGA CACACACAC | 405 |
| Example 2.12 | DY10 | TTTG | CUUCACUGAUAAAGUGGAGAACCGCUUCACCAAUUAGUUGAGUGAAGGUGGGCUGCUUGCAUCAGCCUA AUGUCGAGAAGUGCUUUCUUCGGAAAGUAACCCUCGAAACAAAGAAAGGAAUGCAACCAUCCCCAGGAC ACACACAC | 406 |
| Example 2.13 | DY10 | TTTG | ACCGCUUCACCAAUUAGUUGAGUGAAGGUGGGCUGCUUGCAUCAGCCUAAUGUCGAGAAGUGCUUUCUU CGGAAAGUAACCCUCGAAACAAAGAAAGGAAUGCAACCAUCCCCAGGACACACACAC | 407 |
| Example 1.14 | DY10 | TTTG | ACCGCUUCACUUAGAGUGAAGGUGGGCUGCUUGCAUCAGCCUAAUGUCGAGAAGUGCUUUCUUCGGAAA GUAACCCUCGAAACAAAGAAAGGAAUGCAACCAUCCCCAGGACACACACAC | 437 |

**[Table 07]**

| **Label** | **Target** | **PAM(5' to 3')** | **Engineered Cas12f1 guide RNA sequence (5' to 3')** | **SEQ ID NO** |
|---|---|---|---|---|
| Comparative Example 3.1 | Intergenic-22 | TTTA | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCA UUUGAAAGAAUGAAGGAAUGCAACAGAACACAUACCCCUGGGCC | 408 |
| Example 3.1 | Intergenic-22 | TTTA | GAUAAAGUGGA GAACCGCUUCACCAAAAGCUGUCCCUUAGGGGAUUAGAACUUGAGUGAAGGUGGGCU GCUUGCAUCAGCCUAAUGUCGAGAAGUGCUUUCUUCGGAAAGUAACCCUCGAAACAAA UUCAUUUGAAA GAAUGAAGGAA UGCAACAGAACA CAUACCCCUGGGCC | 409 |
| Example 3.2 | Intergenic-22 | TTTA | UGGAGAACCGCUUCACCAAAAGCUGUCCCUUAGGGGAUUAGAACUUGAGUGAAGGUGGGCUGCUUGCA UCAGCCUAAUGUCGAGAAGUGCUUUCUUCGGAAAGUAACCCUCGAAACAAAUUCAUUUGAAAGAAUGA AGGAAUGCAACAGAACACAUACCCCUGGGCC | 410 |
| Example 3.3 | Intergenic-22 | TTTA | ACCGCUUCACCAAAAGCUGUCCCUUAGGGGAUUAGAACUUGAGUGAAGGUGGGCUGCUUGCAUCAGCCU AAUGUCGAGAAGUGCUUUCUUCGGAAAGUAACCCUCGAAACAAAUUCAUUUGAAAGAAUGAAGGAAUG CAACAGAACACAUACCCCUGGGCC | 411 |
| Example 3.4 | Intergenic-22 | TTTA | CUUCACUGAUAAAGUGGAGAACCGCUUCACCAAAAGCUGUUUAGAUUAGAACUUGAGUGAAGGUGGGC UGCUUGCAUCAGCCUAAUGUCGAGAAGUGCUUUCUUCGGAAAGUAACCCUCGAAACAAAUUCAUUUGAA AGAAUGAAGGAAUGCAACAGAACACAUACCCCUGGGCC | 412 |
| Example 3.5 | Intergenic-22 | TTTA | CUUCACUGAUAAAGUGGAGAACCGCUUCACCAAAAGCUUUAGAGAACUUGAGUGAAGGUGGGCUGCUU GCAUCAGCCUAAUGUCGAGAAGUGCUUUCUUCGGAAAGUAACCCUCGAAACAAAUUCAUUUGAAAGAA UGAAGGAAUGCAACAGAACACAUACCCCUGGGCC | 413 |
| Example 3.6 | Intergenic-22 | TTTA | CUUCACUGAUAAAGUGGAGAACCGCUUCACCAAUUAGUUGAGUGAAGGUGGGCUGCUUGCAUCAGCCUA AUGUCGAGAAGUGCUUUCUUCGGAAAGUAACCCUCGAAACAAAUUCAUUUGAAAGAAUGAAGGAAUGC AACAGAACACAUACCCCUGGGCC | 414 |

**[Table 08]**

| **Label** | **Target** | **PAM(5' to 3')** | **Engineered Cas12f1 guide RNA sequence (5' to 3')** | **SEQ ID NO** |
|---|---|---|---|---|
| Example 3.7 | Intergenic-22 | TTTA | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCA UGAAAAUGAAGGAAUGCAACAGAACACAUACCCCUGGGCC | 415 |
| Example 3.8 | Intergenic-22 | TTTA | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAUUCG AAAGAAGGAAUGCAACAGAACACAUACCCCUGGGCC | 416 |
| Example 3.9 | Intergenic-22 | TTTA | CUUCACUGAUAAAGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCUCGAAACAAAGAAA GGAAUGCAACAGAACACAUACCCCUGGGCC | 417 |
| Example 3.10 | Intergenic-22 | TTTA | ACCGCUUCACCAAUUAGUUGAGUGAAGGUGGGCUGCUUGCAUCAGCCUAAUGUCGAGAAGUGCUUUCUU CGGAAAGUAACCCUCGAAACAAAUUCAUUUGAAAGAAUGAAGGAAUGCAACAGAACACAUACCCCUGGG CC | 418 |
| Example 3.11 | Intergenic-22 | TTTA | ACCGCUUCACCAAAAGCUGUCCCUUAGGGGAUUAGAACUUGAGUGAAGGUGGGCUGCUUGCAUCAGCCU AAUGUCGAGAAGUGCUUUCUUCGGAAAGUAACCCUCGAAACAAAGAAAGGAAUGCAACAGAACACAUA CCCCUGGGCC | 419 |
| Example 3.12 | Intergenic-22 | TTTA | CUUCACUGAUAAAGUGGAGAACCGCUUCACCAAUUAGUUGAGUGAAGGUGGGCUGCUUGCAUCAGCCUA AUGUCGAGAAGUGCUUUCUUCGGAAAGUAACCCUCGAAACAAAGAAAGGAAUGCAACAGAACACAUACC CCUGGGCC | 420 |
| Example 3.13 | Intergenic-22 | TTTA | ACCGCUUCACCAAUUAGUUGAGUGAAGGUGGGCUGCUUGCAUCAGCCUAAUGUCGAGAAGUGCUUUCUU CGGAAAGUAACCCUCGAAACAAAGAAAGGAAUGCAACAGAACACAUACCCCUGGGCC | 421 |
| Example 3.14 | Intergenic-22 | TTTA | ACCGCUUCACUUAGAGUGAAGGUGGGCUGCUUGCAUCAGCCUAAUGUCGAGAAGUGCUUUCUUCGGAAA GUAACCCUCGAAACAAAGAAAGGAAUGCAACAGAACACAUACCCCUGGGCC | 438 |

Here, for each target sequence,
1) Comparative Example n. 1 is a single guide RNA in which a naturally occurring Cas12f1 tracrRNA and a naturally occurring Cas12f1 crRNA are linked by 5'-GAAA-3';
2) Examples n.1 to n.3 are each an engineered Cas12f1 guide RNA with a spacer and an engineered scaffold region having a modified first region.
3) Examples n.4 to n.6 are each an engineered Cas12f1 guide RNA with a spacer and an engineered scaffold region having a modified second region.
4) Examples n.7 to n.9 are each an engineered Cas12f1 guide RNA with a spacer and an engineered scaffold region having modified fourth and fifth regions.
5) Example n.10 is an engineered Cas12f1 guide RNA with a spacer and an engineered scaffold region having a modified first region and a modified second region.
6) Example n.11 is an engineered Cas12f1 guide RNA with a spacer and an engineered scaffold region having a modified first region and modified fourth and fifth regions.
7) Example n.12 is an engineered Cas12f1 guide RNA with a spacer and an engineered scaffold region having a modified second region and modified fourth and fifth regions.
8) Example n.13 and Example n.14 are each an engineered Cas12f1 guide RNA with a spacer and an engineered scaffold region having a modified first region, a modified second region, and modified fourth and fifth regions.

Here, n is 1, 2, or 3 according to the target sequence, wherein a case where n is 1 represents Target 1 (DY2), a case where n is 2 represents Target 2 (DY10), and a case where n is 3 represents Target 3 (Intergenic-22).

The vector constructed in each example was transfected into HEK293 T cells according to Experimental Example 1.3, and indel generation efficiency was measured by Experimental Examples 1.4 to 1.5. The results were analyzed by Experimental Example 1.6 and are shown in FIGS. 2 to 13.

FIGS. 2 to 4 show average indel efficiency for Examples 1.1 to 1.13targeting DY2, FIGS. 5 to 8 show average indel efficiency for Examples 2.1 to 2.13 targeting DY10, FIGS. 9 to 12 show average indel efficiency for Examples 3.1 to 3.13 targeting Intergenic-22, and FIG. 13 shows average indel efficiency for Examples 1.13 to 1.14 targeting DY2, Examples 2.13 to Example 2.14 targeting DY10, and Examples 3.13 to 3.14 targeting Intergenic-22.

From the experimental results, it can be seen that when the engineered Cas12f1 guide RNA having an engineered scaffold region disclosed herein is used for gene editing, gene editing efficiency is generally enhanced as compared with a case where a Cas12f1 guide RNA having a naturally occurring scaffold region is used, and a case where a guide RNA in which a tracrRNA and a crRNA repeat sequence of the naturally occurring scaffold region are linked to each other by the linker 5'-GAAA-3'. In addition, when modifications for respective regions are combined (for example, Examples n.10 to n.14), it can be seen that further enhanced gene editing efficiency is obtained due to a synergy created by the combination.

### INDUSTRIAL APPLICABILITY

The present disclosure provides a CRISPR/Cas12f1 system that can be used for gene editing techniques, in particular, an engineered CRISPR/Cas12f1 system with improved gene editing efficiency caused by introduction of an engineered scaffold region. When the engineered CRISPR/Cas12f1 system provided herein is used for gene editing, this system exhibits high gene editing efficiency as compared with when using a naturally occurring CRISPR/Cas12f1 system is used, and thus can be used for editing a eukaryotic gene.

## Claims

1. An engineered guide RNA for a CRISPR/Cas12f1 system, comprising:
an engineered scaffold region, and
a spacer,
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10); 
5'-
GCUGCUUGCAUCAGCCUAAUGUCGAGAAGUGCUUUCUUCGGAAAGUAAC
CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
5'-AUGCAAC-3'.

2. An engineered guide RNA for a CRISPR/Cas12f1 system, comprising:
an engineered scaffold region, and
a spacer,
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAAGAAAGUUGCAGAACCCGAAUAGACGAAUGAAGGA AUGCAAC-3' (SEQ ID NO: 315), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10); 
5'-
GCUGCUUGCAUCAGCCUAAUGUCGAGAAGUGCUUUCUUCGGAAAGUAAC
CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), 5'-AACAAAUUCAUUUGAAAGAAUGAAGGA-3' (SEQ ID NO: 117), 5'-AACAAAUUCAUUUUGAAACGAAUGAAGGA-3' (SEQ ID NO: 293), 5'-AACAAAUUCAUUUUUGAAAACGAAUGAAGGA-3' (SEQ ID NO: 294), 5'-AACAAAUUCAUUUUUCGAAAGACGAAUGAAGGA-3' (SEQ ID NO: 295), 5'-AACAAAUUCAUUUUUCCGAAAAGACGAAUGAAGGA-3' (SEQ ID NO: 296), 5'-AACAAAUUCAUUUUUCCUGAAAUAGACGAAUGAAGGA-3' (SEQ ID NO: 297), 5'-AACAAAUUCAUUUUUCCUCGAAAAUAGACGAAUGAAGGA-3' (SEQ ID NO: 298), 5'-AACAAAUUCAUUUUUCCUCUGAAAAAUAGACGAAUGAAGGA-3' (SEQ ID NO: 299), 5'-AACAAAUUCAUUUUUCCUCUCGAAAGAAUAGACGAAUGAAGGA-3' (SEQ ID NO: 300), 5'-AACAAAUUCAUUUUUCCUCUCCGAAACGAAUAGACGAAUGAAGGA-3' (SEQ ID NO: 301), 5'-AACAAAUUCAUUUUUCCUCUCCAGAAACCGAAUAGACGAAUGAAGGA-3' (SEQ ID NO: 302), 5'-AACAAAUUCAUUUUUCCUCUCCAAGAAACCCGAAUAGACGAAUGAAGGA-3' (SEQ ID NO: 303), 5'-AACAAAUUCAUUUUUCCUCUCCAAUGAAAACCCGAAUAGACGAAUGAAGG A-3' (SEQ ID NO: 304), 5'-AACAAAUUCAUUUUUCCUCUCCAAUUGAAAAACCCGAAUAGACGAAUGAA GGA-3' (SEQ ID NO: 305), 5'-AACAAAUUCAUUUUUCCUCUCCAAUUCGAAAGAACCCGAAUAGACGAAUG AAGGA-3' (SEQ ID NO: 306), 5'-AACAAAUUCAUUUUUCCUCUCCAAUUCUGAAAAGAACCCGAAUAGACGAA UGAAGGA-3' (SEQ ID NO: 307), 5'-AACAAAUUCAUUUUUCCUCUCCAAUUCUGGAAACAGAACCCGAAUAGAC GAAUGAAGGA-3' (SEQ ID NO: 308), 5'-AACAAAUUCAUUUUUCCUCUCCAAUUCUGCGAAAGCAGAACCCGAAUAG ACGAAUGAAGGA-3' (SEQ ID NO: 309), 5'-AACAAAUUCAUUUUUCCUCUCCAAUUCUGCAGAAAUGCAGAACCCGAAUA GACGAAUGAAGGA-3' (SEQ ID NO: 310), 5'-AACAAAUUCAUUUUUCCUCUCCAAUUCUGCACGAAAUUGCAGAACCCGA AUAGACGAAUGAAGGA-3' (SEQ ID NO: 311), 5'-AACAAAUUCAUUUUUCCUCUCCAAUUCUGCACAGAAAGUUGCAGAACCC GAAUAGACGAAUGAAGGA-3' (SEQ ID NO: 312), 5'-AACAAAUUCAUUUUUCCUCUCCAAUUCUGCACAGAAAUUGCAGAACCCG AAUAGACGAAUGAAGGA-3' (SEQ ID NO: 313), and 5'-AACAAAUUCAUUUUUCCUCUCCAAUUCUGCACAAGAAAGUUGCAGAACC CGAAUAGACGAAUGAAGGA-3' (SEQ ID NO: 314); and
5'-AUGCAAC-3'.

3. The engineered guide RNA of claim 1, wherein the sequence of the scaffold region is such that the following sequences are sequentially linked to each other in a 5' to 3' direction:
5'-A-3'; 
5'-
CCGCUUCACCAAAAGCUGUCCCUUAGGGGAUUAGAACUUGAGUGAAGGU
GG-3' (SEQ ID NO: 10);
 
5'-
GCUGCUUGCAUCAGCCUAAUGUCGAGAAGUGCUUUCUUCGGAAAGUAAC
CCUCGA-3' (SEQ ID NO: 11);
5'-AACAAAGAAAGGA-3' (SEQ ID NO: 110); and
5'-AUGCAAC-3'.

4. The engineered guide RNA of claim 1, wherein the sequence of the scaffold region is such that the following sequences are sequentially linked to each other in a 5' to 3' direction:
5'-A-3';
5'-CCGCUUCACUUAGAGUGAAGGUGG-3' (SEQ ID NO: 430); 
5'-
GCUGCUUGCAUCAGCCUAAUGUCGAGAAGUGCUUUCUUCGGAAAGUAAC
CCUCGA-3' (SEQ ID NO: 11);
5'-AACAAAGAAAGGA-3' (SEQ ID NO: 110); and
5'-AUGCAAC-3'.

5. An engineered guide RNA for a CRISPR/Cas12f1 system, comprising:
an engineered scaffold region, and
a spacer,
wherein the engineered scaffold region and the spacer are sequentially linked to each other in a 5' to 3' direction,
the spacer has a length of 10 to 50 nucleotides and has a sequence complementary to a target sequence, and
a sequence of the engineered scaffold region is such that the following sequences are sequentially linked to each other in a 5' to 3' direction:
a first sequence represented by 5'-A-3';
a second sequence represented by 5'-CCGCUUCAC-3' (SEQ ID NO: 432);
a third sequence represented by 5'-UUAG-3';
a fourth sequence represented by 5'-AGUGAAGGUGG-3' (SEQ ID NO: 433);
a fifth sequence represented by 5'-GCUGCUUGCAUCAGCCUAAUGUCGAGAAGUGCUUUCUUCGGAAAGUAAC CCUCGA-3' (SEQ ID NO: 11);
a sixth sequence represented by 5'-AACAAA-3';
a linker;
a seventh sequence represented by 5'-GGA-3'; and
an eighth sequence represented by 5'-AUGCAAC-3'.

6. The engineered guide RNA of claim 5, wherein the linker is 5'-GAAA-3'.

7. The engineered guide RNA of claim 5, wherein the linker is selected from the group consisting of 5'-GAAA-3', 5'-UGAAAA-3', 5'-UUGAAAAA-3', 5'-UUCGAAAGAA-3' (SEQ ID NO: 425), 5'-UUCAGAAAUGAA-3' (SEQ ID NO: 426), 5'-UUCAUGAAAAUGAA-3' (SEQ ID NO: 427), and 5'-UUCAUUGAAAAAUGAA-3' (SEQ ID NO: 428).

8. The engineered guide RNA of claim 5, wherein the sequence of the engineered scaffold region further comprises a ninth sequence selected from the group consisting of 5'-A-3', 5'-GA-3', 5'-AGA-3', 5'-GAGA-3', 5'-GGAGA-3', 5'-UGGAGA-3', 5'-GUGGAGA-3', 5'-AGUGGAGA-3', 5'-AAGUGGAGA-3', 5'-AAAGUGGAGA-3' (SEQ ID NO: 27), 5'-UAAAGUGGAGA-3' (SEQ ID NO: 28), 5'-AUAAAGUGGAGA-3' (SEQ ID NO: 29), 5'-GAUAAAGUGGAGA-3' (SEQ ID NO: 30), 5'-UGAUAAAGUGGAGA-3' (SEQ ID NO: 31), 5'-CUGAUAAAGUGGAGA-3' (SEQ ID NO: 32), 5'-ACUGAUAAAGUGGAGA-3' (SEQ ID NO: 33), 5'-CACUGAUAAAGUGGAGA-3' (SEQ ID NO: 34), 5'-UCACUGAUAAAGUGGAGA-3' (SEQ ID NO: 35), 5'-UUCACUGAUAAAGUGGAGA-3' (SEQ ID NO: 36), and 5'-CUUCACUGAUAAAGUGGAGA-3' (SEQ ID NO: 37), and the 3' end of the ninth sequence is linked to the 5' end of the first sequence.

9. The engineered guide RNA of claim 5, wherein the sequence of the engineered scaffold region further comprises:
a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 52), and 5'-AAAGCUGUCCC-3' (SEQ ID NO: 53); and
an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 54), 5'-GGAUUAGAACU-3' (SEQ ID NO: 55), and 5'-GGGAUUAGAACU-3' (SEQ ID NO: 56),
wherein the 3' end of the second sequence and the 5' end of the third sequence are linked to each other by the tenth sequence, and
the 3' end of the third sequence and the 5' end of the fourth sequence are linked to each other by the eleventh sequence.

10. The engineered guide RNA of claim 9, wherein
when the tenth sequence is 5'-A-3', the eleventh sequence is 5'-U-3',
when the tenth sequence is 5'-AA-3', the eleventh sequence is 5'-CU-3',
when the tenth sequence is 5'-AAA-3', the eleventh sequence is 5'-ACU-3',
when the tenth sequence is 5'-AAAG-3', the eleventh sequence is 5'-AACU-3',
when the tenth sequence is 5'-AAAGC-3', the eleventh sequence is 5'-GAACU-3',
when the tenth sequence is 5'-AAAGCU-3', the eleventh sequence is 5'-AGAACU-3',
when the tenth sequence is 5'-AAAGCUG-3', the eleventh sequence is 5'-UAGAACU-3', or 5'-UUAGAACU-3',
when the tenth sequence is 5'-AAAGCUGU-3', the eleventh sequence is 5'-AUUAGAACU-3',
when the tenth sequence is 5'-AAAGCUGUC-3', the eleventh sequence is 5'-GAUUAGAACU-3' (SEQ ID NO:54),
when the tenth sequence is 5'-AAAGCUGUCC-3' (SEQ ID NO: 52), the eleventh sequence is 5'-GGAUUAGAACU-3' (SEQ ID NO:55),
when the tenth sequence is 5'-AAAGCUGUCCC-3' (SEQ ID NO: 53), the eleventh sequence is 5'-GGGAUUAGAACU-3' (SEQ ID NO:56),
when the tenth sequence is 5'-AAAAGCUGUCCC-3' (SEQ ID NO: 440), the eleventh sequence may be 5'-GGGAUUAGAACUU-3' (SEQ ID NO: 442), or
when the tenth sequence is 5'-CAAAAGCUGUCCC-3' (SEQ ID NO: 441), the eleventh sequence is 5'-GGGAUUAGAACUUG-3' (SEQ ID NO: 443).

11. The engineered guide RNA of claim 10, wherein the tenth sequence is 5'-CAAAAGCUGUCCC-3' (SEQ ID NO: 441) and the eleventh sequence is 5'-GGGAUUAGAACUUG-3' (SEQ ID NO: 443).

12. The engineered guide RNA of claim 5, wherein the sequence of the engineered scaffold region further comprises:
a twelfth sequence selected from the group consisting of 5'-U-3', 5'-UU-3', 5'-UUC-3', 5'-UUCA-3', 5'-UUCAU-3', 5'-UUCAUU-3', and 5'-UUCAUUU-3'; and
a thirteenth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3',
wherein the 3' end of the sixth sequence and the 5' end of the linker are linked to each other by the twelfth sequence, and
the 3' end of the third sequence and the 5' end of the seventh sequence are linked to each other by the thirteenth sequence.

13. The engineered guide RNA of claim 12, wherein
when the twelfth sequence is 5'-U-3', the thirteenth sequence is 5'-A-3',
when the twelfth sequence is 5'-UU-3', the thirteenth sequence is 5'-AA-3',
when the twelfth sequence is 5'-UUC-3', the thirteenth sequence is 5'-GAA-3',
when the twelfth sequence is 5'-UUCA-3', the thirteenth sequence is 5'-UGAA-3',
when the twelfth sequence is 5'-UUCAU-3', the thirteenth sequence is 5'-AUGAA-3',
when the twelfth sequence is 5'-UUCAUU-3', the thirteenth sequence is 5'-AAUGAA-3', or
when the twelfth sequence is 5'-UUCAUUU-3', the thirteenth sequence is 5'-GAAUGAA-3' .

14. An engineered guide RNA for a CRISPR/Cas12f1 system, comprising:
an engineered scaffold region, and
a spacer,
wherein the spacer has a length of 10 to 50 nucleotides and has a sequence complementary to a target sequence, and
a sequence of the engineered scaffold region comprises in a 5' to 3' direction:
an engineered tracrRNA in which the following sequences are linked to each other:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9),
a sequence selected from the group consisting of 5'-CCGCUUCACUUAGAGUGAAGGUGG-3' (SEQ ID NO: ID NO: 430)' 5'-CCGCUUCACCUUAGGAGUGAAGGUG'-3' (SEQ ID NO: 431)' 5'-CCGCUUCACCAUUAGUGAGUGAAGGUG'-3' (SEQ ID NO: 38)' 5'-CCGCUUCACCAAUUAGUUGAGUGAAGGUG'-3' (SEQ ID NO: 39)' 5'-CCGCUUCACCAAAUUAGCUUGAGUGAAGGUG'-3' (SEQ ID NO: 40)' 5'-CCGCUUCACCAAAAUUAGACUUGAGUGAAGGUG'-3' (SEQ ID NO: 41)' 5'-CCGCUUCACCAAAAGUUAGAACUUGAGUGAAGGUG'-3' (SEQ ID NO: 42)' 5'-CCGCUUCACCAAAAGCUUAGGAACUUGAGUGAAGGUG'-3' (SEQ ID NO: 43), 5'-CCGCUUCACCAAAAGCUUUAGAGAACUUGAGUGAAGGUG'-3' (SEQ ID NO: 44)' 5'-CCGCUUCACCAAAAGCUGUUAGUUAGAACUUGAGUGAAGGUG'-3' (SEQ ID NO: 45)' 5'-CCGCUUCACCAAAAGCUGUUAGUAGAACUUGAGUGAAGGUG'-3' (SEQ ID NO: 46)' 5'-CCGCUUCACCAAAAGCUGUUUAGAUUAGAACUUGAGUGAAGGUG'-3' (SEQ ID NO: 47)' 5'-CCGCUUCACCAAAAGCUGUCUUAGGAUUAGAACUUGAGUGAAGGUG'-3' (SEQ ID NO: 48)' 5'-CCGCUUCACCAAAAGCUGUCCUUAGGGAUUAGAACUUGAGUGAAGGUG'-3' (SEQ ID NO: 49), an' 5'-CCGCUUCACCAAAAGCUGUCCCUUAGGGGAUUAGAACUUGAGUGAAGGU G'-3' (SEQ ID NO: 10),
5'-GCUGCUUGCAUCAGCCUAAUGUCGAGAAGUGCUUUCUUCGGAAAGUAAC CCUCGA-3' (SEQ ID NO: 11), and
a sequence selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 66), 5'-AACAAAUUCAU-3' (SEQ ID NO: 67), 5'-AACAAAUUCAUU-3' (SEQ ID NO: 68), and 5'-AACAAAUUCAUUU-3' (SEQ ID NO: 12); and
an engineered crRNA repeat sequence portion in which the following sequences are linked to each other:
a sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', and 5'-GAAUGAAGGA-3' (SEQ ID NO: 14), and
5'-AUGCAAC-3',
wherein the 3' end of the engineered crRNA repeat sequence is linked to the 5' end of the spacer, and
wherein one in which the sequence of the engineered tracrRNA is the same as 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUU-3' (SEQ ID NO: 1) and the engineered crRNA repeat sequence is the same as 5'-GAAUGAAGGAAUGCAAC-3' (SEQ ID NO: 3) is excluded.

15. An engineered CRISPR/Cas12f1 complex comprising:
a Cas12f1 protein, and
an engineered guide RNA,
wherein the engineered guide RNA comprises:
an engineered scaffold region, and
a spacer,
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
5'-GCUGCUUGCAUCAGCCUAAUGUCGAGAAGUGCUUUCUUCGGAAAGUAAC CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
5'-AUGCAAC-3'.

16. The engineered CRISPR/Cas12f1 complex of claim 15, wherein the sequence of the scaffold region included in the engineered guide RNA is such that the following sequences are sequentially linked to each other in a 5' to 3' direction:
5'-A-3'; 
5'-
CCGCUUCACCAAAAGCUGUCCCUUAGGGGAUUAGAACUUGAGUGAAGGU
GG-3' (SEQ ID NO: 10);
 
5'-
GCUGCUUGCAUCAGCCUAAUGUCGAGAAGUGCUUUCUUCGGAAAGUAAC
CCUCGA-3' (SEQ ID NO: 11);
5'-AACAAAGAAAGGA-3' (SEQ ID NO: 110); and
5'-AUGCAAC-3'.

17. The engineered CRISPR/Cas12f1 complex of claim 15, wherein the sequence of the scaffold region is such that the following sequences are sequentially linked to each other in a 5' to 3' direction:
5'-A-3';
5'-CCGCUUCACUUAGAGUGAAGGUGG-3' (SEQ ID NO: 430); 
5'-
GCUGCUUGCAUCAGCCUAAUGUCGAGAAGUGCUUUCUUCGGAAAGUAAC
CCUCGA-3' (SEQ ID NO: 11);
5'-AACAAAGAAAGGA-3' (SEQ ID NO: 110); and
5'-AUGCAAC-3'.

18. An engineered CRISPR/Cas12f1 complex comprising:
a Cas12f1 protein, and
the engineered guide RNA of any one of claims 5 to 13.

19. A vector capable of expressing respective components of a CRISPR/Cas12f1 system, comprising:
a first sequence comprising a nucleic acid sequence encoding a Cas12f1 protein;
a first promoter sequence operably linked to the first sequence;
a second sequence comprising a nucleic acid sequence encoding an engineered guide RNA; and
a second promoter sequence operably linked to the second sequence,
wherein the engineered guide RNA comprises:
an engineered scaffold region, and
a spacer,
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10); 
5'-
GCUGCUUGCAUCAGCCUAAUGUCGAGAAGUGCUUUCUUCGGAAAGUAAC
CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
5'-AUGCAAC-3'.

20. The vector of claim 19, wherein the sequence of the scaffold region is such that the following sequences are linked to each other in a 5' to 3' direction:
5'-A-3'; 
5'-
CCGCUUCACCAAAAGCUGUCCCUUAGGGGAUUAGAACUUGAGUGAAGGU
GG-3' (SEQ ID NO: 10);
 
5'-
GCUGCUUGCAUCAGCCUAAUGUCGAGAAGUGCUUUCUUCGGAAAGUAAC
CCUCGA-3' (SEQ ID NO: 11);
5'-AACCAAAGAAAGGA-3' (SEQ ID NO: 110); and
5'-AUGCAAC-3'.

21. The vector of claim 19, wherein the sequence of the scaffold region is such that the following sequences are linked to each other in a 5' to 3' direction:
5'-A-3';
5'-CCGCUUCACUUAGAGUGAAGGUGG-3' (SEQ ID NO: 430); 
5'-
GCUGCUUGCAUCAGCCUAAUGUCGAGAAGUGCUUUCUUCGGAAAGUAAC
CCUCGA-3' (SEQ ID NO: 11);
5'-AACCAAAGAAAGGA-3' (SEQ ID NO: 110); and
5'-AUGCAAC-3'.

22. The vector of claim 19, wherein the vector is at least one selected from the group consisting of a plasmid, a retrovirus, a lentivirus, an adenovirus, an adeno-associated virus, a vaccinia virus, a poxvirus, and a herpes simplex virus.

23. A vector capable of expressing respective components of a CRISPR/Cas12f1 system, comprising:
a first sequence comprising a nucleic acid sequence encoding a Cas12f1 protein;
a first promoter sequence operably linked to the first sequence;
a second sequence comprising a nucleic acid sequence encoding the engineered guide RNA of any one of claims 5 to 13; and
a second promoter sequence operably linked to the second sequence.

24. A method of editing a target nucleic acid in a cell, comprising:
delivering, into the cell, a Cas12f1 protein or a nucleic acid encoding the Cas12f1 protein, and an engineered guide RNA or a nucleic acid encoding the engineered guide RNA,
which allows a CRISPR/Cas12f1 complex to be formed in the cell,
wherein the CRISPR/Cas12f1 complex is capable of editing the target nucleic acid, and
the engineered guide RNA comprises:
an engineered scaffold region, and
a spacer,
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUGAAAGA AUGAAGGAAUGCAAC-3' (SEQ ID NO: 7), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10); 
5'-
GCUGCUUGCAUCAGCCUAAUGUCGAGAAGUGCUUUCUUCGGAAAGUAAC
CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), and 5'-AACAAAUUCAUUUGAAAGAAUGAAGGA-3' (SEQ ID NO: 117); and
5'-AUGCAAC-3'.

25. A method of editing a target nucleic acid in a cell, comprising:
delivering, into the cell, a Cas12f1 protein or a nucleic acid encoding the same, and an engineered guide RNA or a nucleic acid encoding the same,
which allows a CRISPR/Cas12f1 complex to be formed in the cell
wherein the CRISPR/Cas12f1 complex is capable of editing the target nucleic acid, and
the engineered guide RNA comprises:
an engineered scaffold region, and
a spacer,
wherein the engineered scaffold region and the spacer are sequentially linked to each other in a 5' to 3' direction,
the spacer comprises 10 nucleotides to 50 nucleotides and has a sequence complementary to a target sequence,
a sequence of the engineered scaffold region is different from 5'-CUUCACUGAUAAAGUGGAGAACCGCUUCACCAAAAGCUGUCCCUUAGGG GAUUAGAACUUGAGUGAAGGUGGGCUGCUUGCAUCAGCCUAAUGUCGA GAAGUGCUUUCUUCGGAAAGUAACCCUCGAAACAAAUUCAUUUUUCCUC UCCAAUUCUGCACAAGAAAGUUGCAGAACCCGAAUAGACGAAUGAAGGA AUGCAAC-3' (SEQ ID NO: 315), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 16), 5'-AAAGUGGAGAA-3' (SEQ ID NO: 17), 5'-UAAAGUGGAGAA-3' (SEQ ID NO: 18), 5'-AUAAAGUGGAGAA-3' (SEQ ID NO: 19), 5'-GAUAAAGUGGAGAA-3' (SEQ ID NO: 20), 5'-UGAUAAAGUGGAGAA-3' (SEQ ID NO: 21), 5'-CUGAUAAAGUGGAGAA-3' (SEQ ID NO: 22), 5'-ACUGAUAAAGUGGAGAA-3' (SEQ ID NO: 23), 5'-CACUGAUAAAGUGGAGAA-3' (SEQ ID NO: 24), 5'-UCACUGAUAAAGUGGAGAA-3' (SEQ ID NO: 25), 5'-UUCACUGAUAAAGUGGAGAA-3' (SEQ ID NO: 26), and 5'-CUUCACUGAUAAAGUGGAGAA-3' (SEQ ID NO: 9);
a sequence selected from the group consisting of 5'-CCGCUUCACUUAGAGUGAAGGUGG-3' (SEQ ID NO: 430), 5'-CCGCUUCACCUUAGGAGUGAAGGUGG-3' (SEQ ID NO: 431), 5'-CCGCUUCACCAUUAGUGAGUGAAGGUGG-3' (SEQ ID NO: 38), 5'-CCGCUUCACCAAUUAGUUGAGUGAAGGUGG-3' (SEQ ID NO: 39), 5'-CCGCUUCACCAAAUUAGCUUGAGUGAAGGUGG-3' (SEQ ID NO: 40), 5'-CCGCUUCACCAAAAUUAGACUUGAGUGAAGGUGG-3' (SEQ ID NO: 41), 5'-CCGCUUCACCAAAAGUUAGAACUUGAGUGAAGGUGG-3' (SEQ ID NO: 42), 5'-CCGCUUCACCAAAAGCUUAGGAACUUGAGUGAAGGUGG-3' (SEQ ID NO: 43), 5'-CCGCUUCACCAAAAGCUUUAGAGAACUUGAGUGAAGGUGG-3' (SEQ ID NO: 44), 5'-CCGCUUCACCAAAAGCUGUUAGUUAGAACUUGAGUGAAGGUGG-3' (SEQ ID NO: 45), 5'-CCGCUUCACCAAAAGCUGUUAGUAGAACUUGAGUGAAGGUGG-3' (SEQ ID NO: 46), 5'-CCGCUUCACCAAAAGCUGUUUAGAUUAGAACUUGAGUGAAGGUGG-3' (SEQ ID NO: 47), 5'-CCGCUUCACCAAAAGCUGUCUUAGGAUUAGAACUUGAGUGAAGGUGG-3' (SEQ ID NO: 48), 5'-CCGCUUCACCAAAAGCUGUCCUUAGGGAUUAGAACUUGAGUGAAGGUG G-3' (SEQ ID NO: 49), and 5'-CCGCUUCACCAAAAGCUGUCCCUUAGGGGAUUAGAACUUGAGUGAAGGU GG-3' (SEQ ID NO: 10);
5'-GCUGCUUGCAUCAGCCUAAUGUCGAGAAGUGCUUUCUUCGGAAAGUAAC CCUCGA-3' (SEQ ID NO: 11);
a sequence selected from the group consisting of 5'-AACAAAGAAAGGA-3' (SEQ ID NO: 110), 5'-AACAAAUGAAAAGGA-3' (SEQ ID NO: 111), 5'-AACAAAUUGAAAAAGGA-3' (SEQ ID NO: 112), 5'-AACAAAUUCGAAAGAAGGA-3' (SEQ ID NO: 113), 5'-AACAAAUUCAGAAAUGAAGGA-3' (SEQ ID NO: 114), 5'-AACAAAUUCAUGAAAAUGAAGGA-3' (SEQ ID NO: 115), 5'-AACAAAUUCAUUGAAAAAUGAAGGA-3' (SEQ ID NO: 116), 5'-AACAAAUUCAUUUGAAAGAAUGAAGGA-3' (SEQ ID NO: 117), 5'-AACAAAUUCAUUUUGAAACGAAUGAAGGA-3' (SEQ ID NO: 293), 5'-AACAAAUUCAUUUUUGAAAACGAAUGAAGGA-3' (SEQ ID NO: 294), 5'-AACAAAUUCAUUUUUCGAAAGACGAAUGAAGGA-3' (SEQ ID NO: 295), 5'-AACAAAUUCAUUUUUCCGAAAAGACGAAUGAAGGA-3' (SEQ ID NO: 296), 5'-AACAAAUUCAUUUUUCCUGAAAUAGACGAAUGAAGGA-3' (SEQ ID NO: 297), 5'-AACAAAUUCAUUUUUCCUCGAAAAUAGACGAAUGAAGGA-3' (SEQ ID NO: 298), 5'-AACAAAUUCAUUUUUCCUCUGAAAAAUAGACGAAUGAAGGA-3' (SEQ ID NO: 299), 5'-AACAAAUUCAUUUUUCCUCUCGAAAGAAUAGACGAAUGAAGGA-3' (SEQ ID NO: 300), 5'-AACAAAUUCAUUUUUCCUCUCCGAAACGAAUAGACGAAUGAAGGA-3' (SEQ ID NO: 301), 5'-AACAAAUUCAUUUUUCCUCUCCAGAAACCGAAUAGACGAAUGAAGGA-3' (SEQ ID NO: 302), 5'-AACAAAUUCAUUUUUCCUCUCCAAGAAACCCGAAUAGACGAAUGAAGGA-3' (SEQ ID NO: 303), 5'-AACAAAUUCAUUUUUCCUCUCCAAUGAAAACCCGAAUAGACGAAUGAAGG A-3' (SEQ ID NO: 304), 5'-AACAAAUUCAUUUUUCCUCUCCAAUUGAAAAACCCGAAUAGACGAAUGAA GGA-3' (SEQ ID NO: 305), 5'-AACAAAUUCAUUUUUCCUCUCCAAUUCGAAAGAACCCGAAUAGACGAAUG AAGGA-3' (SEQ ID NO: 306), 5'-AACAAAUUCAUUUUUCCUCUCCAAUUCUGAAAAGAACCCGAAUAGACGAA UGAAGGA-3' (SEQ ID NO: 307), 5'-AACAAAUUCAUUUUUCCUCUCCAAUUCUGGAAACAGAACCCGAAUAGAC GAAUGAAGGA-3' (SEQ ID NO: 308), 5'-AACAAAUUCAUUUUUCCUCUCCAAUUCUGCGAAAGCAGAACCCGAAUAG ACGAAUGAAGGA-3' (SEQ ID NO: 309), 5'-AACAAAUUCAUUUUUCCUCUCCAAUUCUGCAGAAAUGCAGAACCCGAAUA GACGAAUGAAGGA-3' (SEQ ID NO: 310), 5'-AACAAAUUCAUUUUUCCUCUCCAAUUCUGCACGAAAUUGCAGAACCCGA AUAGACGAAUGAAGGA-3' (SEQ ID NO: 311), 5'-AACAAAUUCAUUUUUCCUCUCCAAUUCUGCACAGAAAGUUGCAGAACCC GAAUAGACGAAUGAAGGA-3' (SEQ ID NO: 312), 5'-AACAAAUUCAUUUUUCCUCUCCAAUUCUGCACAGAAAUUGCAGAACCCG AAUAGACGAAUGAAGGA-3' (SEQ ID NO: 313), and 5'-AACAAAUUCAUUUUUCCUCUCCAAUUCUGCACAAGAAAGUUGCAGAACC CGAAUAGACGAAUGAAGGA-3' (SEQ ID NO: 314); and
5'-AUGCAAC-3'.

26. The method of any one of claim 24 or claim 25, wherein the delivery is achieved by introducing, into the cell, the Cas12f1 protein and the engineered guide RNA as a CRISPR/Cas12f1 complex.

27. The method of claim 24, wherein the delivery is achieved by introducing, into the cell, a vector comprising a nucleic acid encoding the Cas12f1 protein and a nucleic acid encoding the engineered guide RNA.

28. The method of any one of claim 24 or claim 25, wherein the cell is a eukaryotic cell.

29. The vector of claim 27, wherein the vector is at least one selected from the group consisting of a plasmid, a retrovirus, a lentivirus, an adenovirus, an adeno-associated virus, a vaccinia virus, a poxvirus, and a herpes simplex virus.

30. A method of editing a target nucleic acid in a cell, comprising:
delivering, into the cell, a Cas12f1 protein or a nucleic acid encoding the same, and the engineered guide RNA of any one of claims 5 to 13 or a nucleic acid encoding the same,
which allows a CRISPR/Cas12f1 complex to be formed in the cell, and
wherein the CRISPR/Cas12f1 complex is capable of editing the target nucleic acid.

31. DNA encoding the engineered guide RNA of claim 1 or 2.

32. DNA encoding the engineered guide RNA of any one of claims 5 to 13.

33. DNA encoding the engineered guide RNA of claim 14.
